# EUROPEAN PATENT APPLICATION

(11) **EP 1 300 418 A1**
(43) Date of publication of application: **09.04.2003**
(21) Application number: 01203748.7
(22) Date of filing: 04.10.2001
(51) Int. Cl.: C07K 14/59, C07K 5/083, C07K 5/103, C07K 7/06, C07K 7/08, A61K 38/00

(54) **Gene regulation by oligopeptides**

(71) Applicant: Erasmus Universiteit Rotterdam, 3015 GE Rotterdam (NL)
(72) Inventor: Khan, Nisar Ahmed, 3074 AD Rotterdam (NL); Benner, Robbert, 2992 SH Barendrecht (NL)
(74) Representative: Prins, Adrianus Willem

(57) **Abstract**

The invention relates to the modulation of gene expression in a cell, also called gene control, in particular in relation to the treatment of a variety of diseases. The invention provides a method for modulating expression of a gene in a cell comprising providing said cell with a signalling molecule comprising a peptide or functional analogue thereof.. Furthermore, the invention provides a method for identifying or obtaining a signalling molecule comprising a peptide or functional derivative or analogue thereof capable of modulating expression of a gene in a cell comprising providing said cell with a peptide or derivative or analogue thereof and determining the activity and/or nuclear translocation of a gene transcription factor.

SEQUENCE LISTING

<110> Erasmus Universiteit Rotterdam

<120> Gene regulation by oligopeptides

<130> P57495EP00

<140> 01203748.7
<141> 2001-10-04

<160> 187

<170> PatentIn Ver. 2.1

<210> 1
<211> 3
<212> PRT
<213> Artificial Sequence

<220>
<223> Description of Artificial Sequence: oligopeptide

<220>
<221> SITE
<222> (1)..(3)

<400> 1
Leu Gln Gly
  1


<210> 2
<211> 3
<212> PRT
<213> Artificial Sequence

<220>
<223> Description of Artificial Sequence: oligopeptide

<220>
<221> SITE
<222> (1)..(3)

<400> 2
Ala Gln Gly
  1


<210> 3
<211> 4
<212> PRT
<213> Artificial Sequence

<220>
<223> Description of Artificial Sequence: oligopeptide

<220>
<221> SITE
<222> (1)..(4)

<400> 3
Leu Gln Gly Val
  1


<210> 4
<211> 4

<212> PRT
<213> Artificial Sequence

<220>
<223> Description of Artificial Sequence: oligopeptide

<220>
<221> SITE
<222> (1)..(4)

<400> 4
Ala Gln Gly Val
1


<210> 5
<211> 4
<212> PRT
<213> Artificial Sequence

<220>
<223> Description of Artificial Sequence: oligopeptide

<220>
<221> SITE
<222> (1)..(4)

<400> 5
Leu Gln Gly Ala
1


<210> 6
<211> 10
<212> PRT
<213> Artificial Sequence

<220>
<223> Description of Artificial Sequence: oligopeptide

<220>
<221> SITE
<222> (1)..(10)

<400> 6
Val Leu Pro Ala Leu Pro Gln Val Val Cys
 1               5                   10


<210> 7
<211> 6
<212> PRT
<213> Artificial Sequence

<220>
<223> Description of Artificial Sequence: oligopeptide

<220>
<221> SITE
<222> (1)..(6)

<400> 7
Val Leu Pro Ala Leu Pro
 1               5


<210> 8
<211> 6

<212> PRT
<213> Artificial Sequence

<220>
<223> Description of Artificial Sequence: oligopeptide

<220>
<221> SITE
<222> (1)..(6)

<400> 8
Ala Leu Pro Ala Leu Pro
 1               5


<210> 9
<211> 6
<212> PRT
<213> Artificial Sequence

<220>
<223> Description of Artificial Sequence: oligopeptide

<220>
<221> SITE
<222> (1)..(6)

<400> 9
Val Ala Pro Ala Leu Pro
 1               5


<210> 10
<211> 7
<212> PRT
<213> Artificial Sequence

<220>
<223> Description of Artificial Sequence: oligopeptide

<220>
<221> SITE
<222> (1)..(7)

<400> 10
Ala Leu Pro Ala Leu Pro Gln
 1               5


<210> 11
<211> 7
<212> PRT
<213> Artificial Sequence

<220>
<223> Description of Artificial Sequence: oligopeptide

<220>
<221> SITE
<222> (1)..(7)

<400> 11
Val Leu Pro Ala Ala Pro Gln
 1               5


<210> 12
<211> 8

<212> PRT
<213> Artificial Sequence

<220>
<223> Description of Artificial Sequence: oligopeptide

<220>
<221> SITE
<222> (1)..(8)

<400> 12
Val Leu Pro Ala Leu Pro Ala Gln
 1               5


<210> 13
<211> 4
<212> PRT
<213> Artificial Sequence

<220>
<223> Description of Artificial Sequence: oligopeptide

<220>
<221> SITE
<222> (1)..(4)

<400> 13
Leu Ala Gly Val
1


<210> 14
<211> 6
<212> PRT
<213> Artificial Sequence

<220>
<223> Description of Artificial Sequence: oligopeptide

<220>
<221> SITE
<222> (1)..(6)

<400> 14
Val Leu Ala Ala Leu Pro
 1               5


<210> 15
<211> 6
<212> PRT
<213> Artificial Sequence

<220>
<223> Description of Artificial Sequence: oligopeptide

<220>
<221> SITE
<222> (1)..(6)

<400> 15
Val Leu Pro Ala Leu Ala
 1               5


<210> 16
<211> 7

<212> PRT
<213> Artificial Sequence

<220>
<223> Description of Artificial Sequence: oligopeptide

<220>
<221> SITE
<222> (1)..(7)

<400> 16
Val Leu Pro Ala Leu Pro Gln
1               5


<210> 17
<211> 7
<212> PRT
<213> Artificial Sequence

<220>
<223> Description of Artificial Sequence: oligopeptide

<220>
<221> SITE
<222> (1)..(7)

<400> 17
Val Leu Ala Ala Leu Pro Gln
1               5


<210> 18
<211> 7
<212> PRT
<213> Artificial Sequence

<220>
<223> Description of Artificial Sequence: oligopeptide

<220>
<221> SITE
<222> (1)..(7)

<400> 18
Val Leu Pro Ala Leu Pro Ala
1               5


<210> 19
<211> 7
<212> PRT
<213> Artificial Sequence

<220>
<223> Description of Artificial Sequence: oligopeptide

<220>
<221> SITE
<222> (1)..(7)

<400> 19
Gly Val Leu Pro Ala Leu Pro
1               5


<210> 20
<211> 13

<212> PRT
<213> Artificial Sequence

<220>
<223> Description of Artificial Sequence: oligopeptide

<220>
<221> SITE
<222> (1)..(13)

<400> 20
Leu Gln Gly Val Leu Pro Ala Leu Pro Gln Val Val Cys
1               5                   10


<210> 21
<211> 38
<212> PRT
<213> Artificial Sequence

<220>
<223> Description of Artificial Sequence: oligopeptide

<220>
<221> SITE
<222> (1)..(38)

<400> 21
Val Val Cys Asn Tyr Arg Asp Val Arg Phe Glu Ser Ile Arg Leu Pro
1               5                   10                  15

Gly Cys Pro Arg Gly Val Asn Pro Val Val Ser Tyr Ala Val Ala Leu
            20                  25                  30

Ser Cys Gln Cys Ala Leu
        35


<210> 22
<211> 15
<212> PRT
<213> Artificial Sequence

<220>
<223> Description of Artificial Sequence: oligopeptide

<220>
<221> SITE
<222> (1)..(15)

<400> 22
Arg Pro Arg Cys Arg Pro Ile Asn Ala Thr Leu Ala Val Glu Lys
1               5                   10                  15


<210> 23
<211> 20
<212> PRT
<213> Artificial Sequence

<220>
<223> Description of Artificial Sequence: oligopeptide

<220>
<221> SITE
<222> (1)..(20)

<400> 23

Glu Gly Cys Pro Val Cys Ile Thr Val Asn Thr Thr Ile Cys Ala Gly
1               5                   10                  15

Tyr Cys Pro Thr
            20

<210> 24
<211> 18
<212> PRT
<213> Artificial Sequence

<220>
<223> Description of Artificial Sequence: oligopeptide

<220>
<221> SITE
<222> (1)..(18)

<400> 24
Ser Lys Ala Pro Pro Pro Ser Leu Pro Ser Pro Ser Arg Leu Pro Gly
1               5                   10                  15

Pro Ser

<210> 25
<211> 16
<212> PRT
<213> Artificial Sequence

<220>
<223> Description of Artificial Sequence: oligopeptide

<220>
<221> SITE
<222> (1)..(16)

<400> 25
Ser Ile Arg Leu Pro Gly Cys Pro Arg Gly Val Asn Pro Val Val Ser
1               5                   10                  15

<210> 26
<211> 13
<212> PRT
<213> Artificial Sequence

<220>
<223> Description of Artificial Sequence: oligopeptide

<220>
<221> SITE
<222> (1)..(13)

<400> 26
Leu Pro Gly Cys Pro Arg Gly Val Asn Pro Val Val Ser
1               5                   10

<210> 27
<211> 4
<212> PRT
<213> Artificial Sequence

<220>
<223> Description of Artificial Sequence: oligopeptide

<220>
<221> SITE
<222> (1)..(4)

<400> 27
Leu Pro Gly Cys
  1


<210> 28
<211> 4
<212> PRT
<213> Artificial Sequence

<220>
<223> Description of Artificial Sequence: oligopeptide

<220>
<221> SITE
<222> (1)..(4)

<400> 28
Met Thr Arg Val
  1


<210> 29
<211> 3
<212> PRT
<213> Artificial Sequence

<220>
<223> Description of Artificial Sequence: oligopeptide

<220>
<221> SITE
<222> (1)..(3)

<400> 29
Met Thr Arg
  1


<210> 30
<211> 3
<212> PRT
<213> Artificial Sequence

<220>
<223> Description of Artificial Sequence: oligopeptide

<220>
<221> SITE
<222> (1)..(3)

<400> 30
Val Val Cys
  1


<210> 31
<211> 4
<212> PRT
<213> Artificial Sequence

<220>
<223> Description of Artificial Sequence: oligopeptide

<220>
<221> SITE
<222> (1)..(4)

<400> 31
Gln Val Val Cys
1


<210> 32
<211> 17
<212> PRT
<213> Artificial Sequence

<220>
<223> Description of Artificial Sequence: peptide
      signalling molecule

<220>
<221> SITE
<222> (1)..(17)

<400> 32
Met Thr Arg Val Leu Gln Gly Val Leu Pro Ala Leu Pro Gln Val Val
1               5                   10                  15

Cys


<210> 33
<211> 35
<212> PRT
<213> Artificial Sequence

<220>
<223> Description of Artificial Sequence: peptide
      signalling molecule

<220>
<221> SITE
<222> (1)..(35)

<400> 33
Arg Pro Arg Cys Arg Pro Ile Asn Ala Thr Leu Ala Val Glu Lys Glu
1               5                   10                  15

Gly Cys Pro Val Cys Ile Thr Val Asn Thr Thr Ile Cys Ala Gly Tyr
            20                  25                  30

Cys Pro Thr
        35


<210> 34
<211> 21
<212> PRT
<213> Artificial Sequence

<220>
<223> Description of Artificial Sequence: peptide
      signalling molecule

<220>
<221> SITE
<222> (1)..(21)

<400> 34
Cys Ala Leu Cys Arg Arg Ser Thr Thr Asp Cys Gly Gly Pro Lys Asp
1               5                   10                  15

His Pro Leu Thr Cys
            20


<210> 35
<211> 18
<212> PRT
<213> Artificial Sequence

<220>
<223> Description of Artificial Sequence: peptide
      signalling molecule

<220>
<221> SITE
<222> (1)..(18)

<400> 35
Cys Arg Arg Ser Thr Thr Asp Cys Gly Gly Pro Lys Asp His Pro Leu
1               5                   10                  15

Thr Cys



<210> 36
<211> 37
<212> PRT
<213> Artificial Sequence

<220>
<223> Description of Artificial Sequence: peptide
      signalling molecule

<220>
<221> SITE
<222> (1)..(37)

<400> 36
Thr Cys Asp Asp Pro Arg Phe Gln Asp Ser Ser Ser Ser Lys Ala Pro
1               5                   10                  15

Pro Pro Ser Leu Pro Ser Pro Ser Arg Leu Pro Gly Pro Ser Asp Thr
            20                  25                  30

Pro Ile Leu Pro Gln
        35


<210> 37
<211> 10
<212> PRT
<213> Artificial Sequence

<220>
<223> Description of Artificial Sequence: peptide
      signalling molecule

<220>
<221> SITE
<222> (1)..(10)

<400> 37
Leu Gln Gly Val Leu Pro Ala Leu Pro Gln

1                   5                   10

<210> 38
<211> 8
<212> PRT
<213> Artificial Sequence

<220>
<223> Description of Artificial Sequence: peptide
      signalling molecule

<220>
<221> SITE
<222> (1)..(8)

<400> 38
Gly Val Leu Pro Ala Leu Pro Gln
  1               5


<210> 39
<211> 4
<212> PRT
<213> Artificial Sequence

<220>
<223> Description of Artificial Sequence: peptide
      signalling molecule

<220>
<221> SITE
<222> (1)..(4)

<400> 39
Leu Gly Gln Val
  1


<210> 40
<211> 25
<212> DNA
<213> Artificial Sequence

<220>
<223> Description of Artificial Sequence: probe to
      represent the NF-kappaB binding sequence

<220>
<221> misc_feature
<222> (1)..(25)

<400> 40
agctcagagg gggactttcc gagag                                          25


<210> 41
<211> 4
<212> PRT
<213> Artificial Sequence

<220>
<223> Description of Artificial Sequence: peptide LQAV
      showed smaller infarcted area

<220>
<221> SITE
<222> (1)..(4)

<400> 41
Leu Gln Ala Val
1


<210> 42
<211> 10
<212> PRT
<213> Artificial Sequence

<220>
<223> Description of Artificial Sequence: NMPF peptide

<220>
<221> SITE
<222> (1)..(10)

<400> 42
Cys Pro Arg Gly Val Asn Pro Val Val Ser
1               5                   10


<210> 43
<211> 5
<212> PRT
<213> Artificial Sequence

<220>
<223> Description of Artificial Sequence:
      pdb/1DE7/1DE7-A

<220>
<221> SITE
<222> (1)..(5)

<400> 43
Leu Gln Gly Val Val
1               5


<210> 44
<211> 6
<212> PRT
<213> Artificial Sequence

<220>
<223> Description of Artificial Sequence:
      pdb/1DE7/1DE7-A

<220>
<221> SITE
<222> (1)..(6)

<400> 44
Leu Gln Gly Val Val Pro
1               5


<210> 45
<211> 5
<212> PRT
<213> Artificial Sequence

<220>
<223> Description of Artificial Sequence:
      pdb/1DL6/1DL6-A

<220>
<221> SITE
<222> (1)..(5)

<400> 45
Leu Asp Ala Leu Pro
1               5


<210> 46
<211> 4
<212> PRT
<213> Artificial Sequence

<220>
<223> Description of Artificial Sequence:
      pdb/1QMH/1QMH-A

<220>
<221> SITE
<222> (1)..(4)

<400> 46
Leu Gln Thr Val
1


<210> 47
<211> 5
<212> PRT
<213> Artificial Sequence

<220>
<223> Description of Artificial Sequence:
      pdb/1QMH/1QMH-A

<220>
<221> SITE
<222> (1)..(5)

<400> 47
Val Leu Pro Ala Leu
1               5


<210> 48
<211> 10
<212> PRT
<213> Artificial Sequence

<220>
<223> Description of Artificial Sequence:
      pdb/1QMH/1QMH-A

<220>
<221> SITE
<222> (1)..(10)

<400> 48
Leu Val Leu Gln Thr Val Leu Pro Ala Leu
1               5                   10


<210> 49
<211> 3
<212> PRT
<213> Artificial Sequence

<220>
<223> Description of Artificial Sequence: pdb/1LYP/1LYP

<220>
<221> SITE
<222> (1)..(3)

<400> 49
Ile Gln Gly
1


<210> 50
<211> 4
<212> PRT
<213> Artificial Sequence

<220>
<223> Description of Artificial Sequence: pdb/1LYP/1LYP

<220>
<221> SITE
<222> (1)..(4)

<400> 50
Ile Gln Gly Leu
1


<210> 51
<211> 4
<212> PRT
<213> Artificial Sequence

<220>
<223> Description of Artificial Sequence: pdb/1LYP/1LYP

<220>
<221> SITE
<222> (1)..(4)

<400> 51
Leu Pro Lys Leu
1


<210> 52
<211> 5
<212> PRT
<213> Artificial Sequence

<220>
<223> Description of Artificial Sequence: pdb/1LYP/1LYP

<220>
<221> SITE
<222> (1)..(5)

<400> 52
Leu Leu Pro Lys Leu
 1               5


<210> 53
<211> 4
<212> PRT
<213> Artificial Sequence

<220>
<223> Description of Artificial Sequence:
      pdb/1B9O/1B9O-A

<220>
<221> SITE
<222> (1)..(4)

<400> 53
Leu Pro Glu Leu
  1


<210> 54
<211> 4
<212> PRT
<213> Artificial Sequence

<220>
<223> Description of Artificial Sequence:
      pdb/1GLU/1GLU-A

<220>
<221> SITE
<222> (1)..(4)

<400> 54
Pro Ala Arg Pro
  1


<210> 55
<211> 4
<212> PRT
<213> Artificial Sequence

<220>
<223> Description of Artificial Sequence:
      pdb/2KIN/2KIN-B

<220>
<221> SITE
<222> (1)..(4)

<400> 55
Met Thr Arg Ile
  1


<210> 56
<211> 4
<212> PRT
<213> Artificial Sequence

<220>
<223> Description of Artificial Sequence:
      pdb/1SMP/1SMP-I

<220>
<221> SITE
<222> (1)..(4)

<400> 56
Leu Gln Lys Leu
  1


<210> 57

<211> 5
<212> PRT
<213> Artificial Sequence

<220>
<223> Description of Artificial Sequence:
      pdb/1SMP/1SMP-I

<220>
<221> SITE
<222> (1)..(5)

<400> 57
Leu Gln Lys Leu Leu
  1               5


<210> 58
<211> 4
<212> PRT
<213> Artificial Sequence

<220>
<223> Description of Artificial Sequence:
      pdb/1SMP/1SMP-I

<220>
<221> SITE
<222> (1)..(4)

<400> 58
Pro Glu Ala Pro
  1


<210> 59
<211> 9
<212> PRT
<213> Artificial Sequence

<220>
<223> Description of Artificial Sequence:
      pdb/1SMP/1SMP-I

<220>
<221> SITE
<222> (1)..(9)

<400> 59
Leu Gln Lys Leu Leu Pro Glu Ala Pro
  1               5


<210> 60
<211> 3
<212> PRT
<213> Artificial Sequence

<220>
<223> Description of Artificial Sequence: pdb/1ES/1ES7-B

<220>
<221> SITE
<222> (1)..(3)

<400> 60
Leu Pro Gln
  1

<210> 61
<211> 4
<212> PRT
<213> Artificial Sequence

<220>
<223> Description of Artificial Sequence: pdb/1ES/1ES7-B

<220>
<221> SITE
<222> (1)..(4)

<400> 61
Pro Thr Leu Pro
1


<210> 62
<211> 5
<212> PRT
<213> Artificial Sequence

<220>
<223> Description of Artificial Sequence: pdb/1ES/1ES7-B

<220>
<221> SITE
<222> (1)..(5)

<400> 62
Leu Gln Pro Thr Leu
 1               5


<210> 63
<211> 3
<212> PRT
<213> Artificial Sequence

<220>
<223> Description of Artificial Sequence:
      pdb/1BHX/1BHX-F

<220>
<221> SITE
<222> (1)..(3)

<400> 63
Leu Gln Val
1


<210> 64
<211> 4
<212> PRT
<213> Artificial Sequence

<220>
<223> Description of Artificial Sequence:
      pdb/1BHX/1BHX-F

<220>
<221> SITE
<222> (1)..(4)

<400> 64

Leu Gln Val Val
1

<210> 65
<211> 4
<212> PRT
<213> Artificial Sequence

<220>
<223> Description of Artificial Sequence:
pdb/1VCB/1VCB-A

<220>
<221> SITE
<222> (1)..(4)

<400> 65
Pro Glu Leu Pro
1

<210> 66
<211> 4
<212> PRT
<213> Artificial Sequence

<220>
<223> Description of Artificial Sequence:
pdb/1CQK/1CQK-A

<220>
<221> SITE
<222> (1)..(4)

<400> 66
Pro Ala Ala Pro
1

<210> 67
<211> 5
<212> PRT
<213> Artificial Sequence

<220>
<223> Description of Artificial Sequence:
pdb/1CQK/1CQK-A

<220>
<221> SITE
<222> (1)..(5)

<400> 67
Pro Ala Ala Pro Gln
1               5

<210> 68
<211> 6
<212> PRT
<213> Artificial Sequence

<220>
<223> Description of Artificial Sequence:
pdb/1CQK/1CQK-A

<220>

<221> SITE
<222> (1)..(6)

<400> 68
Pro Ala Ala Pro Gln Val
1               5


<210> 69
<211> 4
<212> PRT
<213> Artificial Sequence

<220>
<223> Description of Artificial Sequence: pdb/1BFB/1BFB

<220>
<221> SITE
<222> (1)..(4)

<400> 69
Leu Pro Ala Leu
1


<210> 70
<211> 4
<212> PRT
<213> Artificial Sequence

<220>
<223> Description of Artificial Sequence: pdb/1BFB/1BFB

<220>
<221> SITE
<222> (1)..(4)

<400> 70
Pro Ala Leu Pro
1


<210> 71
<211> 5
<212> PRT
<213> Artificial Sequence

<220>
<223> Description of Artificial Sequence: pdb/1BFB/1BFB

<220>
<221> SITE
<222> (1)..(5)

<400> 71
Pro Ala Leu Pro Glu
1               5


<210> 72
<211> 3
<212> PRT
<213> Artificial Sequence

<220>
<223> Description of Artificial Sequence: pdb/1MBF/1MBF

<220>

<221> SITE
<222> (1)..(3)

<400> 72
Leu Pro Asn
  1


<210> 73
<211> 5
<212> PRT
<213> Artificial Sequence

<220>
<223> Description of Artificial Sequence:
      pdb/1R2A/1R2A-A

<220>
<221> SITE
<222> (1)..(5)

<400> 73
Leu Thr Glu Leu Leu
  1               5


<210> 74
<211> 10
<212> PRT
<213> Artificial Sequence

<220>
<223> Description of Artificial Sequence: C3G peptide

<220>
<221> SITE
<222> (1)..(10)

<400> 74
Pro Pro Pro Ala Leu Pro Pro Lys Lys Arg
  1               5                  10


<210> 75
<211> 4
<212> PRT
<213> Artificial Sequence

<220>
<223> Description of Artificial Sequence:
      pdb/1RLQ/1RLQ-R

<220>
<221> SITE
<222> (1)..(4)

<400> 75
Leu Pro Pro Leu
  1


<210> 76
<211> 4
<212> PRT
<213> Artificial Sequence

<220>
<223> Description of Artificial Sequence:

pdb/1RLQ/1RLQ-R

<220>
<221> SITE
<222> (1)..(4)

<400> 76
Pro Pro Leu Pro
  1


<210> 77
<211> 3
<212> PRT
<213> Artificial Sequence

<220>
<223> Description of Artificial Sequence: pdb/1TNT/1TNT

<220>
<221> SITE
<222> (1)..(3)

<400> 77
Leu Pro Gly
  1


<210> 78
<211> 4
<212> PRT
<213> Artificial Sequence

<220>
<223> Description of Artificial Sequence: pdb/1TNT/1TNT

<220>
<221> SITE
<222> (1)..(4)

<400> 78
Leu Pro Gly Leu
  1


<210> 79
<211> 3
<212> PRT
<213> Artificial Sequence

<220>
<223> Description of Artificial Sequence: pdb/1TNT/1TNT

<220>
<221> SITE
<222> (1)..(3)

<400> 79
Leu Pro Lys
  1


<210> 80
<211> 4
<212> PRT
<213> Artificial Sequence

<220>

<223> Description of Artificial Sequence:
      pdb/1GJS/1GJS-A

<220>
<221> SITE
<222> (1)..(4)

<400> 80
Leu Ala Ala Leu
  1


<210> 81
<211> 5
<212> PRT
<213> Artificial Sequence

<220>
<223> Description of Artificial Sequence:
      pdb/1GJS/1GJS-A

<220>
<221> SITE
<222> (1)..(5)

<400> 81
Leu Ala Ala Leu Pro
  1               5


<210> 82
<211> 4
<212> PRT
<213> Artificial Sequence

<220>
<223> Description of Artificial Sequence:
      pdb/1GBR/1GBR-B

<220>
<221> SITE
<222> (1)..(4)

<400> 82
Pro Lys Leu Pro
  1


<210> 83
<211> 6
<212> PRT
<213> Artificial Sequence

<220>
<223> Description of Artificial Sequence:
      pdb/1A78/1A78-A

<220>
<221> SITE
<222> (1)..(6)

<400> 83
Val Leu Pro Ser Ile Pro
  1               5


<210> 84
<211> 4

<212> PRT
<213> Artificial Sequence

<220>
<223> Description of Artificial Sequence:
      pdb/1FZV/1FZV-A

<220>
<221> SITE
<222> (1)..(4)

<400> 84
Pro Ala Val Pro
  1


<210> 85
<211> 6
<212> PRT
<213> Artificial Sequence

<220>
<223> Description of Artificial Sequence:
      pdb/1FZV/1FZV-A

<220>
<221> SITE
<222> (1)..(6)

<400> 85
Met Leu Pro Ala Val Pro
  1               5


<210> 86
<211> 3
<212> PRT
<213> Artificial Sequence

<220>
<223> Description of Artificial Sequence: pdb/1JLI/1JLI

<220>
<221> SITE
<222> (1)..(3)

<400> 86
Leu Pro Cys
  1


<210> 87
<211> 4
<212> PRT
<213> Artificial Sequence

<220>
<223> Description of Artificial Sequence: pdb/1JLI/1JLI

<220>
<221> SITE
<222> (1)..(4)

<400> 87
Leu Pro Cys Leu
  1

<210> 88
<211> 4
<212> PRT
<213> Artificial Sequence

<220>
<223> Description of Artificial Sequence: pdb/1JLI/1JLI

<220>
<221> SITE
<222> (1)..(4)

<400> 88
Pro Cys Leu Pro
1


<210> 89
<211> 5
<212> PRT
<213> Artificial Sequence

<220>
<223> Description of Artificial Sequence:
      pdb/1HSS/1HSS-A

<220>
<221> SITE
<222> (1)..(5)

<400> 89
Val Pro Ala Leu Pro
1               5


<210> 90
<211> 3
<212> PRT
<213> Artificial Sequence

<220>
<223> Description of Artificial Sequence:
      pdb/3CRX/3CRX-A

<220>
<221> SITE
<222> (1)..(3)

<400> 90
Leu Pro Ala
1


<210> 91
<211> 4
<212> PRT
<213> Artificial Sequence

<220>
<223> Description of Artificial Sequence:
      pdb/1PRX/1PRX-A

<220>
<221> SITE
<222> (1)..(4)

<400> 91
Pro Thr Ile Pro

1

<210> 92
<211> 6
<212> PRT
<213> Artificial Sequence

<220>
<223> Description of Artificial Sequence:
      pdb/1PRX/1PRX-A

<220>
<221> SITE
<222> (1)..(6)

<400> 92
Val Leu Pro Thr Ile Pro
  1               5


<210> 93
<211> 6
<212> PRT
<213> Artificial Sequence

<220>
<223> Description of Artificial Sequence: pdb/1RCY/1RCY

<220>
<221> SITE
<222> (1)..(6)

<400> 93
Val Leu Pro Gly Phe Pro
  1               5


<210> 94
<211> 4
<212> PRT
<213> Artificial Sequence

<220>
<223> Description of Artificial Sequence: pdb/1A3Z/1A3Z

<220>
<221> SITE
<222> (1)..(4)

<400> 94
Pro Gly Phe Pro
  1


<210> 95
<211> 5
<212> PRT
<213> Artificial Sequence

<220>
<223> Description of Artificial Sequence:
      pdb/1GER/1GER-A

<220>
<221> SITE
<222> (1)..(5)

<400> 95
Leu Pro Ala Leu Pro
1               5


<210> 96
<211> 5
<212> PRT
<213> Artificial Sequence

<220>
<223> Description of Artificial Sequence: pdb/1BBS/1BBS

<220>
<221> SITE
<222> (1)..(5)

<400> 96
Met Pro Ala Leu Pro
1               5


<210> 97
<211> 17
<212> PRT
<213> Artificial Sequence

<220>
<223> Description of Artificial Sequence: AI188872

<220>
<221> SITE
<222> (1)..(17)
<223> /note="X on position two stands for unknown"

<400> 97
Met Xaa Arg Val Leu Gln Gly Val Leu Pro Ala Leu Pro Gln Val Val
1               5                   10                  15

Cys



<210> 98
<211> 4
<212> PRT
<213> Artificial Sequence

<220>
<223> Description of Artificial Sequence: AI188872

<220>
<221> SITE
<222> (1)..(4)
<223> /note="X on position two stands for unknown"

<400> 98
Met Xaa Arg Val
1


<210> 99
<211> 3
<212> PRT
<213> Artificial Sequence

<220>
<223> Description of Artificial Sequence: AI188872

<220>
<221> SITE
<222> (1)..(3)
<223> /note="X on position two stands for unkown"

<400> 99
Met Xaa Arg
1


<210> 100
<211> 17
<212> PRT
<213> Artificial Sequence

<220>
<223> Description of Artificial Sequence: AI126906

<220>
<221> SITE
<222> (1)..(17)

<400> 100
Ile Thr Arg Val Met Gln Gly Val Ile Pro Ala Leu Pro Gln Val Val
1               5                   10                  15

Cys



<210> 101
<211> 16
<212> PRT
<213> Artificial Sequence

<220>
<223> Description of Artificial Sequence: AI221581

<220>
<221> SITE
<222> (1)..(16)

<400> 101
Met Thr Arg Val Leu Gln Val Val Leu Leu Ala Leu Pro Gln Leu Val
1               5                   10                  15


<210> 102
<211> 14
<212> PRT
<213> Artificial Sequence

<220>
<223> Description of Artificial Sequence: Mm.42246.3

<220>
<221> SITE
<222> (1)..(14)

<400> 102
Lys Val Ile Gln Gly Ser Leu Asp Ser Leu Pro Gln Ala Val
1               5                   10


<210> 103
<211> 4
<212> PRT

<213> Artificial Sequence

<220>
<223> Description of Artificial Sequence: Mm.42246.3

<220>
<221> SITE
<222> (1)..(4)

<400> 103
Leu Asp Ser Leu
  1


<210> 104
<211> 11
<212> PRT
<213> Artificial Sequence

<220>
<223> Description of Artificial Sequence: Mm.22430.1

<220>
<221> SITE
<222> (1)..(11)

<400> 104
Val Leu Gln Ala Ile Leu Pro Ser Ala Pro Gln
  1               5                  10


<210> 105
<211> 3
<212> PRT
<213> Artificial Sequence

<220>
<223> Description of Artificial Sequence: Mm.22430.1

<220>
<221> SITE
<222> (1)..(3)

<400> 105
Leu Gln Ala
  1


<210> 106
<211> 5
<212> PRT
<213> Artificial Sequence

<220>
<223> Description of Artificial Sequence: Mm.22430.1

<220>
<221> SITE
<222> (1)..(5)

<400> 106
Leu Gln Ala Ile Leu
  1               5


<210> 107
<211> 4
<212> PRT

<213> Artificial Sequence

<220>
<223> Description of Artificial Sequence: Mm.22430.1

<220>
<221> SITE
<222> (1)..(4)

<400> 107
Pro Ser Ala Pro
1


<210> 108
<211> 3
<212> PRT
<213> Artificial Sequence

<220>
<223> Description of Artificial Sequence: Mm.22430.1

<220>
<221> SITE
<222> (1)..(3)

<400> 108
Leu Pro Ser
1


<210> 109
<211> 14
<212> PRT
<213> Artificial Sequence

<220>
<223> Description of Artificial Sequence: Hs.63758.4

<220>
<221> SITE
<222> (1)..(14)

<400> 109
Lys Val Leu Gln Gly Arg Leu Pro Ala Val Ala Gln Ala Val
1               5                   10


<210> 110
<211> 3
<212> PRT
<213> Artificial Sequence

<220>
<223> Description of Artificial Sequence: Hs.63758.4

<220>
<221> SITE
<222> (1)..(3)

<400> 110
Leu Pro Ala
1


<210> 111
<211> 4
<212> PRT

<213> Artificial Sequence

<220>
<223> Description of Artificial Sequence: Hs.63758.4

<220>
<221> SITE
<222> (1)..(4)

<400> 111
Leu Pro Ala Val
  1


<210> 112
<211> 14
<212> PRT
<213> Artificial Sequence

<220>
<223> Description of Artificial Sequence: Mm.129320.2

<220>
<221> SITE
<222> (1)..(14)

<400> 112
Leu Val Gln Lys Val Val Pro Met Leu Pro Arg Leu Leu Cys
  1               5                  10


<210> 113
<211> 3
<212> PRT
<213> Artificial Sequence

<220>
<223> Description of Artificial Sequence: Mm.129320.2

<220>
<221> SITE
<222> (1)..(3)

<400> 113
Leu Val Gln
  1


<210> 114
<211> 4
<212> PRT
<213> Artificial Sequence

<220>
<223> Description of Artificial Sequence: Mm.129320.2

<220>
<221> SITE
<222> (1)..(4)

<400> 114
Leu Pro Arg Leu
  1


<210> 115
<211> 4
<212> PRT

<213> Artificial Sequence

<220>
<223> Description of Artificial Sequence: Mm.129320.2

<220>
<221> SITE
<222> (1)..(4)

<400> 115
Pro Met Leu Pro
  1


<210> 116
<211> 5
<212> PRT
<213> Artificial Sequence

<220>
<223> Description of Artificial Sequence: Mm.22430.1

<220>
<221> SITE
<222> (1)..(5)

<400> 116
Pro Ser Ala Pro Gln
  1               5


<210> 117
<211> 11
<212> PRT
<213> Artificial Sequence

<220>
<223> Description of Artificial Sequence: P20155

<220>
<221> SITE
<222> (1)..(11)

<400> 117
Leu Pro Gly Cys Pro Arg His Phe Asn Pro Val
  1               5                  10


<210> 118
<211> 11
<212> PRT
<213> Artificial Sequence

<220>
<223> Description of Artificial Sequence: Rn.2337.1

<220>
<221> SITE
<222> (1)..(11)

<400> 118
Leu Val Gly Cys Pro Arg Asp Tyr Asp Pro Val
  1               5                  10


<210> 119
<211> 3
<212> PRT

<213> Artificial Sequence

<220>
<223> Description of Artificial Sequence: Rn.2337.1

<220>
<221> SITE
<222> (1)..(3)

<400> 119
Leu Val Gly
  1


<210> 120
<211> 4
<212> PRT
<213> Artificial Sequence

<220>
<223> Description of Artificial Sequence: Rn.2337.1

<220>
<221> SITE
<222> (1)..(4)

<400> 120
Leu Val Gly Cys
  1


<210> 121
<211> 6
<212> PRT
<213> Artificial Sequence

<220>
<223> Description of Artificial Sequence: Hs.297775.1

<220>
<221> SITE
<222> (1)..(6)

<400> 121
Pro Gly Cys Pro Arg Gly
  1               5


<210> 122
<211> 4
<212> PRT
<213> Artificial Sequence

<220>
<223> Description of Artificial Sequence: Hs.297775.1

<220>
<221> SITE
<222> (1)..(4)

<400> 122
Pro Gly Cys Pro
  1


<210> 123
<211> 5
<212> PRT

<213> Artificial Sequence

<220>
<223> Description of Artificial Sequence: Mm.1359.1

<220>
<221> SITE
<222> (1)..(5)

<400> 123
Leu Pro Gly Cys Pro
  1               5


<210> 124
<211> 6
<212> PRT
<213> Artificial Sequence

<220>
<223> Description of Artificial Sequence:
      sptrembl/O56177/O56177

<220>
<221> SITE
<222> (1)..(6)

<400> 124
Val Leu Pro Ala Ala Pro
  1               5


<210> 125
<211> 9
<212> PRT
<213> Artificial Sequence

<220>
<223> Description of Artificial Sequence:
      sptrembl/Q9W234/Q9W234

<220>
<221> SITE
<222> (1)..(9)

<400> 125
Leu Ala Gly Thr Ile Pro Ala Thr Pro
  1               5


<210> 126
<211> 3
<212> PRT
<213> Artificial Sequence

<220>
<223> Description of Artificial Sequence:
      sptrembl/Q9W234/Q9W234

<220>
<221> SITE
<222> (1)..(3)

<400> 126
Leu Ala Gly
  1

<210> 127
<211> 4
<212> PRT
<213> Artificial Sequence

<220>
<223> Description of Artificial Sequence:
      sptrembl/Q9W234/Q9W234

<220>
<221> SITE
<222> (1)..(4)

<400> 127
Pro Ala Thr Pro
  1


<210> 128
<211> 7
<212> PRT
<213> Artificial Sequence

<220>
<223> Description of Artificial Sequence:
      swiss/P81272/NS2B HUMAN

<220>
<221> SITE
<222> (1)..(7)

<400> 128
Gly Val Leu Pro Ala Val Pro
  1               5


<210> 129
<211> 6
<212> PRT
<213> Artificial Sequence

<220>
<223> Description of Artificial Sequence:
      swiss/P81272/NS2B HUMAN

<220>
<221> SITE
<222> (1)..(6)

<400> 129
Val Leu Pro Ala Val Pro
  1               5


<210> 130
<211> 7
<212> PRT
<213> Artificial Sequence

<220>
<223> Description of Artificial Sequence:
      sptrembl/Q9IYZ3/Q9IYZ3

<220>
<221> SITE
<222> (1)..(7)

<400> 130

Gly Leu Leu Pro Cys Leu Pro
1               5


<210> 131
<211> 4
<212> PRT
<213> Artificial Sequence

<220>
<223> Description of Artificial Sequence:
      sptrembl/Q9PVW5/Q9PVW5

<220>
<221> SITE
<222> (1)..(4)

<400> 131
Pro Gly Ala Pro
1


<210> 132
<211> 10
<212> PRT
<213> Artificial Sequence

<220>
<223> Description of Artificial Sequence:
      sptrembl/Q9PVW5/Q9PVW5

<220>
<221> SITE
<222> (1)..(10)

<400> 132
Leu Pro Gln Arg Pro Arg Gly Pro Asn Pro
1               5                   10


<210> 133
<211> 4
<212> PRT
<213> Artificial Sequence

<220>
<223> Description of Artificial Sequence:
      sptrembl/Q9PVW5/Q9PVW5

<220>
<221> SITE
<222> (1)..(4)

<400> 133
Pro Arg Gly Pro
1


<210> 134
<211> 3
<212> PRT
<213> Artificial Sequence

<220>
<223> Description of Artificial Sequence:
      sptrembl/Q9PVW5/Q9PVW5

<220>

<221> SITE
<222> (1)..(3)

<400> 134
Pro Asn Pro
  1


<210> 135
<211> 4
<212> PRT
<213> Artificial Sequence

<220>
<223> Description of Artificial Sequence: Hs.303116.2

<220>
<221> SITE
<222> (1)..(4)

<400> 135
Gly Cys Pro Arg
  1


<210> 136
<211> 6
<212> PRT
<213> Artificial Sequence

<220>
<223> Description of Artificial Sequence:
      pdb/1DU3/1DU3-A

<220>
<221> SITE
<222> (1)..(6)

<400> 136
Gly Cys Pro Arg Gly Met
  1               5


<210> 137
<211> 4
<212> PRT
<213> Artificial Sequence

<220>
<223> Description of Artificial Sequence: pdb/1BIO/1BIO

<220>
<221> SITE
<222> (1)..(4)

<400> 137
Leu Gln His Val
  1


<210> 138
<211> 4
<212> PRT
<213> Artificial Sequence

<220>
<223> Description of Artificial Sequence:
      pdb/4NOS/4NOS-A

<220>
<221> SITE
<222> (1)..(4)

<400> 138
Phe Pro Gly Cys
1


<210> 139
<211> 4
<212> PRT
<213> Artificial Sequence

<220>
<223> Description of Artificial Sequence:
      pdb/1FL7/1FL7-B

<220>
<221> SITE
<222> (1)..(4)

<400> 139
Val Pro Gly Cys
1


<210> 140
<211> 4
<212> PRT
<213> Artificial Sequence

<220>
<223> Description of Artificial Sequence:
      pdb/1HR6/1HR6-A

<220>
<221> SITE
<222> (1)..(4)

<400> 140
Cys Pro Arg Gly
1


<210> 141
<211> 4
<212> PRT
<213> Artificial Sequence

<220>
<223> Description of Artificial Sequence:pdb/1H6/1HR6-A

<220>
<221> SITE
<222> (1)..(4)

<400> 141
Leu Lys Gly Cys
1


<210> 142
<211> 4
<212> PRT
<213> Artificial Sequence

<220>
<223> Description of Artificial Sequence: pdb/1BFA/1BFA

<220>
<221> SITE
<222> (1)..(4)

<400> 142
Pro Pro Gly Pro
1


<210> 143
<211> 8
<212> PRT
<213> Artificial Sequence

<220>
<223> Description of Artificial Sequence: pdb/1BFA/1BFA

<220>
<221> SITE
<222> (1)..(8)

<400> 143
Leu Pro Gly Cys Pro Arg Glu Val
1               5


<210> 144
<211> 4
<212> PRT
<213> Artificial Sequence

<220>
<223> Description of Artificial Sequence: pdb/1BFA/1BFA

<220>
<221> SITE
<222> (1)..(4)

<400> 144
Cys Pro Arg Glu
1


<210> 145
<211> 17
<212> PRT
<213> Artificial Sequence

<220>
<223> Description of Artificial Sequence:
      swissnew/P01229/LSHB HUMAN

<220>
<221> SITE
<222> (1)..(17)

<400> 145
Met Met Arg Val Leu Gln Ala Val Leu Pro Pro Leu Pro Gln Val Val
1               5                   10                  15

Cys


<210> 146

<211> 3
<212> PRT
<213> Artificial Sequence

<220>
<223> Description of Artificial Sequence:
      swissnew/P01229/LSHB HUMAN

<220>
<221> SITE
<222> (1)..(3)

<400> 146
Met Met Arg
  1


<210> 147
<211> 4
<212> PRT
<213> Artificial Sequence

<220>
<223> Description of Artificial Sequence:
      swissnew/P01229/LSHB HUMAN

<220>
<221> SITE
<222> (1)..(4)

<400> 147
Met Met Arg Val
  1


<210> 148
<211> 6
<212> PRT
<213> Artificial Sequence

<220>
<223> Description of Artificial Sequence:
      swissnew/P01229/LSHB HUMAN

<220>
<221> SITE
<222> (1)..(6)

<400> 148
Val Leu Pro Pro Leu Pro
  1               5


<210> 149
<211> 4
<212> PRT
<213> Artificial Sequence

<220>
<223> Description of Artificial Sequence:
      swissnew/P01229/LSHB HUMAN

<220>
<221> SITE
<222> (1)..(4)

<400> 149
Pro Pro Leu Pro

1


<210> 150
<211> 7
<212> PRT
<213> Artificial Sequence

<220>
<223> Description of Artificial Sequence:
      swissnew/P01229/LSHB HUMAN

<220>
<221> SITE
<222> (1)..(7)

<400> 150
Val Leu Pro Pro Leu Pro Gln
  1               5


<210> 151
<211> 7
<212> PRT
<213> Artificial Sequence

<220>
<223> Description of Artificial Sequence:
      swissnew/P01229/LSHB HUMAN

<220>
<221> SITE
<222> (1)..(7)

<400> 151
Ala Val Leu Pro Pro Leu Pro
  1               5


<210> 152
<211> 8
<212> PRT
<213> Artificial Sequence

<220>
<223> Description of Artificial Sequence:
      swissnew/P01229/LSHB HUMAN

<220>
<221> SITE
<222> (1)..(8)

<400> 152
Ala Val Leu Pro Pro Leu Pro Gln
  1               5


<210> 153
<211> 17
<212> PRT
<213> Artificial Sequence

<220>
<223> Description of Artificial Sequence:
      swissnew/P07434/CGHB PAPAN

<220>
<221> SITE

<222> (1)..(17)

<400> 153
Met Met Arg Val Leu Gln Ala Val Leu Pro Pro Val Pro Gln Val Val
1               5                   10                  15

Cys

<210> 154
<211> 4
<212> PRT
<213> Artificial Sequence

<220>
<223> Description of Artificial Sequence:
      swissnew/P07434/CGHB PAPAN

<220>
<221> SITE
<222> (1)..(4)

<400> 154
Leu Gln Ala Gly
1

<210> 155
<211> 6
<212> PRT
<213> Artificial Sequence

<220>
<223> Description of Artificial Sequence:
      swissnew/P07434/CGHB PAPAN

<220>
<221> SITE
<222> (1)..(6)

<400> 155
Val Leu Pro Pro Val Pro
1               5

<210> 156
<211> 7
<212> PRT
<213> Artificial Sequence

<220>
<223> Description of Artificial Sequence:
      swissnew/P07434/CGHB PAPAN

<220>
<221> SITE
<222> (1)..(7)

<400> 156
Val Leu Pro Pro Val Pro Gln
1               5

<210> 157
<211> 7
<212> PRT
<213> Artificial Sequence

<220>
<223> Description of Artificial Sequence:
      swissnew/P07434/CGHB PAPAN

<220>
<221> SITE
<222> (1)..(7)

<400> 157
Ala Val Leu Pro Pro Val Pro
  1               5


<210> 158
<211> 8
<212> PRT
<213> Artificial Sequence

<220>
<223> Description of Artificial Sequence:
      swissnew/P07434/CGHB PAPAN

<220>
<221> SITE
<222> (1)..(8)

<400> 158
Ala Val Leu Pro Pro Val Pro Gln
  1               5


<210> 159
<211> 4
<212> PRT
<213> Artificial Sequence

<220>
<223> Description of Artificial Sequence:
      swissnew/Q28376/TSHB HORSE

<220>
<221> SITE
<222> (1)..(4)

<400> 159
Met Thr Arg Asp
  1


<210> 160
<211> 4
<212> PRT
<213> Artificial Sequence

<220>
<223> Description of Artificial Sequence:
      swissnew/Q28376/TSHB HORSE

<220>
<221> SITE
<222> (1)..(4)

<400> 160
Gln Asp Val Cys
  1

<210> 161
<211> 3
<212> PRT
<213> Artificial Sequence

<220>
<223> Description of Artificial Sequence:
      swissnew/Q28376/TSHB HORSE

<220>
<221> SITE
<222> (1)..(3)

<400> 161
Asp Val Cys
  1


<210> 162
<211> 4
<212> PRT
<213> Artificial Sequence

<220>
<223> Description of Artificial Sequence:
      swissnew/Q28376/TSHB HORSE

<220>
<221> SITE
<222> (1)..(4)

<400> 162
Ile Pro Gly Cys
  1


<210> 163
<211> 5
<212> PRT
<213> Artificial Sequence

<220>
<223> Description of Artificial Sequence:
      sptrembl/Q9Z284/Q9Z284

<220>
<221> SITE
<222> (1)..(5)

<400> 163
Pro Ala Leu Pro Ser
  1               5


<210> 164
<211> 6
<212> PRT
<213> Artificial Sequence

<220>
<223> Description of Artificial Sequence:
      sptrembl/Q9UCG8/Q9UCG8

<220>
<221> SITE
<222> (1)..(6)

<400> 164

Leu Pro Gly Gly Pro Arg
1               5

<210> 165
<211> 4
<212> PRT
<213> Artificial Sequence

<220>
<223> Description of Artificial Sequence:
      sptrembl/Q9UCG8/Q9UCG8

<220>
<221> SITE
<222> (1)..(4)

<400> 165
Leu Pro Gly Gly
1

<210> 166
<211> 4
<212> PRT
<213> Artificial Sequence

<220>
<223> Description of Artificial Sequence:
      sptrembl/Q9UCG8/Q9UCG8

<220>
<221> SITE
<222> (1)..(4)

<400> 166
Gly Gly Pro Arg
1

<210> 167
<211> 4
<212> PRT
<213> Artificial Sequence

<220>
<223> Description of Artificial Sequence: XP_028754

<220>
<221> SITE
<222> (1)..(4)

<400> 167
Leu Gln Arg Gly
1

<210> 168
<211> 5
<212> PRT
<213> Artificial Sequence

<220>
<223> Description of Artificial Sequence: XP_028754

<220>
<221> SITE
<222> (1)..(5)

<400> 168
Leu Gln Arg Gly Val
1               5


<210> 169
<211> 4
<212> PRT
<213> Artificial Sequence

<220>
<223> Description of Artificial Sequence: XP_028754

<220>
<221> SITE
<222> (1)..(4)

<400> 169
Leu Gly Gln Leu
1


<210> 170
<211> 13
<212> PRT
<213> Artificial Sequence

<220>
<223> Description of Artificial Sequence: SignalP (CBS)

<220>
<221> SITE
<222> (1)..(13)

<400> 170
Met Thr Arg Val Leu Gln Gly Val Leu Pro Ala Leu Pro
1               5                   10


<210> 171
<211> 9
<212> PRT
<213> Artificial Sequence

<220>
<223> Description of Artificial Sequence: HLA molecule
      type I (A_0201)

<220>
<221> SITE
<222> (1)..(9)

<400> 171
Val Leu Gln Gly Val Leu Pro Ala Leu
1               5


<210> 172
<211> 9
<212> PRT
<213> Artificial Sequence

<220>
<223> Description of Artificial Sequence: HLA molecule
      type I (A_0201)

<220>

<221> SITE
<222> (1)..(9)

<400> 172
Gly Val Leu Pro Ala Leu Pro Gln Val
1               5


<210> 173
<211> 9
<212> PRT
<213> Artificial Sequence

<220>
<223> Description of Artificial Sequence: HLA molecule
      type I (A_0201)

<220>
<221> SITE
<222> (1)..(9)

<400> 173
Val Leu Pro Ala Leu Pro Gln Val Val
1               5


<210> 174
<211> 9
<212> PRT
<213> Artificial Sequence

<220>
<223> Description of Artificial Sequence: HLA molecule
      type I (A_0201)

<220>
<221> SITE
<222> (1)..(9)

<400> 174
Arg Leu Pro Gly Cys Pro Arg Gly Val
1               5


<210> 175
<211> 9
<212> PRT
<213> Artificial Sequence

<220>
<223> Description of Artificial Sequence: HLA molecule
      type I (A_0201)

<220>
<221> SITE
<222> (1)..(9)

<400> 175
Thr Met Thr Arg Val Leu Gln Gly Val
1               5


<210> 176
<211> 15
<212> PRT
<213> Artificial Sequence

<220>

<223> Description of Artificial Sequence: MHC II (H2-Ak
      15-mers)

<220>
<221> SITE
<222> (1)..(15)

<400> 176
Cys Pro Thr Met Thr Arg Val Leu Gln Gly Val Leu Pro Ala Leu
1               5                   10                  15


<210> 177
<211> 15
<212> PRT
<213> Artificial Sequence

<220>
<223> Description of Artificial Sequence: MHC II (H2-Ak
      15-mers)

<220>
<221> SITE
<222> (1)..(15)

<400> 177
Pro Gly Cys Pro Arg Gly Val Asn Pro Val Val Ser Tyr Ala Val
1               5                   10                  15


<210> 178
<211> 15
<212> PRT
<213> Artificial Sequence

<220>
<223> Description of Artificial Sequence: HLA-DRB1*0101
      15-mers

<220>
<221> SITE
<222> (1)..(15)

<400> 178
Pro Arg Gly Val Asn Pro Val Val Ser Tyr Ala Val Ala Leu Ser
1               5                   10                  15


<210> 179
<211> 15
<212> PRT
<213> Artificial Sequence

<220>
<223> Description of Artificial Sequence: HLA-DRB1*0101
      15-mers

<220>
<221> SITE
<222> (1)..(15)

<220>
<221> SITE
<222> (1)..(15)

<400> 179
Thr Arg Val Leu Gln Gly Val Leu Pro Ala Leu Pro Gln Val Val
1               5                   10                  15

<210> 180
<211> 15
<212> PRT
<213> Artificial Sequence

<220>
<223> Description of Artificial Sequence: HLA-DRB1*0101
      15-mers

<220>
<221> SITE
<222> (1)..(15)

<400> 180
Leu Gln Gly Val Leu Pro Ala Leu Pro Gln Val Val Cys Asn Tyr
  1               5                  10                  15


<210> 181
<211> 15
<212> PRT
<213> Artificial Sequence

<220>
<223> Description of Artificial Sequence: HLA-DRB1*0301
      (DR17) 15-mers

<220>
<221> SITE
<222> (1)..(15)

<400> 181
Met Thr Arg Val Leu Gln Gly Val Leu Pro Ala Leu Pro Gln Val
  1               5                  10                  15


<210> 182
<211> 15
<212> PRT
<213> Artificial Sequence

<220>
<223> Description of Artificial Sequence: HLA-DRB1*0301
      (DR17) 15-mers

<220>
<221> SITE
<222> (1)..(15)

<400> 182
Ser Ile Arg Leu Pro Gly Cys Pro Arg Gly Val Asn Pro Val Val
  1               5                  10                  15


<210> 183
<211> 7
<212> PRT
<213> Artificial Sequence

<220>
<223> Description of Artificial Sequence: NMPF-56
      peptide

<220>
<221> SITE
<222> (1)..(7)

<400> 183
Val Ala Pro Ala Leu Pro Gln
1               5


<210> 184
<211> 35
<212> PRT
<213> Artificial Sequence

<220>
<223> Description of Artificial Sequence: NMPF-62
      peptide

<220>
<221> SITE
<222> (1)..(35)

<400> 184
Val Val Cys Asn Tyr Arg Asp Val Arg Phe Glu Ser Ile Arg Leu Pro
1               5                   10                  15

Gly Cys Pro Arg Gly Val Asn Pro Val Val Ser Tyr Ala Val Ala Leu
            20                  25                  30

Ser Cys Gln
        35


<210> 185
<211> 7
<212> PRT
<213> Artificial Sequence

<220>
<223> Description of Artificial Sequence: NMPF-67
      peptide

<220>
<221> SITE
<222> (1)..(7)

<400> 185
Cys Pro Arg Gly Val Asn Pro
1               5


<210> 186
<211> 14
<212> PRT
<213> Artificial Sequence

<220>
<223> Description of Artificial Sequence: NMPF-70
      peptide

<220>
<221> SITE
<222> (1)..(14)

<400> 186
Met Thr Arg Val Leu Gln Gly Val Leu Pro Ala Leu Pro Gln
1               5                   10


<210> 187
<211> 18

<212> PRT
<213> Artificial Sequence

<220>
<223> Description of Artificial Sequence: NMPF-75
      peptide

<220>
<221> SITE
<222> (1)..(18)

<400> 187
Ser Lys Ala Pro Pro Pro Ser Leu Pro Ser Pro Ser Arg Leu Pro Gly
  1               5                  10                  15

Pro Cys

## Description

The invention relates to the modulation of gene expression in a cell, also called gene control, either or not in relation to the treatment of a variety of diseases.

Gene control is generally thought to occur at four levels: 1) transcription (either initiation or termination), 2) processing of primary transcripts, 3) stabilization or destabilization of mRNAs, and 4) mRNA translation. The primary function of gene control in cells is to adjust the enzymatic machinery of the cell to its nutritional, chemical and physical environment.

It is generally thought that gene expression is regulated at both the level of transcription and translation. Modulation or regulation of gene expression requires factors called transcriptional factors. The term gene control or regulation also refers to the formation and use of mRNA. Although control can be exerted at a number of different molecular steps, differential transcription probably most frequently underlies the differential rate of protein synthesis in prokayotes as well as eukaryotes. It is generally thought that activator proteins (also called transcription factors or transcriptional activators) bind to DNA and recruit the transcriptional machinery in a cell to a promotor, thereby stimulating gene expression. Further, differential processing of RNA transcripts in the cell nucleus, differential stabilization of mRNA in the cytoplasm, and differential translation of mRNA into protein are also important in eukaryotic gene control. These steps define the regulatory decisions in a transcriptional circuit and misregulation at any stage can result in a variety of diseases.

Where in unicellular organisms, be it of prokaryotic or eukaryotic origin, a cell' s response to its environment is influenced by many stimuli from the outside world, reflecting the often widely variable environment of the single cell, most cells in multicellular organisms experience a fairly constant environment. Perhaps for this reason, genes that are devoted to responses to environmental changes constitute a much smaller fraction of the total number of genes in multicellular organisms than in single-cell organisms.

As said above, cells react to environmental changes, which they perceive through extracellular signals. These signals can be either physical (e.g. light, temperature, pressure and electricity) or chemical (e.g. food, hormones and neurotransmitters). Cells can both sense and produce signals. This makes it possible that they communicate with each other. In order to achieve this, there are complex signal-sensing and -producing mechanisms in uni- and multicellular organisms.

Two groups of chemical signals can be distinguished: membrane permeable and membrane-impermeable signals. The membrane-permeable signal molecules comprise the large family of steroid hormones, such as estrogens, progesterone and androgens. Steroids pass the plasma membrane and bind to specific receptors, which are localized in the cytoplasm or nucleus of the cell. After binding of the hormone, the receptor undergoes a conformational change. The receptor is then able to bind to DNA itself or to proteins which can in turn interact with DNA. In general, steroid hormones can directly regulate gene expression by means of this process. The membrane-impermeable signal molecules include acetylcholine, growth factors, extracellular matrix components, thrombin, lysophosphatidic acid, the yeast mating factors and, for the social amoeba *Dictyostellium discoideum,* folic acid and cyclic AMP. They are recognized by receptors, which are localized on the plasma membrane of the cell. The receptors are specific for one particular signal molecule or a family of closely related signal molecules. Upon binding of their ligands, these receptors transduce the signals by several mechanisms.

The most characteristic and exacting requirement of gene control on multicellular organisms is the execution of precise developmental decisions so that the right gene is activated in the right cell at the right time. These developmental decisions include not only those related to the development of an organism perse, as for example can be seen during embryogenesis and organogenesis ,or in response to disease but also relate to the differentiation or proliferation or apoptosis of those cells that merely carry out their genetic program essentially without leaving progeny behind.

Such cells , such as skin cells, red blood cells, lens cells of the eye, antibody-producing cells, are also often regulated by patterns of gene control that serve the need of the whole organism and not the survival of an individual cell..

It is generally reasoned that there are at least three components of gene control: molecular signals, levels and mechanisms. Firstly, it is reasoned that specific signalling molecules exist to which a specific gene can respond. Secondly, control is exerted on one or more levels (i.e. the step or steps) in the chain of events leading from the transcription of DNA to the use of mRNA in protein synthesis. Thirdly, at each of those levels, specific molecular mechanisms are employed to finally exert the control over the gene to be expressed.

Many genes are regulated not by a signalling molecule that enters the cells but by molecules that bind to specific receptors on the surface of cells. Interaction between cell-surface receptors and their ligands can be followed by a cascade of intracellular events including variations in the intracellular levels of so-called second messengers (diacylglycerol, Ca²⁺, cyclic nucleotides). The second messengers in turn lead to changes in protein phosphorylation through the action of cyclic AMP, cyclic GMP, calcium-activated protein kinases, or protein kinase C, which is activated by diaglycerol.

Many of the responses to binding of ligands to cell-surface receptors are cytoplasmatic and do not involve immediate gene activation in the nucleus. Some receptor-ligand interactions, however, are known to cause prompt nuclear transcriptional activation of a specific and limited set of genes. For example, one proto-oncogene, *c-fos*, is known to be activated in some cell types by elevation of almost every one of the known second messengers and also by at least two growth factors , platelet-derived growth factor and epidermal growth factor. However, progress has been slow in determining exactly how such activation is achieved. In a few cases, the transcriptional proteins that respond to cell-surface signals have been characterized.

One of the clearest examples of activation of a pre-existing inactive transcription factor following a cell-surface interaction is the nuclear factor (NF)-kappaB, which was originally detected because it stimulates the transcription of genes encoding immunoglobulins of the kappa class in B-lymphocytes. The binding site for NK-kappaB in the kappa gene is well defined (see for example P.A. Baeuerle and D.Baltimore, 1988, Science 242:540), providing an assay for the presence of the active factor. This factor exists in the cytoplasm of lymphocytes complexed with an inhibitor. Treatment of the isolated complex in vitro with mild denaturing conditions dissociates the complex, thus freeing NK-kappaB to bind to its DNA site. Release of active NF-kappaB in cells is now known to occur after a variety of stimuli including treating cells with bacterial lipopolysaccharide (LPS) and extracellular polypeptides as well as chemical molecules (e.g. phobol esters) that stimulate intracellular phosphokinases. Thus a phosphorylation event triggered by many possible stimuli may account for NF-kappaB conversion to the active state. The active factor is then translocated to the cell nucleus to stimulate transcription only of genes with a binding site for active NF-kappaB. Considering that NF-kappaB is thought by many to be a primary effector of disease (A.S. Baldwin, J. Clin. Invest., 2001, 107:3-6), numerous efforts are underway to develop safe inhibitors of NF-kappaB to be used in treatment of both chronic and acute disease situations. Specific inhibitors of NF-kappaB should reduce side effects associated with drugs such as NSAIDS and glucocorticoids and would offer significant potential for the treatment of a variety of human and animal diseases. Specific diseases or syndromes where patients would benefit from NF-kappaB inhibition vary widely, and range from rheumatoid artrhritis, athereosclerosis, multiple sclerosis, chronic inflammatory demyelinating polyradiculoneuritis, asthma, inflammatory bowel disease, to Helicobacter pylori-associated gastritis and other inflammatory responses, and a variety of drugs that have effects of NF-kappaB activity, such as corticosteroids, sulfasalazine, 5-aminosalicylic acid, aspirin, tepoxalin, leflunomide, curcumin, antioxidants and proteasome inhibitors.These drugs are considered to be nonspecific and often only applicable in high concentrations that may end up toxic for the individual treated.

Inactive cytoplasmatic forms of transcription factors can thus be activated by removal of an inhibitor, as is the case with NF-kapppaB, or alternatively, by association of two (or more) proteins neither of which is active by itself as in the case of interferon-alpha-stimulated factor (D.E. Levy et al., 1989, Genes and Development 3:1362). After interferon-alpha attaches to its cell-surface receptor, one of the proteins is changed within a minute or less, and the two can combine. The active (combined) factor is then translocated to the cell nucleus to stimulate transcription only of genes with a binding site for the protein. Considering that interferon-alpha is a mediator of responses of the body directed at pathogens and self-antigens, modulating regulation of genes that are under influence of the interferon-alpha-stimulated factor would contribute to the treatment of a variety of human and animal diseases.

Other typical examples of signalling molecules that affect gene expression via cell-surface receptor interaction are polypeptide hormones such as insuline, glucagon, various growth factors such as EGF, VEGF, and so on.

The steroid hormones and their receptors represent one of the best understood cases that affect transcription. Because steroid hormones are soluble in lipid membranes, they can diffuse into cells. They affect transcription by binding to specific intracellular receptors that are site specific DNA-binding molecules. Other examples of signalling molecules that enter the cell and act intra-cellularly are thyroid hormone (T₃), vitamine D and retinoic acid, other small lipid-soluble signalling molecules that enter cells and modulate gene expression. The characteristic DNA-binding sites for the receptors for these signalling molecules are also known as response elements.

Another example of a small molecule that is involved in regulation of gene expression is ethylene, a gas that for example induces the expression of genes involved in fruit ripening. Also, small plant hormones, known as auxines and cytokinins regulate plant growth and differentiation directly by regulating gene expression.

Given the critical role of regulatory factors in gene regulation, the development of artificial or synthetic counterparts that could be used in methods to rectify errors in gene expression has been a long standing goal at the interface of chemistry and biology.

The invention provides a method for modulating expression of a gene in a cell comprising providing said cell with a signalling molecule comprising an oligopeptide or functional analogue or derivative thereof. Such a signalling molecule is herein also called NMPF and referenced by number. Since peptides, and functional analogues and derivatives of relatively short amino acid sequences, are easily synthesised these days, the invention provides a method to modulate gene expression with easily obtainable synthetic compounds such as synthetic peptides or functional analogues or derivatives thereof..

A functional analogue or derivative of a peptide is defined as an amino acid sequence, or other sequence monomers, which has been altered such that the functional properties of said sequence are essentially the same in kind, not necessarily in amount. An analogue or derivative can be provided in many ways, for instance through conservative amino acid substitution. Also peptidomimetic compounds can be designed that functionally or structurally resemble the original peptide taken as starting point but that are for example composed of non-naturally occurring amino acids or polyamides. With conservative amino acid substitution, one amino acid residue is substituted with another residue with generally similar properties (size, hydrophobicity, etc), such that the overall functioning is likely not to be seriously affected. However, it is often much more desirable to improve a specific function. A derivative can also be provided by systematically improving at least one desired property of an amino acid sequence. This can for instance be done by an Ala-scan and/or replacement net mapping method. With these methods, many different peptides are generated, based on an original amino acid sequence but each containing a substitution of at least one amino acid residue. Said amino acid residue may either be replaced by alanine (Ala-scan) or by any other amino acid residue (replacement net mapping). This way, many positional variants of said original amino acid sequence are synthesized. Every positional variant is screened for a specific activity. The generated data are used to design improved peptide derivatives of a certain amino acid sequence.

A derivative or analogue can also for instance be generated by substitution of an L-amino acid residue with a D-amino acid residue. This substitution, leading to a peptide which does not naturally occur in nature, can improve a property of an amino acid sequence. It is for example useful to provide a peptide sequence of known activity of all D-amino acids in retro inversion format, thereby allowing for retained activity and increase half-life values. By generating many positional variants of an original amino acid sequence and screening for a specific activity, improved peptide derivatives comprising such D-amino acids can be designed with further improved characteristics.

A person skilled in the art is well able to generate analogous compounds of an amino acid sequence. This can for instance be done through screening of a peptide library. Such an analogue has essentially the same functional properties of said sequence in kind, not necessarily in amount. Also, peptides or analogues can be circularised (for example by providing them with (terminal) cysteines, dimerised or multimerised, for example by linkage to lysine or cystein or other compounds with side-chains that allow linkage or multimerisation, brought in tandem- or repeat-configuration, conjugated or otherwise linked to carriers known in the art, if only by a labile link that allows dissociation.

The invention also provides a signalling molecule for modulating expression of a gene in a cell comprising a small peptide or functional analogue or derivative thereof. Surprisingly, the inventors found that a small peptide acts as a signalling molecule that can modulate gene expression. A functional analogue or derivative of a small peptide that acts as such a signalling molecule for modulating expression of a gene in a cell can be identified or obtained by at least one of various methods for finding such a signalling molecule as provided herein.

For example, one method as provided herein for identifying or obtaining a signalling molecule comprising a peptide or functional derivative or analogue thereof capable of modulating expression of a gene in a cell comprises providing said cell with a peptide or derivative or analogue thereof and determining the activity and/or nuclear translocation of a gene transcription factor. Such activity can be determined in various ways using means and/or methods honed to the specific transcription factor under study. In the detailed description, it is provided to study NF-kappaB/Rel protein translocation and/or activity, but it is of course also easily possible to study translocation and/or activity of any other transcription factor for which such tools are available or can be designed. One such other transcription factor is for example the interferon-alpha-stimulated factor as discussed above.). Other useful transcription factors to study in this context comprise c-Jun, ATF-2, Fos, and their complexes, ELK-1, EGR-1, IRF-1, IRF-3/7, AP-1, NF-AT, C/EBPs, Sp1, CREB, PPARgamma, to name a few.

To allow for improved bio-availability of such a signalling molecule (which is a pharmacon, especially when produced artificially, the invention also provides a method for determining whether a small peptide or derivative or analogue thereof can act as a functional signalling molecule according to the invention, said method further comprising determining whether said signalling molecule is membrane-permeable. Another method provided herein for identifying or obtaining a signalling molecule comprising a peptide or functional derivative or analogue thereof capable of modulating expression of a gene in a cell comprises providing said cell with a peptide or derivative or analogue thereof and determining relative up-regulation and/or down-regulation of at least one gene expressed in said cell. Said up-regulation can classically be studied by determining via for example Northern or Western blotting or nucleic acid detection by PCR or immunological detection of proteins whether a cell or cells make more (in the case of up-regulation) or less (in the case of down-regulation) of a gene expression product such as mRNA or protein after the cell or cells have been provided with said peptide or derivative or analogue thereof. Of course, various methods of the invention can be combined to better analyse the functional analogue of the peptide or derivative or analogue under study. Furthermore, relative up-regulation and/or down-regulation of a multitude or clusters of genes expressed in said cell can be easily studied as well, using libraries of positionally or spatially addressable predetermined or known relevant nucleic acid sequences or unique fragments thereof bound to an array or brought in an array format, using for example a nucleic acid library or so-called gene chip expression analysis systems. Lysates of cells or lysates of cytoplasma and/or nuclei of cells that have been provided with said peptide or derivative or analogue under study are than contacted with said library and relative binding of for example mRNA to individual nucleic acids of said library is than determined, as further described herein in the detailed description.

A functional analogue or derivative of a small peptide that can act as a signalling molecule for modulating expression of a gene in a cell can also be identified or obtained by method for identifying or obtaining a signalling molecule comprising an oligopeptide or functional derivative or analogue thereof capable of modulating expression of a gene in a cell comprising providing a peptide or derivative or analogue thereof and determining binding of said peptide or derivative or analogue thereof to a factor related to gene control. Such a factor related to gene control can be any factor related to transcription (either initiation or termination), processing of primary transcripts, stabilization or destabilization of mRNAs, and mRNA translation.

Binding of a peptide or derivative or analogue thereof to such a factor can be determined by various methods known in the art. Classically, peptides or derivatives or analogues can be (radioactively) labelled and binding to said factor can be determined by detection of a labelled peptide-factor complex, such as by electrophoresis, or other separation methods known in the art. However, for determining binding to such factors, array techniques, such as used with peptide libraries, can also be employed, comprising providing a multitude of peptides or derivatives or analogues thereof and determining binding of at least one of said peptides or derivatives or analogues thereof to a factor related to gene control.

In a preferred embodiment, said factor related to gene control comprises a transcription factor, such as a NF-kappaB-Rel protein or another transcription factor desired to be studied. When binding of a functional analogue according to the invention to such factor has been established, it is of course possible to further analyse said analogue by providing a cell with said peptide or derivative or analogue thereof and determining the activity and/or nuclear translocation of a gene transcription factor in said cell, and/or by providing a cell with said peptide or derivative or analogue thereof and determining relative up-regulation and/or down-regulation of at least one gene expressed in said cell.

The invention thus provides a signalling molecule useful in modulating expression of a gene in a cell and identifiable or obtainable by employing a method according to the invention. Useful examples of such a signalling molecule can be selected from the group of oligopeptides LQG, AQG, LQGV, AQGV, LQGA, VLPALPQVVC, VLPALP, ALPALP, VAPALP, ALPALPQ, VLPAAPQ, VLPALAQ, LAGV, VLAALP, VLAALP, VLPALA, VLPALPQ, VLAALPQ, VLPALPA, GVLPALP, LQGVLPALPQVVC, VVCNYRDVRFESIRLPGCPRGVNPVVSYAVALSCQCAL, RPRCRPINATLAVEK, EGCPVCITVNTTICAGYCPT, SKAPPPSLPSPSRLPGPS, SIRLPGCPRGVNPVVS, LPGCPRGVNPVVS, LPGC, MTRV, MTR, VVC, QVVC and functional analogues or derivatives thereof.

A preferred size of a signalling molecule according to the invention is at most 30 - 40 amino acids, although much smaller molecules, in particular of oligopeptide size, have been shown to be particularly effective. Surprisingly, the invention provides here the insight that gene expression can be modulated or regulated by small peptides, that are most likely breakdown products of larger polypeptides such as chorionic gonadotrophin (CG) and growth hormones or growth factors such as fibroblast growth factor, EGF, VEGF, RNA 3'terminal phosphate cyclase and CAP18. In principle such regulating peptide sequences can be derived from a part of any protein of polypeptide molecule produced by prokaryotic and/or eukaryotic cells, and the invention provides the insight that breakdown products of polypeptides, preferably oligopeptides at about the sizes as provided herein, are naturally involved as signalling molecule in modulation of gene expression. In particular, as signalling molecule a (synthetic) peptide is provided obtainable or derivable from beta-human chorionic gonadotrophin (beta-HCG), preferably from nicked beta-HCG. It was thought before that breakdown products of nicked-beta hCG were involved in immuno-modulation (WO99/59671) or in the treatment of wasting syndrome (WO97/49721) but a relationship with modulation of gene expression was not forwarded in these publications. Of course, such an oligopeptide, or functional equivalent or derivative thereof is likely obtainable or derivable from other proteins that are subject to breakdown or proteolysis and that are close to a gene regulatory cascade. Preferably, said peptide signalling molecule is obtained from a peptide having at least 10 amino acids such as a peptide having an amino acid sequence MTRVLQGVLPALPQVVC, SIRLPGCPRGVNPVVS, VVCNYRDVRFESIRLPGCPRGVNPVVSYAVALSCQCAL, RPRCRPINATLAVEKEGCPVCITVNTTICAGYCPT, CALCRRSTTDCGGPKDHPLTC, SKAPPPSLPSPSRLPGPS, CRRSTTDCGGPKDHPLTC, TCDDPRFQDSSSSKAPPPSLPSPSRLPGPSDTPILPQ or functional fragment (e.g. a breakdown product) or functional analogue thereof. Functional analogue herein relates to the signalling molecular effect or activity as for example can be measured by measuring nuclear translocation of a relevant transcription factor, such as NF-kappaB in an NF-kappaB assay, or AP-1 in an AP-1 assay, or by another method as provided herein. Fragments can be somewhat (i.e. 1 or 2 amino acids) smaller or larger on one or both sides, while still providing functional activity.

Not wishing to be bound by theory, it is postulated herein that an unexpected mode of gene regulation has been uncovered. Polypeptides, such as endogenous CG, EGF etc, but also polypeptides of pathogens such as viral, bacterial or protozoal polypeptides, are subject to breakdown into distinct oligopeptides, for example by intracellular proteolysis. Distinct proteolytic enzymes are widely available in the cell, for example in eukaryotes in the lysosomal or proteasomal system. Some of the resulting breakdown products are oligopeptides of 3 to 15, preferably 4 to 9, most preferably 4 to 6 amino acids long that are surprisingly not without any function or effect to the cell, but as demonstrated herein may be involved, possibly via a feedback mechanism in the case of breakdown of endogenous polypeptides, as signalling molecules in the regulation of gene expression, as demonstrated herein by the regulation of the activity or translocation of a gene transcription factor such as NF-kappaB by for example peptide LQGV, VLPALPQVVC, LQGVLPALPQ, LQG, GVLPALPQ, VLPALP, VLPALPQ, GVLPALP, VVC, MTRV, MTR. Synthetic versions of these oligopeptides as described above, and functional analogues or derivatives of these breakdown products are herein provided to modulate gene expression in a cell and be used in methods to rectify errors in gene expression or the treatment of disease. Oligopeptides such as LQG, AQG, LQGV, AQGV, LQGA, VLPALP, ALPALP, VAPALP, ALPALPQ, VLPAAPQ, VLPALAQ, LAGV, VLAALP, VLAALP, VLPALA, VLPALPQ, VLAALPQ, VLPALPA, GVLPALP, LQGVLPALPQVVC, SIRLPGCPRGVNPVVS, SKAPPPSLPSPSRLPGPS, LPGCPRGVNPVVS, LPGC, MTRV, MTR, VVC, or functional analogues thereof, are particularly effective.

By using the insight as expressed herein, in a preferred embodiment, the invention provides a method for modulating expression of a gene in a cell comprising providing said cell with a signalling molecule comprising an oligopeptide or functional analogue or derivative thereof wherein said signalling molecule is membrane-permeable in that it enters said cell. Most small peptides as described herein have already an inherent propensity to become intracellularly involved, but signalling molecules as provided herein can also be provided with additional peptide sequences, such as arginine or lysine rich stretches of amino acids, that allow for improved internalisation across a lipid bilayer membrane, and may possibly be cleaved off later by internal proteolytic activity.

In a preferred embodiment, the invention provides a method for modulating expression of a gene in a cell comprising providing said cell with a signalling molecule comprising a small peptide (amino acid sequence) or functional analogue or derivative thereof, wherein said signalling molecule modulates NF-kappaB/Rel protein conversion or translocation. As said, NF-κB was originally identified as a gene transcription factor that bound to an enhancer element in the gene for the Igκ light chain and was believed to be B cell-specific. However, subsequent studies revealed that NF-kappaB/Rel proteins are ubiquitously expressed and play a central role as transcription factor in regulating the expression of many genes, particularly those involved in immune, inflammatory, developmental and apoptotic processes. NF-κB related gene transcription factors can be activated by different stimuli such as microbial products, proinflammatory cytokines, T- and B-cell mitogens, and physical and chemical stresses. NF-κB in turn regulates the inducible expression of many cytokines, chemokines, adhesion molecules, acute phase proteins, and antimicrobial peptides.

NF-κB represents a group of structurally related and evolutionarily conserved gene transcription factors. So far, five mammalian NF-κB proteins named Rel (c-Rel), RelA (p65), RelB, NF-κB (p50 and its precursor p105), and NF-κB (p52 and it precursor p100) have been described. NF-κB proteins can exist as homo- or heterodimers, and although most NF-κB dimers are activators of transcription, the p50/p50 and p52/p52 homodimers often repress the transcription of their target genes. In Drosophila, three NF-κB homologs named Dorsal, Dif, and Relish have been identified and characterized. Structurally, all NF-κB/Rel proteins share a highly conserved NH₂-terminal Rel homology domain (RHD) that is responsible for DNA binding, dimerization, and association with inhibitory proteins known as IκBs. In resting cells, NF-κB/Rel dimers are bound to IκBs and retained in an inactive form in the cytoplasm. Like NF-κB, IkBs are also members of a multigene family containing seven known mammalian members including IκBα, IκBβ, Iκbγ, IκBε, Bcl-3, the precursor Rel-proteins, p100, and p105, and one *Drosophila* IκB named Cactus. The IκB family is characterized by the presence of multiple copies of ankyrin repeats, which are protein-protein interaction motifs that interact with NF-κB via the RHD. Upon appropriate stimulation, IκB is phosphorylated by IκB kinases (IKKs), polyubiquitinated by a ubiquitin ligase complex, and degraded by the 26S proteosome. Consequently, NF-κB is released and translocates into the nucleus to initiate gene expression.

NF-κB related transcription factors regulate the expression of a wide variety of genes that play critical roles in innate immune responses. Such NF-κB target genes include those encoding cytokines (e.g., IL-1, IL-2, IL-6, IL-12, TNF-α, LTα, LTβ, and GM-CSF), adhesion molecules (e.g., ICAM, VCAM, endothelial leukocyte adhesion molecule [ELAM]), acute phase proteins (e.g., SAA), and inducible enzymes (e.g., iNOS and COX-2). In addition, it has been demonstrated recently that several evolutionary conserved antimicrobial peptides, e.g., β-defensins, are also regulated by NF-κB, a situation similar to *Drosophila..* Besides regulating the expression of molecules involved in innate immunity, NF-κB also plays a role in the expression of molecules important for adaptive immunity, such as MHC proteins, and the expression of critical cytokines such as IL-2, IL-12 and IFN-γ. Finally NF-κB plays an important role in the overall immune response by affecting the expression of genes that are critical for regulating the apoptotic process, such as c-IAP-1 and c-IAP-2, Fas ligand, c-myc, p53, and cyclin D1.

Under normal conditions, NF-kappaB is rapidly activated upon microbial and viral invasion, and this activation usually correlates with resistance of the host to infection. However, persistent activation of NF-kappaB may lead to the production of excessive amounts of pro-inflammatory mediators such as IL-12 and TNF-alpha, resulting in tissue damage, as in insulin dependent diabetis mellitus, atherosclerosis, Crohn's disease, organ failure, and even death of the host, as in bacterial infection-induced septic shock. It is interesting to note that in order to survive in the host, certain pathogens, such as Bordetella, Yersinia, Toxoplasma gondii and African Swine Fever Virus have evolved mechanisms to counteract or escape the host system by inhibiting NF-kappaB activation. On the other hand, some viruses, including HIV-1, CMV and SV-40 take advantage of NF-kappaB as a host factor that is activated at sites of infection.

In short, the invention surprisingly provides a signalling molecule capable of modulating expression of a gene in a cell, said molecule being a short peptide, preferably of at most 30 amino acids long or a functional analogue or derivative thereof. In a much preferred embodiment, said peptide is an oligopeptide of from about 3 to at about 15 amino acids long, preferably 4 to 12, more preferably 4 to 9, most preferably 4 to 6 amino acids long, or a functional analogue or derivative thereof. Of course, such signalling molecule can be longer, for example by extending it (N- and/or C-terminally, with more amino acids or other side groups, which can for example be (enzymatically) cleaved off when the molecule enters the place of final destination. Such extension may even be preferable to prevent the signalling molecule from becoming active in an untimely fashion, however, the core or active fragment of said molecule comprises the aforementioned oligopeptide or analogue or derivative thereof. In particular, the invention provides an inhibitor of NF-kappaB/Rel protein activation comprising a signalling molecule according to the invention. Such inhibitors are widely searched after these days. Furthermore, the invention provides use of a signalling molecule according to the invention for the production of a pharmaceutical composition for the modulation of gene expression.

The term pharmaceutical composition as used herein is intended to cover both the active signalling molecule alone and a composition containing the signalling molecule together with a pharmaceutically acceptable carrier, diluent or excipient. Acceptable diluents of an oligopeptide as described herein in the detailed description are for example physiological salt solutions or phosphate buffered salt solutions. In one embodiment of the present invention, a signal molecule is administered in an effective concentration to an animal or human systemically, e.g. by intravenous, intra-muscular or intraperitoneal administration. Another way of administration comprises perfusion of organs or tissue, be it in vivo or ex vivo, with a perfusion fluid comprising a signal molecule according to the invention. Topical administration, e.g. in ointments or sprays, may also apply, e.g. in inflammations of the skin or mucosal surfaces of for example mouth, nose and/or genitals. The administration may be done as a single dose, as a discontinuous sequence of various doses, or continuously for a period of time sufficient to permit substantial modulation of gene expression In the case of a continuous administration, the duration of the administration may vary depending upon a number of factors which would readily be appreciated by those skilled in the art.

The administration dose of the active molecule may be varied over a fairly broad range. The concentrations of an active molecule which can be administered would be limited by efficacy at the lower end and the solubility of the compound at the upper end. The optimal dose or doses for a particular patient should and can be determined by the physician or medical specialist involved, taking into consideration well known relevant factors such as the condition, weight and age of the patient, etc.

The active molecule may be administered directly in a suitable vehicle, such as e.g. phosphate-buffered saline (PBS) or as solutions in alcohol or DMSO. Pursuant to preferred embodiments of the present invention, however, the active molecule is administered through a single dose delivery using a drug-delivery system, such as a sustained-release delivery system, which enables the maintenance of the required concentrations of the active molecule for a period of time sufficient for adequate modulation of gene expression. A suitable drug-delivery system would be pharmacologically inactive or at least tolerable. It should preferably not be immunogenic nor cause inflammatory reactions, and should permit release of the active molecule so as to maintain effective levels thereof over the desired time period. A large variety of alternatives are known in the art as suitable for purposes of sustained release and are contemplated as within the scope of the present invention. Suitable delivery vehicles include, but are not limited to, the following: microcapsules or microspheres; liposomes and other lipid-based release systems; viscous instillates; absorbable and/or biodegradable mechanical barriers and implants; and polymeric delivery materials, such as polyethylene oxide/polypropylene oxide block copolymers, polyesters, cross-linked polyvinylalcohols, polyanhydrides, polymethacrylate and polymethacrylamide hydrogels, anionic carbohydrate polymers, etc. Useful delivery systems are well known in the art.

A highly suitable formulation to achieve the active molecule release comprises injectable microcapsules or microspheres made from a biodegradable polymer, such as poly(dl-lactide), poly(dl-lactide-co-glycolide), polycaprolactone, polyglycolide, polylactic acid-co-glycolide, poly(hydroxybutyric acid), polyesters or polyacetals. Injectable systems comprising microcapsules or microspheres having a diameter of about 50 to about 500 micrometers offer advantages over other delivery systems. For example, they generally use less active molecule and may be administered by paramedical personnel. Moreover, such systems are inherently flexible in the design of the duration and rate of separate drug release by selection of microcapsule or microsphere size, drug loading and dosage administered. Further, they can be successfully sterilised by gamma irradiation.

The design, preparation and use of microcapsules and microspheres are well within the reach of persons skilled in the art and detailed information concerning these points is available in the literature. Biodegradable polymers (such as lactide, glycolide and caprolactone polymers) may also be used in formulations other than microcapsules and microspheres; e.g. pre-made films and spray-on films of these polymers containing the active molecule would be suitable for use in accordance with the present invention. Fibres or filaments comprising the active molecule are also contemplated as within the scope of the present invention.

Another highly suitable formulation for a single-dose delivery of the active molecule in accordance with the present invention involves liposomes. The encapsulation of an active molecule in liposomes or multilamellar vesicles is a well known technique for targeted drug delivery and prolonged drug residence. The preparation and use of drug-loaded liposomes is well within the reach of persons skilled in the art and well documented in the literature.

Yet another suitable approach for single dose delivery of an active molecule in accordance with the present invention involves the use of viscous installates. In this technique, high molecular weight carriers are used in admixture with the active molecule, giving rise to a structure which produces a solution with high viscosity. Suitable high molecular weight carriers include, but are not limited to, the following: dextrans and cyclodextrans; hydrogels; (cross-linked) viscous materials, including (cross-linked) viscoelastics; carboxymethylcellulose; hyaluronic acid; and chondroitin sulfate. The preparation and use of drug-loaded viscous instillates is well known to persons skilled in the art.

Pursuant to yet another approach, the active molecule may be administered in combination with absorbable mechanical barriers such oxidised regenerated cellulose. The active molecule may be covalent or non-covalently (e.g. ionically) bound to such a barrier, or it may simply be dispersed therein. A pharmaceutical composition as provided herein is particularly useful for the modulation of gene expression by inhibiting NF-kappaB/Rel protein activation.

NF-kappaB/Rel proteins are a group of structurally related and evolutionarily conserved proteins (Rel). Well known are c-Rel, RelA (p65), RelB, NF-kappaB1 (p50 and its precursor p105), and NF-kappaB2 (p52 and its precursor p100). Most NF-kappaB dimers are activators of transcription, p50/p50 and p52/p52 homodimers repress the transcription of their target genes. All NF-kappaB/Rel proteins share a highly conserved NH2-terminal Rel homology domain (RHD). RHD is responsible for DNA binding, dimerization, association with inhibitory proteins known as IkappaBs. In resting cells, NF-kappaB/Rel dimers are bound to IkappaBs and retained in an inactive form in the cytoplasm. IkappaBs are members of a multigene family (IkappaBalpha, IkappaBbeta, IkappaBgamma, IkappaBepsilon, Bcl-3, the precursor Rel-proteins, p100 and p105. Presence of multiple copies of ankyrin repeats to interact with NF-kappaB via the RHD (protein-protein interaction. Upon appropriate stimulation, IkappaB is phosphorylated by IkappaB Kinase (IKKs), polyubiquitinated by ubiquitin ligase complex, and degraded by the 26S proteosome. NF-kappaB is released and translocates into nucleus to initiate gene expression NF-kappaB regulation of gene expression includes innate immune responses: such as regulated by cytokines IL-1, IL-2, IL-6, IL-12, TNF-alpha, LT-alpha, LT-beta, GM-CSF; expression of adhesion molecules (I CAM, VCAM, endothelial leukocyte adhesion molecule [ELAM]), acute phase proteins (SAA), iInducible enzymes (iNOS and COX-2) and antimicrobial peptides (beta-defensins). For adaptive immunity MHC proteins, IL-2, IL-12 and IFN-alpha are regulated by NF-kappaB. Regulation of overall immune response includes the regulation of genes critical for regulation of apoptosis (c-IAP-1 and c-IAP-2, Fas Ligand, c-myc, p53 and cyclin D1.

Considering that NF-kappaB is a cardinal pro-inflammatory transcription factor, and considering that the invention provides a signalling molecule, such as an oligopeptide and functional analogues or derivatives thereof that are capable of inhibiting NF-kappaB, herein also called NF-kappaB inhibitors, the invention provides a method for modulating NF-kappaB activated gene expression, in particular for inhibiting said expression and thus inhibiting a central pro-inflammatory pathway.

The consequence of this potency to inhibit this pro-inflammatory pathway is wide and far reaching. The invention for example provides a method to mitigate or treat inflammatory airway disease such as asthma. Generally, asthma patients show persistent activation of NF-kappaB of cells lining the respiratory tract. Providing these patients, for example by aerosol application, with a signalling molecule according to the invention, such as LQGV or AQGV or MTRV or functional analogue or derivative thereof, will alleviate the inflammatory airway response of these individuals, by inhibiting NF-kappaB activation of the cells. Such compositions can advantageously be made with signalling molecules that are taken up in liposomes.

The invention also provides a method to mitigate or treat neonatal lung disease, also called chronic lung disease of prematurity, a condition often seen with premature children who develop a prolonged pulmonary inflammation or bronchopulmonary dysplasia. Treating such premature children with an NF-kappaB inhibitor, such as oligopeptide LQGV, or functional analogue or derivative thereof, as provided herein allows such lung conditions to be prevented or ameliorated as well.

Furthermore, considering that an important pathogenic component in the development of insulin-dependent diabetis mellitus (type 1) comprises over-activation of the NF-kappaB pathway as seen in dendritic cells, treatment with an NF-kappaB inhibitor according to the invention will lead to reduced symptoms of diabetes, or at least to a prolonged time to onset of said disease. Particularly effective oligopeptides signalling molecule according to the invention in this context are MTRV, VLPALPQVVC, VLPALP, LPGCPRGVNPVVS, LPGC which were shown herein to postpone onset of diabetes in an Non-obese Diabetic Mouse (NOD). Another approach to treatment of diabetes, in particular insulin - independent diabetes (type 2) comprises inhibition of the PPARgamma cascade with an oligopeptide signalling molecule or functional analogue or derivative thereof.

Other use that is provided relates to a method for combating or treating auto-immune disease. A non-limiting list of an immune diseases includes:
Hashimoto's thyroditis, primary myxoedema thyrotoxicosis, pernicious anaemia, autoimmune atrophic gastritis, Addison's disease, premature menopause, insulin-dependent diabetes mellitus, stiff-man syndrome, Goodpasture's syndrome, myasthenia gravis, male infertility, pemphigus vulgaris, pemphigoid, sympathetic ophthalmia, phacogenic uveitis, multiple sclerosis, autoimmune haemolytic anaemia, idiopathic thrombocytopenic purpura, idiopathic leucopenia, primary biliary cirrhosis, active chronic hepatitis,
cryptogenic cirrhosis, ulcerative colitis, Sjögren's syndrome, rheumatoid arthritis, dermatomyositis, polymyositis, scleroderma, mixed connective tissue disease, discoid lupus erythematosus, and systemic lupus erythematosus.

Other use that is provided relates to a method for combating or treating infections caused by micro-organisms, in particular those infections that are caused by micro-organisms that activate the NF-kappaB pathway during infections.

Such micro-organisms are manifold, including bacteria, viruses, fungi, protozoa, but other pathogens (e.g. worms) can have the same effect. Activation of the NFkappaB pathway by a microbial infection in general occurs via activation of the Toll-like receptor pathway. The invention provides a method to modulate and in particularly inhibit parts gene expression that is related to the inflammatory responses of an organism that are generally activated through this Toll-like receptor pathway.

Toll-like receptor-mediated NF-kappa-B activation is central in recognition of pathogens by a host. Such recognition of pathogens generally occurs through germline-encoded molecules, the so-called pattern recognition receptors (PRRs). These PRRs recognize widespread pathogen associated molecular patterns (PAMPs). The pattern recognition receptors are expressed as either membrane-bound or soluble proteins. They include CD14, beta2-integrins (CD11/CD18), C-type lectins, macrophage scavenger receptors, complement receptors (CR1/CD35, CR2/CD21) and Toll-like receptors (TLRs). TLRs are distinguished from other PRRs by their ability to recognise and discriminate between different classes of pathogens. TLRs represent a family of transmembrane proteins that have an extracellular domain comprising multiple copies of leucine rich repeats (LRRs) and a cytoplasmic: Toll/IL-1R (TIR) motif that has significant homology to the intracellular signaling domain of the type I IL-1 receptor (IL-1RI). Therefore, TLRs are thought to belong to the IL-1R superfamily.

Pathogen associated molecular patterns (PAMPS) are not expressed by hosts but are components of the pathogenic micro-organism. Such PAMPS comprise bacterial cell wall components such as lipopolysaccharides (LPS), lipoproteins (BLP), peptidoglycans (PGN), lipoarabinomannan (LAM), lipoteichoic acid (LTA), DNA containing unmethylated CpG motifs, yeast and fungal cell walls mannans and beta-glucans, double stranded RNA, several unique glycosylated proteins and lipids of protozoa, and so on.

Recognition of these PAMPS foremost provides for differential recognition of pathogens by TLRs. For example, TLR2 is generally activated in response to BLPs, PGNs of gram-positive bacteria, LAM of mycobacteria, and mannans of yeasts, whereas TLR4 is often activated by LPS of gram-negative bacteria and LTA of gram-negative bacteria; also a secreted small molecule MD-2 can account for TLR4 signalling.

Several oligopeptides capable of modulating gene expression according to the invention have earlier been tested, both ex vivo as in vivo, and in small animals but a relationship with modulation of gene expression was not brought forward. A beneficial effect of these oligopeptides on LPS induced sepsis in mice, namely the inhibition of the effect of the sepsis, was observed. Immunomodulatory effects with these oligopeptides have been observed *in vitro* and in *ex vivo* such as in T-cell assays showing the inhibition of pathological Th1 immune responses, suppression of inflammatory cytokines (MIF), increase in production of anti-inflammatory cytokines (IL-10, TGF-beta) and immunomodulatory effects on antigen presenting cells (APC) like dendritic cells, monocytes and macrophages.

Now that the insight has been provided that distinct synthetic oligopeptides or functional analogues or derivatives thereof, for example those that resemble breakdown products which can be derived by proteolysis from endogenous proteins such as hCG, can be used to modulate gene expression, for example by NF-kappaB inhibition, such oligopeptides find much wider application. Release of active NF-kappaB in cells is now known to occur after a variety of stimuli including treating cells with bacterial lipopolysaccharide (LPS) and the interaction with a Toll-like receptor (see for example Guha and Mackman, Cell. Sign. 2001, 13:85-94). In particular LPS stimulation of dendritic cells, monocytes and macrophages induces many genes that are under the influence of activation by transcription factors such as NF-kappaB, p50, EGR-1, IRF-1 and others that can be modulated by a signalling molecule according to the invention. Considering that LPS induction of EGR-1 is required for maximal induction of TNF-alpha, it is foreseen that inhibition of EGR-1 considerably reduces the effects of sepsis seen after LPS activation. Now knowing the gene modulatory effect of the signalling molecules such as oligopeptides as provided herein allows for rational design of signal molecule mixtures that better alleviate the symptoms seen with sepsis. One such mixture, a 1:1:1 mixture of LGQV, AQGV and VLPALP was administered to primates in a gram-negative induced rhesus monkey sepsis model for prevention of septic shock and found to be effective in this primate model. Accordingly, the invention provides a pharmaceutical composition for the treatment of sepsis in a primate, and a method for the treatment of sepsis in a primate comprising subjecting said primate to a signalling molecule according to the invention, preferably to a mixture of such signalling molecules. Administration of such a signalling molecule or mixture preferably occurs systematically, e.g. by intravenous or intraperitoneal administration.

Other use that is contemplated relates to a method for combating or treating viral infections, in particular those infections that are caused by viruses that activate the NF-kappaB pathway during infections. Such virus infections are manifold, classical examples are hepatitis B virus induced cell transformation by persistent activation of NF-kappaB. Use of a signalling molecule according to the invention is herein provided to counter or prevent this cell transformation.

Other disease where persistent NF-kappaB activation is advantageously inhibited by a signalling molecule according to the invention is a transplantation-related disease such as transplantation-related immune responses, graft-versus-host-disease, in particular with bone-marrow transplants, acute or chronic xeno-transplant rejection, and post-transfusion thrombocytopenia.

Other case where persistent NF-kappaB activation is advantageously inhibited by a signalling molecule according to the invention is found in the prevention or mitigation of ischemia-related tissue damage seen after infarcts, seen for example in vivo in brain or heart, or ex vivo in organs or tissue that is being prepared or stored in preparation of further use as a transplant. Ischemia related tissue damage can now be mitigated by perfusing the (pre)ischemic area with a signalling molecule according to the invention that inhibits NF-kappaB activation. Example of conditions where ischemia (also called underperfusion) plays a role are for example eclampsia which can be ascribed to focal cerebral ischemia resulting from vasoconstriction, consistent with the evidence of changes detected by new cerebral imaging techniques. The liver dysfunction intrinsic to the HELLP (hemolysis, elevated liver enzymes, and low platelet count) syndrome could also be attributed to the effects of acute underperfusion. Other conditions of ischemia are seen after coronary occlusion leading to irreversible myocardial damage produced by prolonged episodes of coronary artery occlusion and reperfusion in vivo, which has already been discussed in PCT/NL01/00259 as well.

Now that the insight has been provided that distinct synthetic oligopeptides, for example those that resemble breakdown products which can be derived by proteolysis from endogenous proteins such as hCG, can be used to modulate gene expression, for example by NF-kappaB inhibition, the oligopeptides find much wider application. For example, the invention provides a method for perfusing a transplant with a perfusing fluid comprising at least one signalling molecule according to the invention; ischemic or pre-implantation damage due to activation of NF-kappaB in said transplant can than greatly be diminished, allowing a wider use of said transplants.

The invention provides a signalling molecule useful in modulating expression of a gene in a cell . Several examples of the use of such a signalling molecule for the production of a pharmaceutical composition for the treatment medical or veterinary conditions are herewith given. In one embodiment, the invention provides such use in the treatment of an immune-mediated-disorder, in particular of those cases whereby a central role of NF-kappaB/Rel proteins in said immune response is found. However as said, modulating gene expression via modulating activity of other transcription factors, such as AP-1 or PPARgamma, an others is also provided, now that the gene modulating role of signalling molecules such as the oligopetides or analogues or derivatives thereof is understood. As also said, now knowing that oligopeptides, likely breakdown products, play such a central role in modulation of gene expression, the invention provides straightforward ways for identifying further gene expression modulating oligopeptides, and provides synthetic versions of these , and analogues and derivatives thereof for use in a wide variety of disorders and for use in the preparation of a wide variety of pharmaceutical compositions. Examples of such treatment and useful pharmaceutical compositions are for example found in relation to conditions wherein said immune-mediated disorder comprises chronic inflammation, such as diabetes, multiple sclerosis or acute or chronic transplant rejection, in particular in those cases whereby antigen presenting cells (APC's) or dendritic cells (DCs) are enhanced by (overactive) and persistent NF-kappaB expression or wherein said immune-mediated disorder comprises acute inflammation, such as septic or anaphylactic shock or hyper-acute transplant rejection. Other immune-mediated disorders that can be treated with a pharmaceutical composition comprising a signalling molecule according to the invention comprise auto-immune disease, such as systemic lupus erythematosus or rheumatoid arthritis (in particular by inhibiting Il-8and/or Il-15 production by inhibiting NF-kappaB activity on the expression of these genes), allergy, such as asthma or parasitic disease, overly strong immune responses directed against an infectious agent, such as a virus or bacterium (in particular responses that include rapid haemorrhagic disease caused by infection with organisms such as Yersinia pestis, Ebola-virus, Stafylococcus aureus (e.g. in cases of tampon-disease), bacterial (such as meningococcal) or viral meningitis and/or encephalitis, and other life-threatening conditions). Such overly strong overly responses are seen with for example pre-eclampsia, recurrent spontaneous abortions (RSA) or preterm parturition or other pregnancy related disorders. Especially with forms of eclampsia/pre-eclampsia that are associated with genetically programmed increased production of tumour-growth factor beta-1, treatment according to the invention is recommended. Also, in situations where RSA is likely attributable to increased IL-10 levels during pregnancy, or to increased TNF-alpha activity, for example due to the presence of an unfavourable allele, in particular of a G to A polymorphism in the promotor of the gene encoded TNF-alpha, treatment with a pharmaceutical composition as provided herein is recommended. Treatment direct at such pregnancy-related immune disorders is herein also provided by inhibiting NF-kappaB activity directed at activating natural killer (NK) cell activity. Also, LPS induced reduced fertility, or abortions, seen in pregnant sows, can be reduced by applying a signalling molecule or method as provided herein.

Such use in treatment of an immune-mediated disorder preferably comprises regulating relative ratios and/or cytokine activity of lymphocyte, dendritic or antigen presenting cell subset-populations in a treated individual, in particular wherein said subset populations comprise Th1 or Th2, or DC1 or DC2 cells. Other embodiments of the invention comprise use of a signalling molecule according to the invention for the manufacture of a medicament for modulating a cardiovascular or circulatory disorder, such as coronary arterial inclusion, and also in a pregnancy related cardiovascular or circulatory disorder.

Furthermore, the invention provides a pharmaceutical composition for modulating a cardiovascular or circulatory disorder, in particular a pregnancy related cardiovascular or circulatory disorder, comprising a signalling molecule according to the invention or mixtures thereof. Such a composition finds it use in a method for modulating a cardiovascular or circulatory disorder, in particular a pregnancy related cardiovascular or circulatory disorder, comprising subjecting an animal (in particular a mammal) to treatment with at least one signalling molecule according to the invention.

The invention also provides use of a signalling molecule for the preparation of a pharmaceutical composition or medicament and methods of treatment various medical conditions that are other than use in the preparation of a pharmaceutical composition for the treatment of an immune-mediated-disorder or a method of treatment of an immune-mediated-disorder. For example, the invention provides topical application, for example in an ointment or spray comprising a signal molecule according to the invention, for the prevention or mitigation of skin afflictions, such as eczemas, psoriasis, but also of skin damage related to over-exposure to UV-light.

Also, use is contemplated in palliative control, whereby a gene related to prostaglindin synthesis is modulated such that COX2 pathways are effected.

Furthermore, the invention also provides use of a signalling molecule for the preparation of a pharmaceutical composition or medicament and methods of treatment various medical conditions that are other than use in the preparation of a pharmaceutical composition for the treatment of wasting syndrome, such as the treatment of particular individuals that are suffering from infection with HIV or a method of treatment of wasting syndrome of such individuals.

In one embodiment, the invention provides the use of a signalling molecule according to the invention for the preparation of a pharmaceutical composition or medicament for modulating angiogenesis or vascularisation, in particular during embryonal development, or after transplantation to stimulate vascularisation into the transplanted organ or inhibit it in a later phase. Signalling molecules that effect angiogenesis are disclosed herein in the detailed description.

Use as provided herein also comprises regulating TNF-alpha receptor (e.g. CD27) expression on cells, thereby modulating the relative ratios and/or cytokine activity of lymphocyte, dendritic or antigen presenting cell subset-populations in a treated individual. As for example described in the detailed description, particular oligopeptide according to the invention is capable of down-regulating CD27 expression on cells of the T-cell lineage.

Down-regulating TNF-alpha itself, and/or a recpetor for TNF-alpha, as is herein also provided, is also beneficial in individuals with Chagas cardiomyopathy.

Also, use of a signalling molecule according to the invention for the preparation of a pharmaceutical composition for modulation of vascularisation or angiogenesis in wound repair, in particular of burns, is herein provided. Also, use of a pharmaceutical composition as provided herein is provided in cases of postoperative physiological stress, whereby not only vascularisation will benefit from treatment, but the general well-being of the patient is improved as well.

Another use of a signalling molecule according to the invention comprises its use for the preparation of another pharmaceutical composition for the treatment of cancer. Such a pharmaceutical composition preferably acts via modulating and up-regulating apoptotic responses that are classically down-regulated by NF-kappaB activity. Inhibiting said activity with a signalling molecule according to the invention allows for increased cell death of tumorous cells. Another anti-cancerous activity of a signalling molecule as provided herein comprises down-regulation of c-myb, in particular in the case of hematopoietic tumors in humans. In this context, down-regulation of 14.3.3 protein is also provided.

A further use of a signalling molecule according to the invention comprises its use for the preparation of a further pharmaceutical composition for the treatment of cancer. Such a pharmaceutical composition preferably acts via modulating and down-regulating transferrine receptor availability, in particular on tumorous cells. Transferrine receptors are classically up-regulated by NF-kappaB activity. Inhibiting said activity with a signalling molecule according to the invention allows for reduced iron up-take and increased cell death of tumorous cells. In particular erytrhoide and thromboide cells are susceptible said treatment.

Yet a further use of a signalling molecule according to the invention comprises its use for the preparation of yet another pharmaceutical composition for the treatment of cancer, in particular of cancers that are caused by viruses, such as is the case with retroviral-induced malignancies and other viral-induuced malignancies. Such a pharmaceutical composition preferably acts via modulating and down-regulating cell-proliferative responses that are classically up-regulated by virus-induced transcriptional or NF-kappaB activity. Inhibiting said activity with a signalling molecule according to the invention allows for decreased proliferation and increased cell death of tumorous cells. Such a pharmaceutial composition may also act via modulating angiogenic responses induced by Il-8, whereby for example inhibition of Il-8 expression via inhibition of transcription factor AP-1 or NF-kappaB expression results in the inhibition of vascularisation dependent tumor growth.

Furthermore, the invention provides the use of a signalling molecule for the preparation of a pharmaceutical composition for optimising human or animal fertility and embryo survival, and a method for optimising fertility and embryo survival. In particularly, the invention provided for a method and composition allowing the down-regulation of TNF-alpha in the fertilized individual, optimally in combination with a composition and method for up-regulating Il-10 in said individual. Such a composition and method finds immediate use in both human and veterinary medicine.

Also, the invention provides the use of a signalling molecule for the preparation of a pharmaceutical composition for modulating the body weight of an individual, in particular by modulating gene expression of a gene under influence of peroxisome proliferator-activated receptor gamma (PPARgamma) activation and lipid metabolism by applying a signalling molecule according to the invention, and a method for modulating bodyweight comprising providing an individual with a signalling molecule according to the invention.

A further use of a signalling molecule as provided herein lies in the modulation of expression of a gene in a cultured cell. Such a method as provided herein comprises subjecting a signalling molecule according to the invention to said cultured cell. Proliferation and/or differentiation of cultured cells (cells having been or being under conditions of in vitro cell culture known in the art) can be modulated by subjecting said cultured cell to a signalling molecule according to the invention. It is contemplated that for example research into proliferation or differentiation of cells, such as stem-cell research will benefit greatly from understanding that a third major way of effecting gene modulation exists, and considering the ease of application of synthetic peptides , and analogues or derivatives thereof.

Furthermore, it is contemplated that s signalling molecule as provided herein finds an advantageous use as a co-stimulatory substance in a vaccine, accompanying modern day adjuvants or replacing the classically used mycobacterial adjuvants, especially considering that certain mycobacteria express HCG-like proteins, of which it is now postulated that these bacteria have already made use of the this third pathway found in gene modulation as provided herein by providing the host with breakdown products mimicking the signalling molecules identified herein. Treatment and use of the compositions as provided herein is not restricted to animals only, plants and other organisms are also subject to this third pathway as provided herein. Furthermore, now that the existence of such a pathway has been demonstrated, it is herein provided to make it subject of diagnosis as well, for example to determine the gene modulatory state of a cell in a method comprising determining the presence or absence of a signalling molecule as provided herein.

### Figure legends

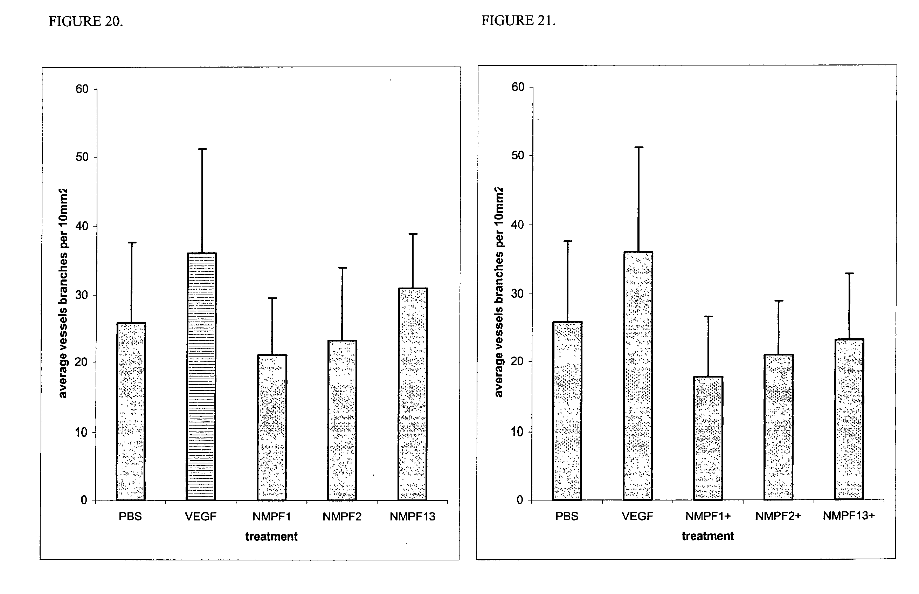

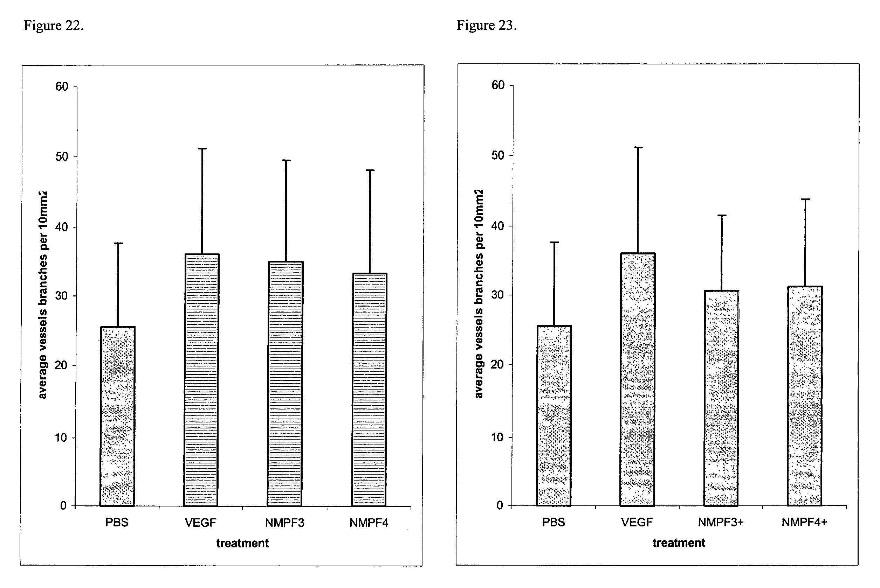

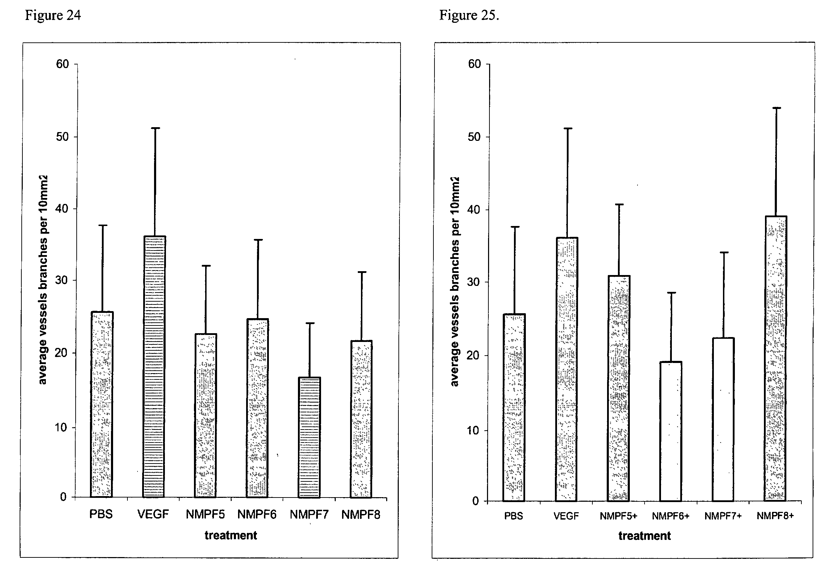

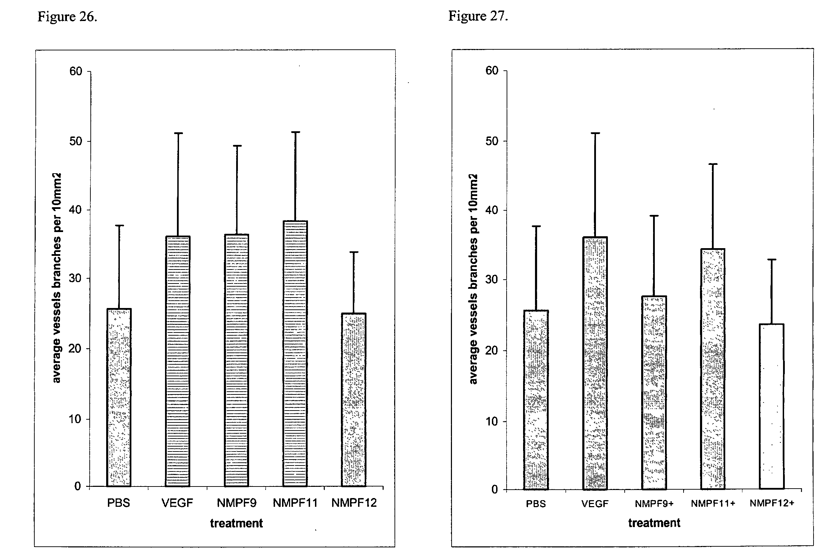

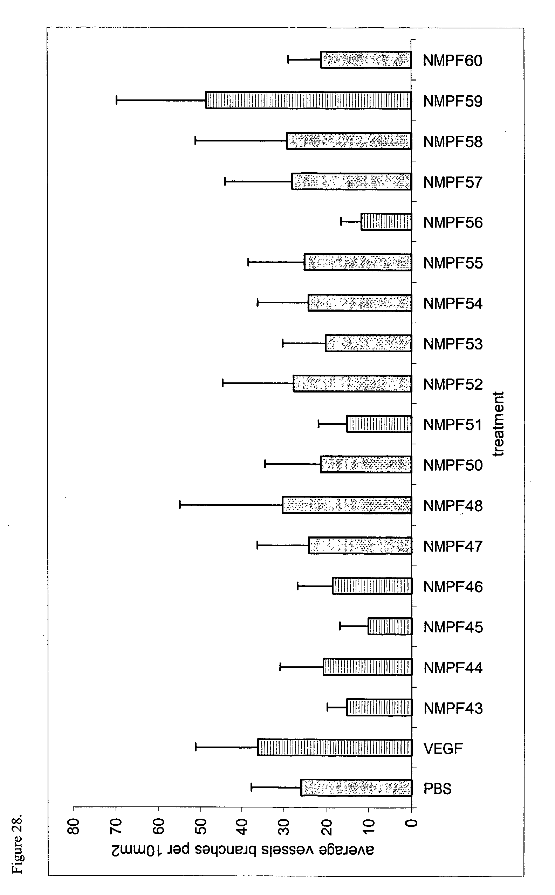

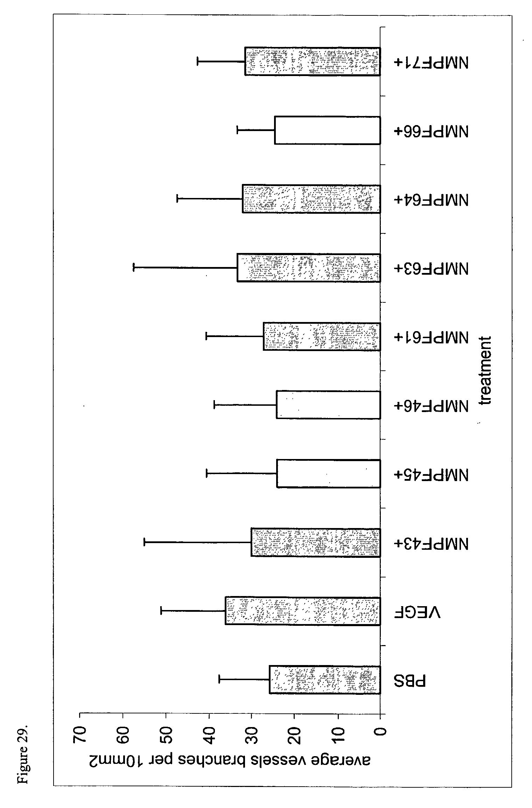

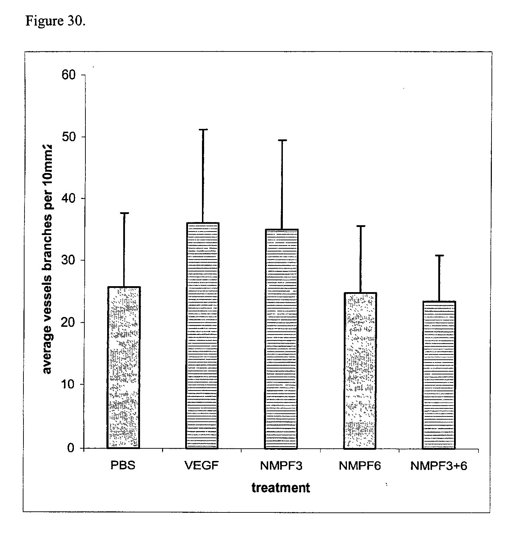

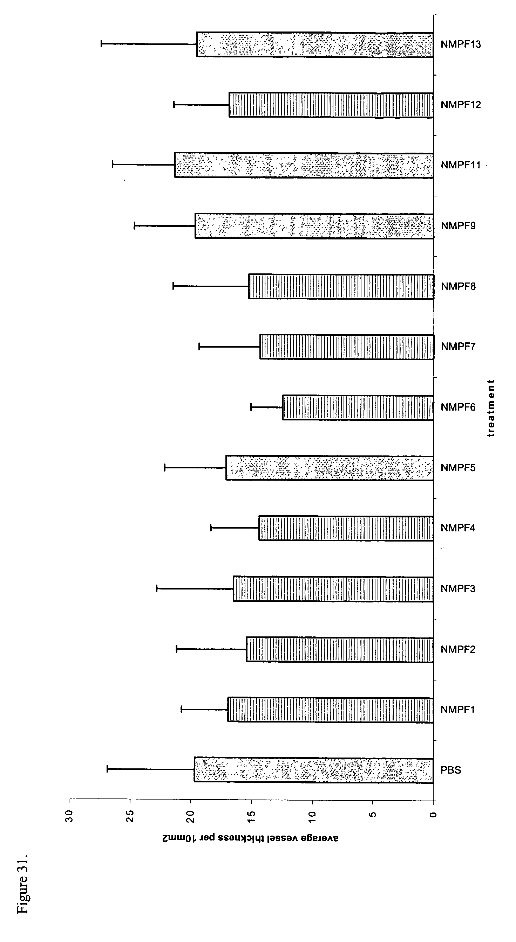

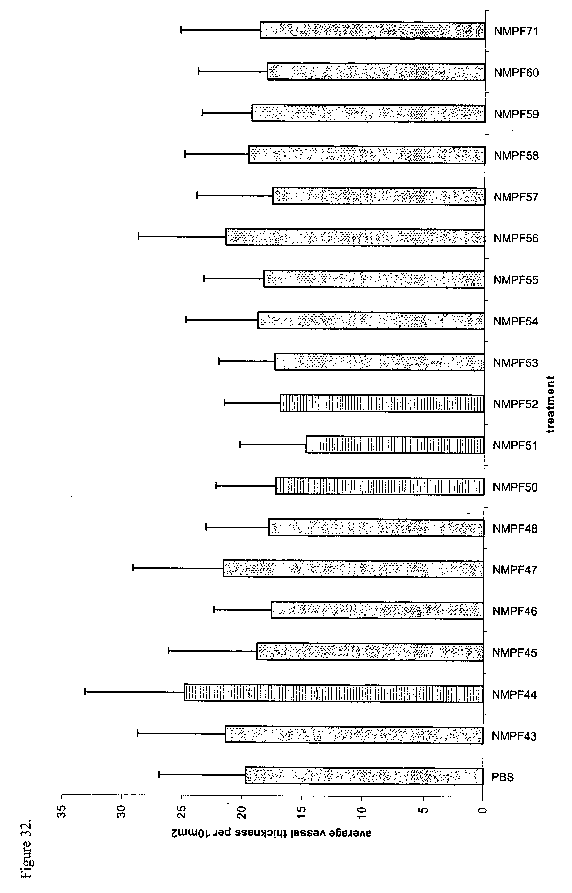

Figure 1-2.
   Bone marrow (BM) cell yield of treated BALB/c mice (n=6).
   BM cells were isolated from treated mice and cultured *in vitro* in the presence of rmGM-CSF for nine days. These figures show cell yield after nine days of culture of BM cells isolated from mice treated with PBS, LPS or LPS in combination with NMPF peptides 4, 46, 7 and 60.
   In these figures cell yield is expressed in relative percentage of cells compared to PBS. Each condition consists of 6 Petri dishes and results shown in these figures are representative of 6 dishes. Differences of ≥ 20% were considered significant and line bars represent significant data as compared to LPS control group.
   A representative experiment is shown. Findings involving all experimental conditions were entirely reproduced in 3 additional experiments.
Figure 3.
   Effect of *in vivo* treatment on MHC-II expression on CD11c⁺ cells.
   Bone marrow (BM) cells were isolated from treated BALB/c mice (n=6) and cultured *in vitro* in the presence of rmGM-CSF for nine days.
   This figure shows MHC-II expression expressed in mean fluorescence intensity (MFI) after nine days of culturing of BM cells isolated from PBS, LPS or LPS in combination with NMPF. Each condition consists of 6 Petri dishes and results shown in these figures are representative of 6 dishes. Differences of ≥ 20% were considered significant and line bars represent significant data as compared to LPS control group.
   A representative experiment is shown. Findings involving all experimental conditions were entirely reproduced in 3 additional experiments.
Figure 4-7.
   Bone marrow (BM) cell yield of *in vitro* treated BM cultures.
   BM cells from BALB/c mice (n=3) were cultured *in vitro* and treated with either PBS, LPS (t=6), NMPF 4, 7, 46, 60 (t=0 or t=6) or combination of NMPF with LPS (t=6), in the presence of rmGM-CSF for nine days.
   These figures show cell yield expressed in relative percentage of cells compared to PBS after nine days of culture of BM cells. Each condition consists of 6 Petri dishes and results shown in these figures are representative of 6 dishes. Differences of ≥ 20% were considered significant. Line bars represent significant data as compared to LPS control group and dotted bars represent significant data as compared to PBS group.
   A representative experiment is shown. Findings involving all experimental conditions were entirely reproduced in 3 additional experiments.
Figure 8-11.
   Effect of *in vitro* treatment on MHC-II expression on CD11c⁺ cells.
   BM cells from BALB/c mice (n=3) were cultured *in vitro* and treated with either PBS, LPS (t=6), NMPF 4, 7, 46, 60 (t=0 or t=6 days) or combination of NMPF with LPS (t=6 days), in the presence of rmGM-CSF for nine days. These figures show MHC-II expression expressed in mean fluorescence intensity (MFI) of CD11c positive cells after nine days of culturing of BM cells. Each condition consists of 6 Petri dishes, and results shown in these figures are representative of 6 dishes. Differences of ≥ 20% were considered significant. Line bars represent significant data as compared to LPS control group and dotted bars represent significant data as compared to PBS group.
   A representative experiment is shown. Findings involving all experimental conditions were entirely reproduced in 3 additional experiments.
Figure 12-15.
   Bone marrow (BM) cell yield of treated BALB/c mice (n=6).
   BM cells were isolated from treated mice and cultured *in vitro* in the presence of rmGM-CSF for nine days. These figures show cell yield after nine days of culture of BM cells in suspension (unattached) and attached to Petri dish (attached). BM cells were isolated from mice treated with PBS, LPS or LPS in combination with different NMPF peptides. In these figures cell yield is expressed in relative percentage of cells compared to PBS. Each condition consists of 6 Petri dishes and results shown here are representative of 6 dishes. Differences of ≥ 20% were considered significant and line bars represent significant data as compared to LPS control group.
   A representative experiment is shown. Findings involving all experimental conditions were entirely reproduced in 3 additional experiments.
Figure 16-17.
   Bone marrow (BM) cell yield of *in vitro* treated BM cultures from NOD mice.
   BM cells from 15 weeks old female NOD mice (n=3) were cultured *in vitro* and treated with either PBS or NMPF in the presence of rmGM-CSF for nine days. These figures show cell yield after nine days of culture of BM cells in suspension (unattached) and attached to Petri dishes (attached). In these figures cell yield is expressed in relative percentage of cells compared to PBS. Each condition consists of 6 Petri dishes and results shown here are representative of 6 dishes. Differences of ≥ 20% were considered significant and dotted bars represent significant data as compared to PBS control group.
   A representative experiment is shown. Findings involving all experimental conditions were entirely reproduced in 3 additional experiments.
Figure 20-32.
   In vivo treatment of fertilised chicken eggs with NMPF and the effect of NMPF on angiogenesis.
   Fertile chicken eggs (day 0) were treated with either PBS, NMPF, VEGF or VEGF in combination with NMPF. Ten eggs were injected for every condition. On day 8 of incubation, the embryos were removed from the eggs and were placed in a 100-mm Petri dish. The embryo and the blood vessels were photographed in vivo with the use of a microscope. Of each egg one overview picture was taken and 4 detail pictures of the blood vessels were taken.
   Quantification of angiogenesis (vessels branches) was accomplished by counting the number of blood vessels branches. Quantification of this vasculogenesis was accomplished by measuring the blood vessel thickness. The number of blood vessel branches and vessel thichness was measured in the pictures and were correlated to a raster (in the pictures) of 10mm² for comparison.
   The mean number of branches and the mean blood vessel thickness of each condition (N=10) were calculated and compared to the either PBS or VEGF controls using a Student's T-test. Line bars represent significant (p<0.05) data as compared to PBS control group and dotted bars represent significant (p<0.05) data as compared to VEGF group. Figures 20-30 show the effect of NMPF on vessel branches. Figures 31-32 show the effect of NMPF on vessel thickness.
Figure 33.
   Detection of NF-kB via EMSA. This figures shows the presence of NF-kB in the nuclear extracts of RAW264.7 cells treated with LPS or NMPF in combination with LPS for 4 hours. Numbers 1-13 correspond to nuclear extracts from cells treated with NMPF and LPS. *CTL* corresponds to nuclear extracts from cells treated with LPS only. Specificity of the radioactively labeled NF-kB probe is shown by competition with the unlabeld oligonucleotide (u1,u2,u3) in three different concentrations (1x, 10x, 100x) with nuclear extracts of CTL and *olg* corresponds to samples containing only labeled oligonucleotide (without nuclear extract). Description: (NMPF-1)VLPALPQVVC, (NMPF-2)LQGVLPALPQ, (NMPF-3)LQG, (NMPF-4)LQGV, (NMPF-5)GVLPALPQ, (NMPF-6)VLPALP, (NMPF-7)VLPALPQ, (NMPF-8)GVLPALP, (NMPF-9)VVC, (NMPF-11)MTRV, (NMPF-12)MTR
Figure 34.
   HPLC chromatograph (wave length 206) in which data profile obtained from the nuclear protein extracts of LPS and LPS in combination with NMPF stimulated RAW264.7 cells are overlayed.
Figure 35.
   MSn analysis of NMPF-4 peptide.
Figure 36.
   MSn analysis of fraction from nuclear extract of LPS stimulated RAW264.7 cells. Upper panel shows full spectrum of the fraction and lower panel shows the MS/MS spectrum of mass 413.13.
Figure 37.
   MSn analysis of fraction from nuclear extract of LPS in combination with NMPF-4 stimulated RAW264.7 cells. Upper panel shows full spectrum of the fraction and lower panel shows the MS/MS spectrum of mass 416.07.

### Detailed description

Cells react to environmental and intrinsic changes, which they perceive through extracellular and inter- as well as intracellular signals. The nature of these signals can be either for example physical or chemical. Moreover, different classes of molecules present in blood react to each other and induce cascade of reaction that have direct effect on other molecules and/or eventually lead to cellular responses, for example complement system and blood coagulation proteins.

Many genes are regulated not by a signalling molecule that enters the cells but by molecules that bind to specific receptors on the surface of cells for example receptors with enzymatic activity (receptor tyrosine kinases, receptor-like protein tyrosine phosphateases, receptor serine/threonine kinases, histidine kinases, guanylyl cyclases) and receptors without enzymatic activity (cytokine receptors, intergrins, G-protein-coupled receptors). Interaction between cell-surface receptors and their ligands can be followed by a cascade of intracellular events that modulate one or more intracellular transducing proteins, including variations in the intracellular levels of so-called second messengers (diacylglycerol, Ca²⁺, cyclic nucleotides, inositol(1,4,5) trisphosphate, phosphatidylinositol(3,4,5) trisphosphate, phosphatidylinositol transfer protein (PITP)). This leads to the activation or inhibition of a so-called 'effector protein'. The second messengers in turn lead to changes in protein for example protein phosphorylation through the action of cyclic AMP, cyclic GMP, calcium-activated protein kinases, or protein kinases (for example AGC group serine/threonine protein kinases, CAMK group serine/threonine protein kinases, CMGC group serine/threonine kinases, protein tyrosine kinase group, or others like MEK/Ste7p). Phosphorylation by protein kinases is one of the regulatory mechanisms in signal transmission that modulate different cellular pathways such as Ras/MAPK pathway, MAP kinase pathway, JAK-STAT pathway, wnt-pathway. In many instances this all results in altered gene expression (for example genes for the regulation of other genes, cell survial, growth, differentiation, maturation, functional activity).

Many of the responses to binding of ligands to cell-surface receptors are cytoplasmatic and do not involve immediate gene activation in the nucleus. In some instances a pre-existing inactive transcription factor following a cell-surface interaction is activated that lead to immediate gene activation . For example the protein NF-kappaB, which can be activated within minutes by a variety of stimuli, including membrane receptors (for example pattern recognition receptors like Toll-like receptor binding to pathogen-associated molecular patterns), inflammatory cytokines such as TNF-a, IL-1, T-cell activation signals, growth factors and stress inducers.

Our genomic experiment with NMPF peptide LQGV showed very immediate effects on signal transduction and gene regulation since the cells were treated with the peptide for only four hours. In this short period of time LQGV down-regulated at least 120 genes and up-regulatedat least 6 genes in the presence of a strong stimulator (PHA/IL-2 stimulated T-cell line (PM1)), demonstrating the profound effect a signalling molecule according to the invention has of tge modulation of gene expression. The genes affected by LQGV include onco-genes, genes for transcription factors, intracellular enzymes, membrane receptors, intracellular receptors, signal transducing proteins (for example kinases) and some genes for unkown molecules. This shows that LQGV as an example of the synthetic signalling molecule ( oligopeptide or functional analogue or derivative thereof) as described here, has broad spectrum of effects at different intracellular levels. In addition, our HPLC/MS data have shown the presence of LQGV in the nucleus of a macrophage cell line (RAW267.4) within half hour and also indicates the direct effects on DNA level as well as at intracellular level which is further supported by NF-kappaB experiments. The ultimate modulatory effect of LQGV is dependent on, for example, type of the cell, differentiation and maturation status of the cell, the functional status and the presence of other regulatory molecules. This was evident by shock experiment in which different NMPF peptides had similar or different effects on the disease. Same results were obtained with DC, fertilized chicken egg experiments, and CAO experiments; NMPF effects were dependent on type of co-stimulation (GM-CSF alone or in combination with LPS, or VEGF) and time of the treatment. Due to this NMPF have the ability to modulate cellular responses at different levels.

### MATERIAL AND METHODS

### Peptide synthesis

The peptides as mentioned in this document such as LQG, AQG, LQGV, AQGV, LQGA, VLPALP, ALPALP, VAPALP, ALPALPQ, VLPAAPQ, VLPALAQ, LAGV, VLAALP, VLAALP, VLPALA, VLPALPQ, VLAALPQ, VLPALPA, GVLPALP, VVCNYRDVRFESIRLPGCPRGVNPVVSYAVALSCQCAL, RPRCRPINATLAVEK EGCPVCITVNTTICAGYCPT, SKAPPPSLPSPSRLPGP, LQGVLPALPQVVC, SIRLPGCPRGVNPVVS, LPGCPRGVNPVVS, LPGC, MTRV, MTR, VVC were prepared by solid-phase synthesis (Merrifield, 1963) using the fluorenylmethoxycarbonyl (Fmoc)/tert-butyl-based methodology (Atherton, 1985) with 2-chlorotrityl chloride resin (Barlos, 1991) as the solid support. The side-chain of glutamine was protected with a trityl function. The peptides were synthesised manually. Each coupling consisted of the following steps: (i) removal of the alpha-amino Fmoc-protection by piperidine in dimethylformamide (DMF) (ii) coupling of the Fmoc amino acid (3 eq) with diisopropylcarbodiimide (DIC)/1-hydroxybenzotriazole (HOBt) in DMF/N-methylformamide (NMP) (iii) capping of the remaining amino functions with acetic anhydride/diisopropylethylamine (DIEA) in DMF/NMP. Upon completion of the synthesis, the peptide resin was treated with a mixture of trifluoroacetic acid (TFA)/H2O/triisopropylsilane (TIS) 95:2.5:2.5. After 30 minutes TIS was added until decouloration. The solution was evaporated in vacuo and the peptide precipitated with diethylether. The crude peptides were dissolved in water (50-100 mg/ml) and purified by reverse-phase high-performance liquid chromatography (RP-HPLC). HPLC conditions were: column: Vydac TP21810C18 (10 x 250 mm); elution system: gradient system of 0.1% TFA in water v/v (A) and 0.1% TFA in acetonitrile (ACN) v/v (B); flow rate 6 ml/min; absorbance was detected from 190-370 nm. There were different gradient systems used. For example for peptides LQG and LQGV: 10 minutes 100% A followed by linear gradient 0-10% B in 50 minutes. For example for peptides VLPALP and VLPALPQ: 5 minutes 5% B followed by linear gradient 1% B/minute. The collected fractions were concentrated to about 5 ml by rotation film evaporation under reduced pressure at 40°C. The remaining TFA was exchanged against acetate by eluting two times over a column with anion exchange resin (Merck II) in acetate form. The elute was concentrated and lyophilised in 28 hours. Peptides later were prepared for use by dissolving them in PBS.

### Transcription factor experiment

*Macrophage cell line.* The RAW 264.7 macrophages, obtained from American Type Culture Collection (Manassas, VA), were cultured at 37°C in 5% CO2 using DMEM containing 10% FBS and antibiotics (100 U/ml of penicillin, and 100 µg/ml streptomycin). Cells (1 x10⁶/ml) were incubated with peptide (10 □g/ml) in a volume of 2 ml. After 8 h of cultures cells were washed and prepared for nuclear extracts.

*Nuclear extracts.* Nuclear extracts and EMSA were prepared according to Schreiber et al. Methods (Schriber et al. 1989, Nucleic Acids Research 17). Briefly, nuclear extracts from peptide stimulated or nonstimulated macrophages were prepared by cell lysis followed by nuclear lysis. Cells were then suspended in 400 µl of buffer (10 mM HEPES (pH 7.9), 10 mM KCl, 0.1 mM KCL, 0.1 mM EDTA, 0.1 mM EGTA, 1 mM DTT, 0.5 mM PMSF and protease inhibitors), vigorously vortexed for 15 s, left standing at 4°C for 15 min, and centrifuged at 15,000 rpm for 2 min. The pelleted nuclei were resuspended in buffer (20 mM HEPES (pH 7.9), 10% glycerol, 400 mM NaCl, 1 mM EDTA, 1mM EGTA, 1 mM DTT, 0.5 mM PMSF and protease inhibitors) for 30 min on ice, then the lysates were centrifuged at 15,000 rpm for 2 min. The supernatants containing the solubilized nuclear proteins were stored at -70°C until used for the Electrophoretic Mobility Shift Assays (EMSA).

*EMSA.* Electrophoretic mobility shift assays were performed by incubating nuclear extracts prepared from control (RAW 264.7) and peptide treated RAW 264.7 cells with a 32P-labeled double-stranded probe (5' AGCTCAGAGGGGGACTTTCCGAGAG 3') synthesized to represent the NF-kappaB binding sequence. Shortly, the probe was end-labeled with T4 polynucleotide kinase according to manufacturer's instructions (Promega, Madison, WI). The annealed probe was incubated with nuclear extract as follows: in EMSA, binding reaction mixtures (20 µl) contained 0.25 µg of poly(dI-dC) (Amersham Pharmacia Biotech) and 20,000 rpm of 32P-labeled DNA probe in binding buffer consisting of 5 mM EDTA, 20% Ficoll, 5 mM DTT, 300 mM KCl and 50 mM HEPES. The binding reaction was started by the addition of cell extracts (10 µg) and was continued for 30 min at room temperature. The DNA-protein complex was resolved from free oligonucleotide by electrophoresis in a 6% polyacrylamide gel. The gels were dried and exposed to x-ray films.

### RESULTS

### NF-kB experiments

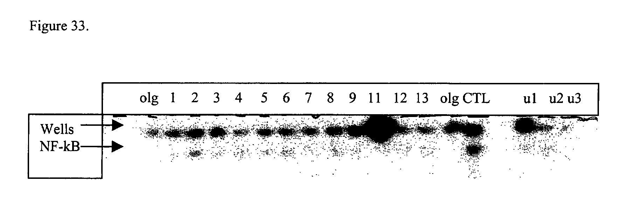

The transcription factor NF-kB participates in the transcriptional regulation of a variety of genes. Nuclear protein extracts were prepared from LPS and peptide treated RAW264.7 cells or from LPS treated RAW264.7 cells. In order to determined whether the peptide modulates the translocation of NF-kB into the nucleus, on these extracts EMSA was performed. Figure 33 shows the amount of NF-kB present in the nuclear extracts of RAW264.7 cells treated with LPS or peptde in combination with LPS for 4 hours. Here we see that indeed some peptides are able to modulate the translocation of NF-kB since the amount of labeled oligonucleotide for NF-kB is reduced. In this experiment peptides that show the modulation of translocation of NF-kB are: (NMPF-1)VLPALPQVVC, (NMPF-2)LQGVLPALPQ, (NMPF-3)LQG, (NMPF-4)LQGV, (NMPF-5)GVLPALPQ, (NMPF-6)VLPALP, (NMPF-7)VLPALPQ, (NMPF-8)GVLPALP, (NMPF-9)VVC, (NMPF-11)MTRV, (NMPF-12)MTR.

### Nuclear location of peptide experiment

Reverse-phase high-performance liquid chromatography (RP-HPLC) method was used to prove the presence of synthetic oligo-peptide in the nuclear extracts. We used a Shimadzu HPLC system equipped with Vydac monomeric C18 column (column218MS54, LC/MS C18 reversed phase, 300A, 5□m, 4.6mm ID x 250mm L); elution system: gradient system of 0.01% TFA and 5% acetonitrile (CAN) in water v/v (A) and 0.01% TFA in 80% acetonitrile (ACN) v/v (B); flow rate 0.5 ml/min; absorbance was detected from 190-370 nm. The gradient time programe was as follows:

| Time (min) | Buffer B concentration |
|---|---|
| 0.01 | 0 |
| 5.0 | 0 |
| 30.0 | 80 |
| 40.0 | 100 |
| 60.0 | 100 |
| 65.0 | 0 |
| 70.0 | 0 |

The elution time of peptide LQGV was determined by injecting 2 □g of the peptide in a separate run. Mass spectrometry (MS) analysis of fraction which contained possible NMPF-4 (LQGV) (elution time was determined by injecting the peptide in the same or separate run) was performed on LCQ Deca XP (Thermo Finnigan).

### RESULTS

### Nuclear location of peptide experiment

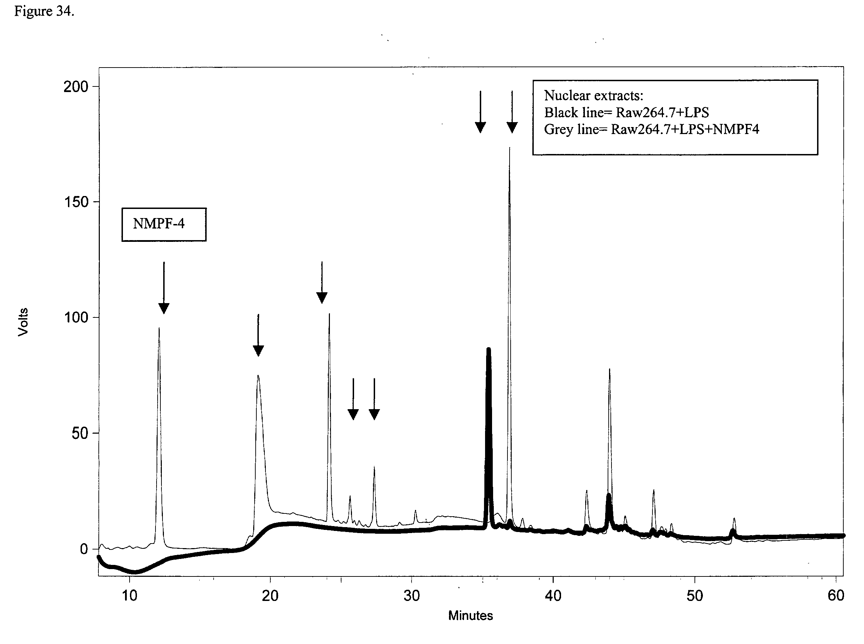

The nuclear protein extracts used in EMSA experiments were also checked for the presence of LQGV by means of HPLC and MS. Figure 34 shows HPLC chromatograph (wave length 206) in which data profile obtained from the nuclear protein extracts of LPS and LPS in combination with NMPF-4 (LQGV) stimulated RAW264.7 cells are overlayed. This figure also show the presence or absence of number of molecule signals in the nuclear extracts of oligopeptide+LPS treated cells as compared to nuclear extracts of LPS treated cells. Since HPLC profile of LQGV showed that the peptide elutes at around 12 minutes (data not shown), fraction corresponding to region 10-15 minutes was collected and analysed for the presence of this peptide in MS.

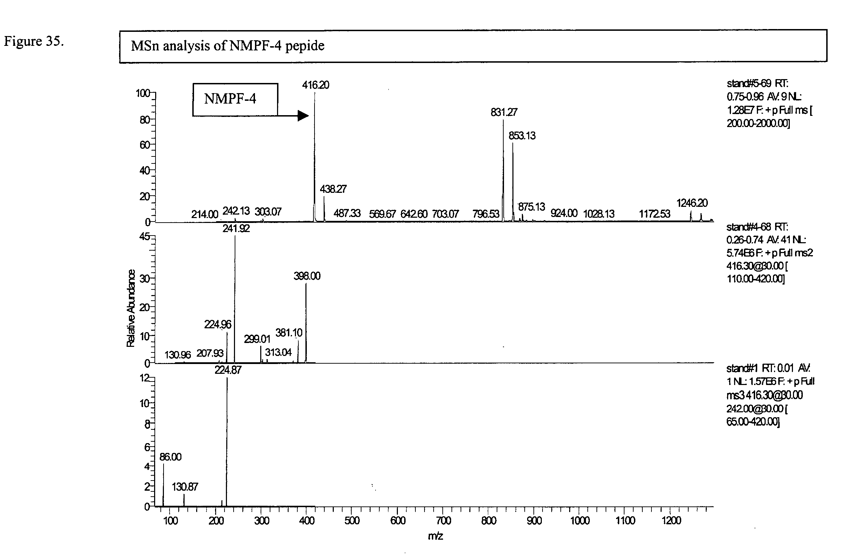

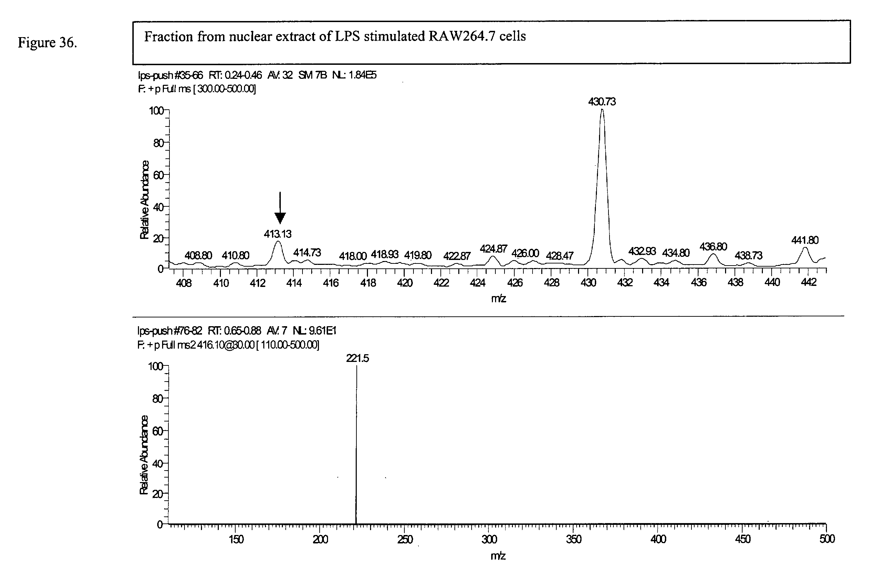

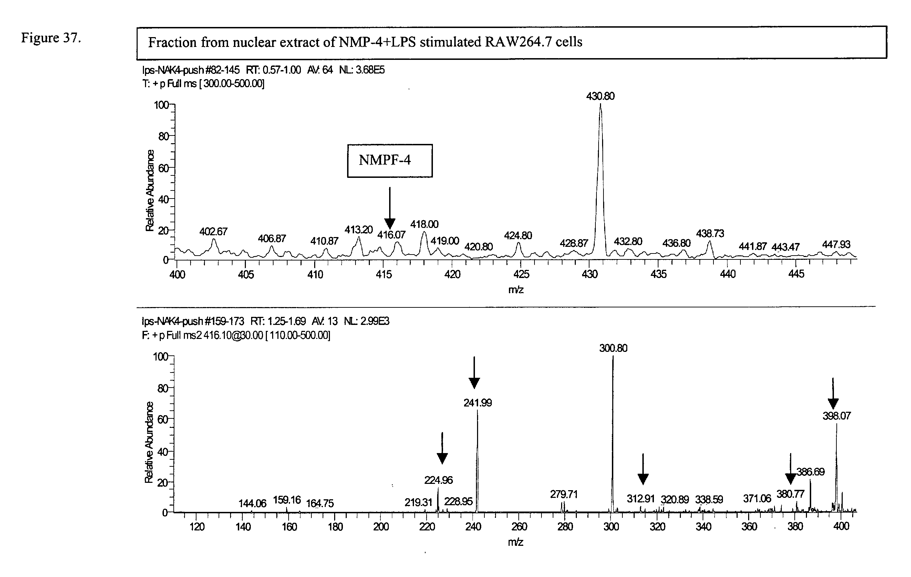

The molecular weight of this peptide is around 416 dalton. Besides 416 mass figure 35 shows some other molecular weights. This is to be explained by the high concentration of the peptide which induces the formation of dimers and sodium-adducts (m/z 416- [M+H]+, 438-[M+Na]+, 831-[2M+H]+, 853-[2M+Na]+, 1245-[3M+H]+, 1257-[3M+Na]+). Figure 36 shows the MS results of 10-15 min. fraction of nuclear extract obtained from LPS stimulated cells. These results show the absence of 416 dalton mass, while figure 37 shows the presence of 416 dalton mass of which the MSn data (figure 37) and MS-sequence confirm the presence of LQGV peptide in the nuclear protein extract obtained from LQGV+LPS stimulated RAW264.7 cells.

### Endotoxin shock model (Sepsis)

*Sepsis.* For the endotoxin model, BALB/c mice were injected i.p. with 8-9 mg/kg LPS (E. coli 026:B6; Difco Lab., Detroit, MI, USA). Control groups (PBS) were treated with PBS i.p. only. To test the effect of NMPF from different sources (synthetic, commercial hCG preparation [c-hCG]), we treated BALB/c with a dose of 300-700 IU of different hCG preparations (PG23;Pregnyl batch no. 235863, PG25; Pregnyl batch no. 255957) and with synthetic peptides (5 mg/kg) after two hours of LPS injection. In other experiments BALB/c mice were injected i.p. either with 10 mg/kg or with 11 mg/kg LPS (E. coli 026:B6; Difco Lab., Detroit, MI, USA). Subsequently mice were treated after 2 hours and 24 hours of LPS treatment with NMPF peptides.

*Semi-quantitative sickness measurements.* Mice were scored for sickness severity using the following measurement scheme:
1 Percolated fur, but no detectable behaviour differences compared to normal mice.
2 Percolated fur, huddle reflex, responds to stimuli (such as tap on cage), just as active during handling as healthy mouse.
3 Slower response to tap on cage, passive or docile when handled, but still curious when alone in a new setting.
4 Lack of curiosity, little or no response to stimuli, quite immobile.
5 Laboured breathing, inability or slow to self-right after being rolled onto back (moribund)
**6** Sacrificed

### RESULTS

### Endotoxin shock model (Sepsis)

*Sepsis experiments.* To determine the effect of synthetic peptides (NMPF) in high-dose LPS shock model, BALB/c mice were injected intraperitoneally with different doses of LPS and survival was assessed daily for 5 days. In this experiment (for the LPS endotoxin model) BALB/c mice were injected i.p. with 8-9 mg/kg LPS (E. coli 026:B6; Difco Lab., Detroit, MI, USA). Control groups (PBS) were treated with PBS i.p. only. We treated BALB/c mice with a dose of 300-700 IU of different hCG preparations (PG23;Pregnyl batch no. 235863, PG25; Pregnyl batch no. 255957) or with peptides (5 mg/kg) after two hours of LPS injection.

These experiments showed (table 1.) that NMPF peptides 4, 6, 66 and PG23 inhibited shock completely (all mice had in first 24 hours sickness scores not higher than 2; shortly thereafter they recovered completely and had sickness scores of 0), while peptides 2, 3 and 7 accelerated shock (all mice had in first 24 hours sickness scores of 5 and most of them died, while the control mice treated with LPS+PBS had sickness scores of 3-4 in first 24 hours and they most of them died after 48 hours with sickness scores of 5 (17% survival rate at 72 hours). In addition, peptides 1, 5, 8, 9, 11, 12, 13, 14 and 64 showed in number of different experiments variability in effectiveness as well as in the kind (inhibitory vs accelerating) of activity. This variability is likely attributable to the rate of breakdown of the various peptides, and the different effects the various peptides and their breakdown products have *in vivo*. In addition, these experiments also showed the variability in anti-shock activity in c-hCG preparations that is likely attributable to the variation in the presence of anti-shock and shock accelerating NMPF. Visible signs of sickness were apparent in all of the experimental animals, but the kinetics and obviously the severity of this sickness were significantly different. These data are representative of at least 10 separate experiments.

In table 2 we see the effect of ALA-replacement (PEPSCAN) in peptide LQG, LQGV, VLPALP, VLPALPQ in septic shock experiments. We conclude, that the change in even one amino acid by a neutral amino acid can lead to different activity. So, genomic differences as well as polymorphism in these peptides can regulate the immune response very precise. Derivatives of these peptides, for example (but not limited to) by addition of classical and non-classical amino acids or derivatives that are differentially modified during or after synthesis, for example benzylation, amidation, glycosylation, proteolytic cleavage, linkage to an antibody molecule or other cellular ligand etc. could also lead to a better effectiveness of the activity.

To determine whether treatment of BALB/c mice with NMPF inhibit septic shock at different stages of disease, synthetic peptides (NMPF) were injected i.p. at 2 and 24 hours after the induction of septic shock with high dose LPS (10 mg/kg). As shown in table 3 and 4., control mice treated PBS after the shock induction, reached a sickness score of 5 at 14 and 24 hours, and remained so after the second injection with PBS. The survival rate in control group mice was 0% at 48 hours. In contrast to control mice, mice treated with NMPF 9, 11, 12, 43, 46, 50 and 60 reached a maximum sickness score of 2-3 at 24 hours after the induction of septic shock and further reached a maximum sickness score of 1-2 at 48 hours after the second injection of NMPF. In addition, mice treated with NMPF 5, 7, 8, 45, 53 and 58 reached a sickness score of 5 and after the second injection with NMPF all mice returned to a sickness score of 1-2 and survival rates in NMPF groups were 100%. Mice treated with NMPF 3 reached sickness scores of 3-4 and the second NMPF injection did save these mice. These experiments show that NMPF peptides have anti-shock activity at different stages of the disease and NMPF have anti-shock activity even at disease stage when otherwise irreversible damage had been done. This indicates that NMPF have effects on different cellular levels and also have repairing and regenerating capacity.

### Dendritic cells experiments

*Mice.* The mouse strain used in this study was BALB/c (Harlan, Bicester, Oxon, GB). All mice used in experiments were females between 8 and 12 weeks of age. Mice were housed in a specific-pathogen-free facility. The Animal Use Committee at the Erasmus University Rotterdam, The Netherlands approved all studies.

*In vivo treatment.* At least six mice per group were injected intraperitonally (i.p) with LPS (10 mg/kg; Sigma). After 2 and 24 hrs of LPS induction, mice were injected i.p. with either NMPF (5 mg/kg) or Phosphate Buffered Saline (PBS), in a volume of 100 µl. LPS induced shock in this model had more than 90% mortality at 48 hrs.

*Bone marrow cell culture*. From treated mice, bone-marrow cells were isolated and cultured as follows. BALB/c mice were killed by suffocation with CO₂. The femurs and tibiae were removed and freed of muscles and tendons under aseptic conditions. The bones were placed in R10 medium (RPMI 1640, supplemented with 50 U/ml penicillin, 50 µg/ml streptomycin, 0.2 M Na-pyruvate, 2 mM glutamine, 50 µM 2-mercaptoethanol and 10% fetal calf serum (Bio Whittaker, Europe)).

The bones were then cleaned more thoroughly by using an aseptic tissue and were transferred to an ice cold mortier with 2 ml of R10 medium. The bones were crushed with a mortel to get the cells out of the bones. Cells were filtered through a sterile 100 µM filter (Beckton Dickinson Labware) and collected in a 50 ml tube (FALCON). This procedure was repeated until bone parts appeared translucent.

The isolated cells were resuspended in 10 ml of R10 and 30 ml of Geys medium was added. The cell suspension was kept on ice for 30 minutes, to lyse the red blood cells. Thereafter, the cells were washed twice in R10 medium. Upon initiation of the culture, the cell concentration was adjusted to 2 x 10⁵ cells per ml in R10 medium supplemented with 20 ng/ml recombinant mouse Granulocyte Monocyte-Colony Stimulating Factor (rmGM-CSF; BioSource International, Inc., USA) and seeded in 100 mm non-adherent bacteriological Petri dishes (Falcon). For each condition six Petri dishes were used and for further analysis, cells were pooled and analysed as described ahead. The cultures were placed in a 5% CO₂-incubator at 37°C. Every three days after culture initiation, 10 ml fresh R10 medium supplemented with rmGM-CSF at 20 ng/ml was added to each dish.

Nine days after culture initiation, non-adherent cells were collected and counted with a Coulter Counter (Coulter).

Alternatively, BM cells from untreated mice were isolated and cultured as described above and were *in vitro* treated with the following conditions: NMPF 4, NMPF 46, NMPF 7, NMPF 60 (20 µg/ml) were added to the culture either at day 0 or day 6 after culture initiation. Or LPS (1 µg/ml) was added to the culture at day 6 with or without the NMPF.

*Immunofluorescence staining*. Cells (2 x 10⁵) were washed with FACS-buffer (PBS with 1% BSA and 0.02% sodium azide), and transferred to a roundbottomed 96-well plate (NUNC). The antibodies used for staining were against MHC-II (I-A/I-E) PE and CD11c/CD18 FITC (PharMingen/Becton Dickinson, Franklin Lakes, NJ, USA).

Cells were resuspended in 200 µl FACS-buffer containing both of the antibodies at a concentration of 2.5 ng/µl per antibody. Cells were then incubated for 30 min at 4°C. Thereafter, cells were washed 3 times and finally resuspended in 200 µl FACS-buffer for flow-cytometric analysis in a FACSCalibur flow cytometer (Becton Dickinson, Heidelberg, Germany).

All FACS-data were analyzed with CellQuest software (Becton Dickinson, Heidelberg, Germany).

*Statistical analysis* All differences greater than 20% are considered to be significant.

### RESULTS

### Dendritic cells experiments

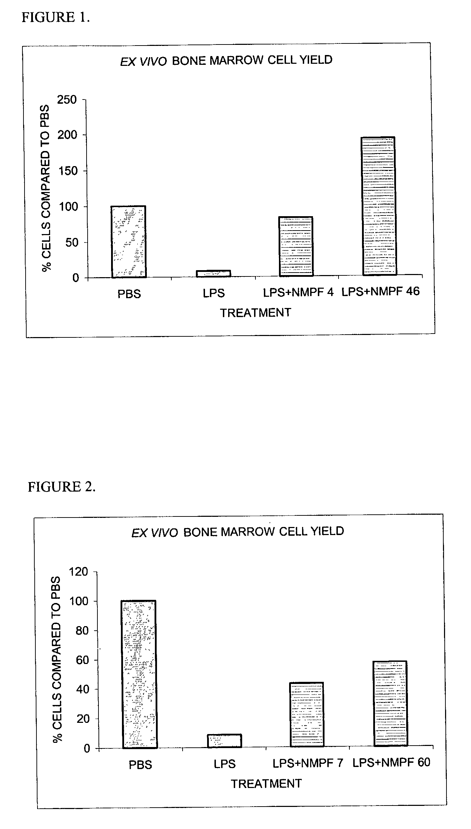

*Cell yield of ex vivo bone-marrow cell cultures.* To determine the *in vivo* effect of LPS and NMPF treatment on the cell yield obtained from a nine-day culture of bone-marrow with rmGM-CSF, cells were isolated from the BM of treated mice and cultured, harvested and counted as described. As shown in Figure 1 and 2, the cell yield of the bone-marrow cultures of LPS (10 mg/kg) treated mice is significantly decreased compared to PBS treated mice. Mice treated with NMPF 4, NMPF 7, NMPF 46 and NMPF 60 after LPS shock induction, had a significantly increased cell yield compared to LPS in the presence of rmGM-CSF. In addition, BM cultures from NMPF 46 treated mice gave a significantly increased cell yield even compared to the PBS group.

*Immunofluorescence staining of in vivo treated bone-marrow derived DC.* Culture of BM cells in the presence of rmGM-CSF gave rise to an increased population of cells that are positive for CD11c and MHC-II. Cells positive for these cell membrane markers are bone-marrow derived dendritic cells (DC). DC are potent antigen presenting cells (APC) and modulate immune responses. In order to determine the maturation state of myeloid derived DC, cells were stained with CD11c and MHC-II.

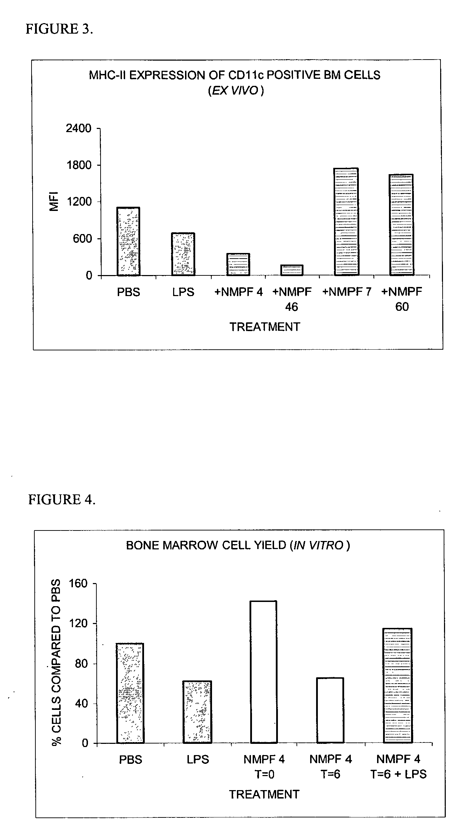

As shown in Figure 3, the expression of the MHC-II molecule was significantly decreased on CD11c-positive cells from LPS treated mice as compared to the PBS group. This decrease in MHC-II expression was further potentiated by the *in vivo* treatment with NMPF 4 and NMPF 46. However, treatment of mice with NMPF 7 and NMPF 60 significantly increased the expression of the MHC-II molecule even as compared to the PBS group.

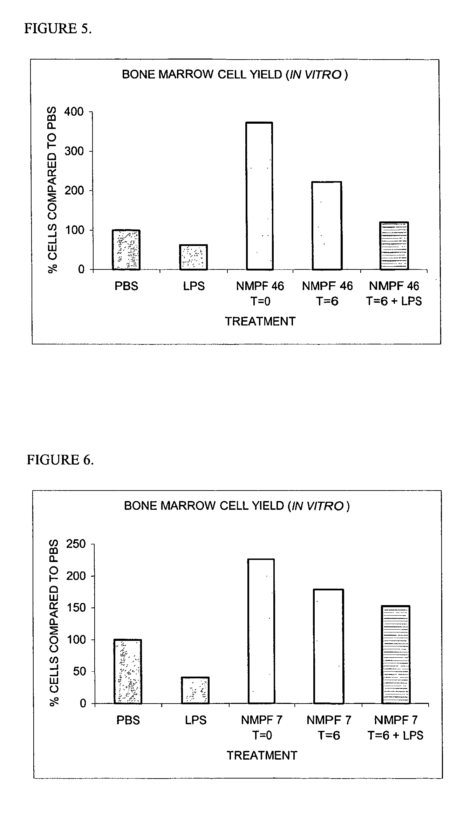

*Cell yields of in vitro bone-marrow cell cultures.* To determine the effect of LPS and NMPF *in vitro* on the cell yield of a nine-day culture of bone-marrow cells, we isolated the BM cells from untreated BALB/c mice and cultured in the presence of rmGM-CSF. In addition to rmGM-CSF, cultures were supplemented with NMPF at either day 0 or day 6 with or without the addition of LPS at day 6. As shown in Figure 4-7, there is a significant decrease in cell yield in LPS treated BM cells as compared to PBS. BM cells treated with NMPF 4, 7, 46 or 60 at time point t=0 or t=6 without LPS, showed a significant increase in cell yield as compared to the PBS group. However, BM cell cultures treated with NMPF 4 at time point t=6 showed significant decrease in cell yield as compared to the PBS group and this effect is comparable with the effect of LPS (Figure 4). In addition, BM cells treated with NMPF 4, 7, 46 or 60 at time point t=6 in combination with LPS showed a significant increase in cell yield as compared to the LPS group and even in the group of NMPF 7 the cell yield was significantly increased as compared to the PBS group.

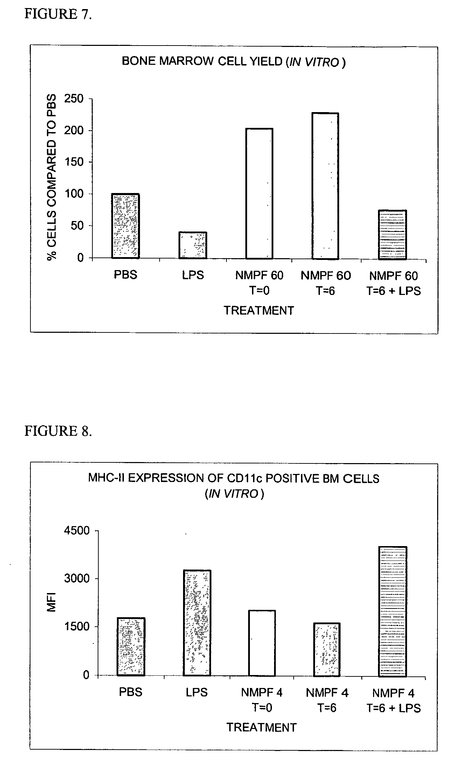

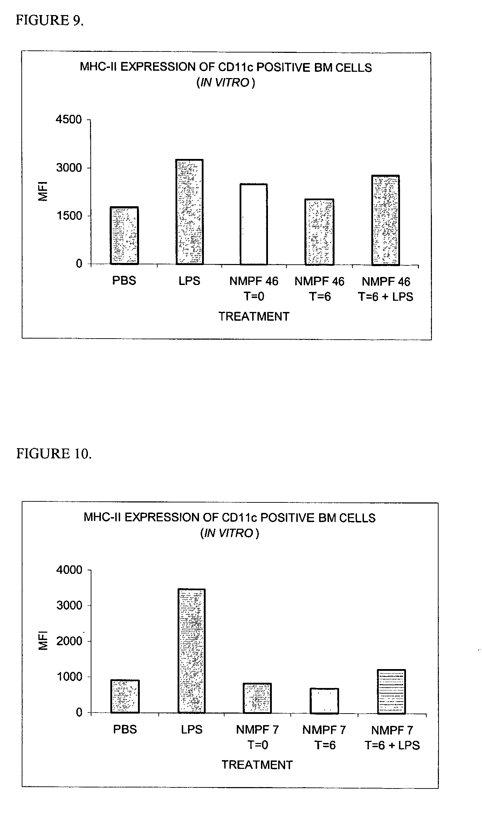

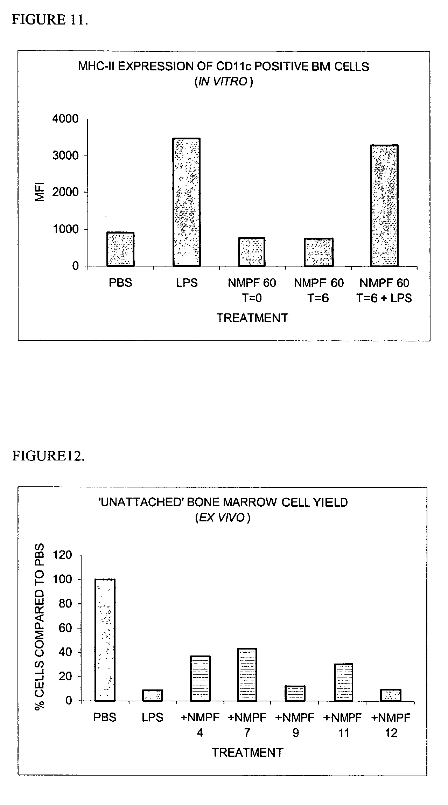

*Immunofluorescence staining of in vitro treated bone-marrow derived DC.* To determine the maturation state of DC, CD11c positive cells were stained for MHC-II antibody. Figure 7-11 show that there is an opposite effect of LPS on MHC-II expression as compared to *in vivo* experiments, namely, MHC-II expression is significantly increased with LPS treatment *in vitro* as compared to PBS. NMPF 4 with LPS further potentiated the effect of LPS, while NMPF 7 with or without LPS (t=6), significantly inhibited the expression of MHC-II as compared to LPS and PBS, respectively. However, cells treated with NMPF 46 without LPS (t=0) showed significantly increased expression of MHC-II on CD11c positive cells. Furthermore, no significant differences were found in the group NMPF 60 with or without LPS on MHC-II expression as compared to LPS and PBS treated cells.

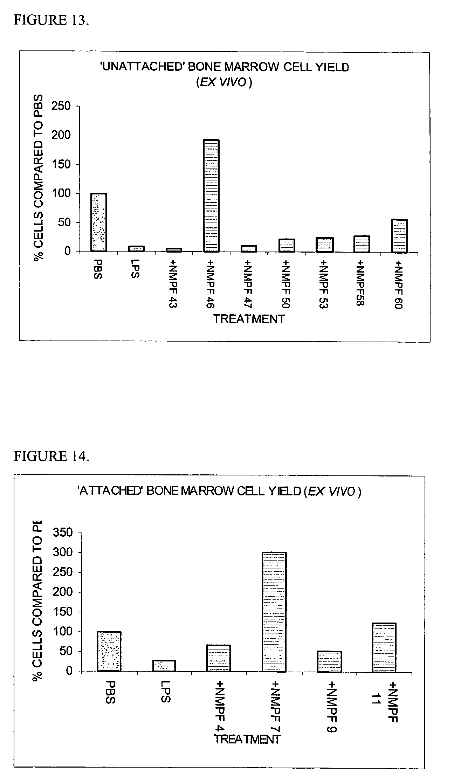

To determine the *in vivo* effect of LPS and NMPF treatment on the cell yield obtained from a nine-day culture of bone-marrow with rmGM-CSF, cells were isolated from the BM of treated mice and cultured, harvested and counted as described. The cell yield of 'attached' cells was significant increased with NMPF 4, 7, 9, 11, 43, 46, 47, 50, 53, 58 60 and even in the group of NMPF 7, 46 and 60 the cell yield was significant increased as compared to the PBS group (figure 14-15). In addition, cell yield of 'un-attached' cells was significant increased with NMPF 4, 7, 9, 11, 46, 50, 53, 58 60 and agin in the group of NMPF 46 the cell yield was significant increased as compared to the PBS group (figure 12-13).

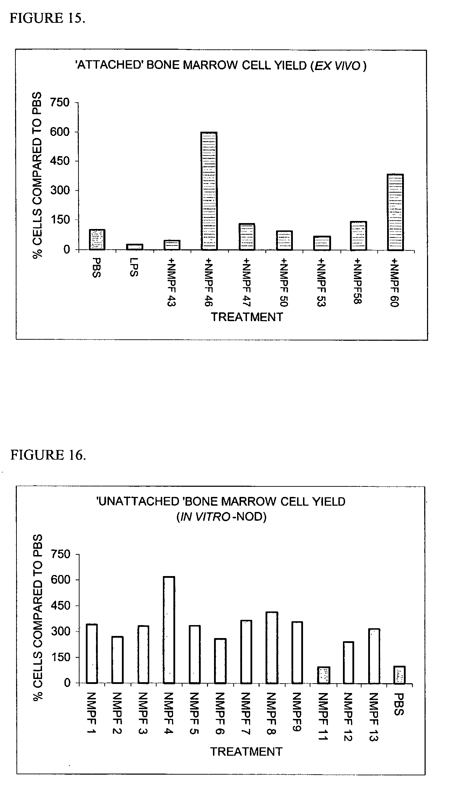

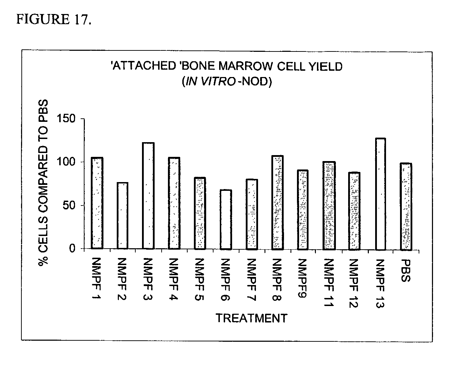

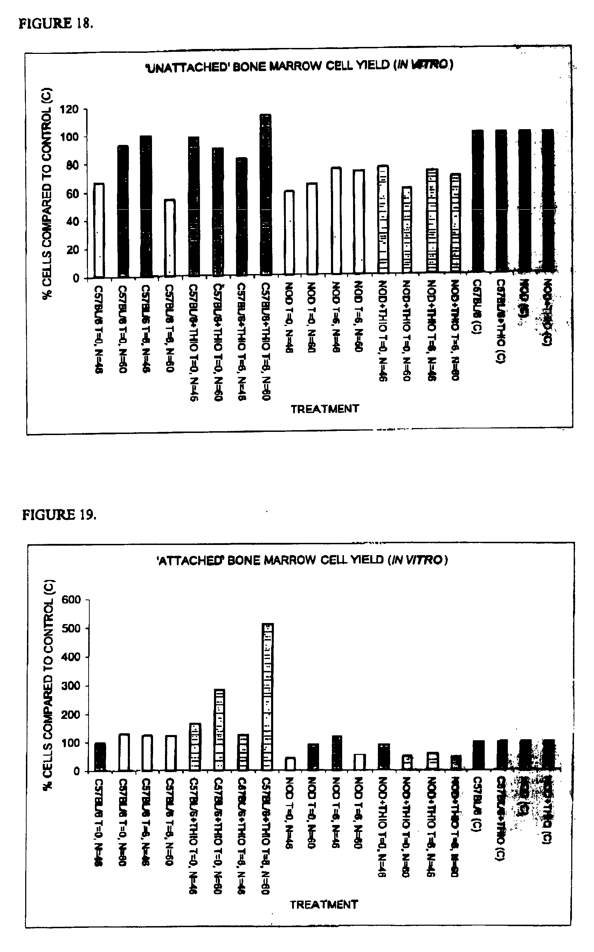

To determine the effect of LPS and NMPF *in vitro* on the cell yield of a nine-day culture of bone-marrow cells of female NOD mice, we isolated the BM cells from untreated NOD mice and cultured in the presence of rmGM-CSF. In addition to rmGM-CSF, cultures were supplemented with NMPF. In these experiments the bone-marrow cell yield of 'un-attached' cells was significant increased with NMPF 1,2,3,4,5,6,7,8,9, 12 and 13 as compared to PBS group and no effect was observed with NMPF 11 (figure 16). The 'attached' bone-marrow cells of these experiments showed different yield than the 'un-attached' cells, namely there was a significant increased in cell yield in cultures treated with NMPF 3 and 13, while cultures treated with NMPF 2 and 6 showed significant decrease in the cell yield as compared to PBS (figure 17) (more additional results are summarised in table 5).

### Coronary Artery Occlusion (CAO) experiments

*CAO induction and treatment.* NMPF have immunoregulatory effects in chronic inflammatory as well as acute inflammatory mice models. Since certain cytokines like TGF-beta1, TNF-alpha, IL-1 and ROS (reactive oxygen species) have been implicated in irreversible myocardial damage produced by prolonged episodes of coronary artery occlusion and reperfusion in vivo that leads to ischaemia and myocardial infarct, we tested the cardio-protective properties of peptides in ad libitum fed male Wistar rats (300 g). The experiments were performed in accordance with the Guiding principles in the Care and Use of Animals as approved by the Council of the American Physiological Society and under the regulations of the Animal Care Committee of the Erasmus University Rotterdam. Shortly, rats (n=3) were stabilised for 30 minutes followed by i.v. 1 ml of peptide treatment (0.5 mg/ml) in 10 minutes. Five minutes after completion of treatment, rats were subjected to a 60-min coronary artery occlusion (CAO). In the last 5 minutes of CAO, rats were again treated over 10 minutes i.v. with 1 ml of peptide (0.5 mg/ml) followed by 120 minutes of reperfusion (IP). Experimental and surgical procedures are described in detail in Cardiovascular Research 37(1998) 76-81. At the end of each experiment, the coronary artery was re-occluded and was perfused with 10 ml Trypan Blue (0.4%, Sigma Chemical Co.) to stain the normally perfused myocardium dark blue and delineate the nonstained area at risk (AR). The heart was then quickly excised and cut into slices of 1 mm from apex to base. From each slice, the right ventricle was removed and the left ventricle was divided into the AR and the remaining left ventricle, using micro-surgical scissors. The AR was then incubated for 10 min in 37°C Nitro-Blue-Tetrazolium (Sigma Chemical Co.; 1 mg per 1 ml Sorensen buffer, pH 7.4), which stains vital tissue purple but leaves infarcted tissue unstained. After the infarcted area (IA) was isolated from the noninfarcted area, the different areas of the LV were dried and weighed separately. Infarct size was expressed as percentage of the AR. Control rats were treated with PBS.

### RESULTS

### Coronary Artery Occlusion (CAO) experiments

Our CAO data showed that 15 rats in controle group treated with only PBS had infarcted area of 70±2% (avearge±standard error) after 60-minutes of CAO followed by 2 hours of reperfusion. While rats treated with peptide VLPALP, LQGV, VLPALPQVVC, LQGVLPALPQ ,LAGV, LQAV and MTRV showed infarcted area of 62±6%, 55±6%, 55±5%, 67±2%, 51±4%, 62±6% and 68±2%, respectively. Here, we see that certain peptides (such as VLPALP, LQGV, VLPALPQVVC, LAGV) have protective on the area at risk for infarction. In addition, peptide LQAV showed smaller infarcted area but in some instances the area was haemorhagic infarcted. In addition NMPF-64 (LPGCPRGVNPVVS) had also protective effect (35%) in CAO experiment. It is important to note that mice treated with certain above mentioned peptides showed less viscousity of blood. Apart from immunological effect, these peptides may have also effect on blood coagulation system directly or indirectly since there is certain homology with blood coagulation factors ( for additional results of NMPF peptides see table 5.) So, in both model the circulatory system plays an important role in the pathogensis of the disease.

### Chicken eggs experiments

*In vivo treatment of fertilised chicken eggs with NMPF*. Fertile chicken eggs (Drost Loosdrecht BV, the Netherlands) were incubated in a diagonal position in an incubator (Pas Reform BV, the Netherlands) at 37 °C and 32% relative humidity.

Solutions of NMPF peptides (1mg/ml) and VEGF were made in PBS. At least ten eggs were injected for every condition. The treatment was performed as follows: on day 0 of incubation, a hole was drilled into the eggshell to open the air cell. A second hole was drilled 10 mm lower and right from the first hole for injection. The holes in the eggshell were disinfected with jodium. The NMPF peptides (100□g/egg) and/or VEGF (100 ng/ml) were injected in volume of 100µl. The holes in the eggshell were sealed with tape (Scotch Magic™ Tape, 3M) and the eggs were placed into the incubator.

*Quantification of angiogenesis.* On day 7 of incubation, the eggs were viewed under an UV lamp to check if the embryos were developing in a normal way and the dead embryos were counted. On day 8 of incubation, the embryos were removed from the eggs by opening the shell at the bottom of the eggs. The shell membrane was carefully dissected and removed. The embryos were placed in a 100-mm Petri dish. The embryo and the blood vessels were photographed (Nikon E990, Japan) *in vivo* with the use of a microscope (Zeiss Stemi SV6, Germany). One overview picture was taken and 4 detail pictures of the blood vessels were taken. Only eggs with vital embryos were evaluated.

*Data analysis.* Quantification of angiogenesis was accomplished by counting the number of blood vessels branches. Quantification of vasculogeneis was accomplished by measuring the blood vessel thickness. The number of blood vessel branches and the blood vessel thickness were counted in the pictures (4 pictures/egg) using Corel Draw 7. Thereafter, the number of blood vessel branches and the thickness of the blood vessels were correlated to a raster of microscope (10 mm²) for comparison.

The mean number of branches and the mean blood vessel thickness of each condition (n=10) were calculated and compared to the PBS control eggs using a Student's T-test.

### RESULTS

### Chicken eggs experiments

In order to determined the effect of NMPF on angiogenesis and vasculogenesis we treated ferterized chicken eggs with NMPF or NMPF in combination with VEGF as described in materials and methods section. Figures 20-0 show that NMPF 3, 4, 9 and 11 promoted angiogenesis (p<0.05), while NMPF VEGF, 7, 43, 44, 45, 46, 51 and 56 inhibited angiogenesis (p<0.05). NMPF 6, 7, 12, 45, 46 and 66 were able to inhibit angiogensis induced by VEGF. Moreover, NMPF 6 itself did not show any effect on angiogensis, but it inhibited (p<0.05) NMPF 3 induced angiogenesis.

Figures 31-32 show that NMPF 1, 2,3, 4, 6, 7, 8, 12, 50, 51, and 52 had vasculogenesis inhibiting (p<0.05) effect, while only NMPF 44 promoted (p<0.05) vasculogensis.

### NOD experiment

Female NOD mice at the age of 13-14 weeks were treated i.p. with PBS (n=6) or NMPF peptides (VLPALPQVVC, LQGV, GVLPALPQ, VLPALP, VLPALPQ, MTRV, LPGCPRGVNPVVS, CPRGVNPVVS, LPGC, MTRVLQGVLPALPQVVC) (5 mg/kg, n=6) three times a week for 2 weeks. After fours days urine was checked for the presence of glucose (Gluketur Test; Boehringer Mannheim, Mannheim, Germany). All mice used in these studies were maintained in a pathogen-free facility. They were given free access to food and water. The experiments were approved by the Animal Experiments Committee of the Erasmus University Rotterdam.

### RESULTS

### NOD experiment

In order to determine whether NMPF has effect on the disease development in NOD mice, we tested NMPF on pre-diabetic female NOD mice at the age of 13-14 weeks. After only two weeks of treatment glucosuria data of all NOD mice was analysed at the of 17 weeks. Profound anti-diabetic effect (negative for glucosuria) was observed in different NMPF groups as compared to PBS group, especially in NMPF groups treated with peptide VLPALPQVVC, VLPALP, MTRV, LPGCPRGVNPVVS and LPGC. In addition, impairment of the glucose tolerance test is positively correlated to insulitis, but negatively correlated to the number of functional beta cells, also this test showed that NOD mice treated with NMPF were tolerant for glucose as compared to PBS group.

### Analysis of different peptides in data bases

Examples of different data bases in which peptides were analysed are:
Proteomics tools: Similarity searches
BLAST data base (ExPasy, NCBI)
SMART (EMBL)
PATTINPROT (PBIL)
Post-translational modification prediction
SignalP (CBS)
Primary structure analysis
HLA Peptide Binding Predictions (BIMAS)
Prediction of MHC type I and II peptide binding
(SYFPEITHI)
Amino acid scale representation (Hydrophobicity, other conformational parameters, etc.) (PROTSCALE)
Representations of a protein fragment as a helical wheel(HelixWheel / HelixDraw)

### RESULTS

### Relevant oligopeptides derivable from protein data bases

pdb | 1DE7 | 1DE7-A INTERACTION OF FACTOR XIII ACTIVATION PEPTIDE WITH ALPHA- THROMBIN
   LQGV, LQGVV, LQGVVP
pdb | 1DL6 | 1DL6-A SOLUTION STRUCTURE OF HUMAN TFIIB N-TERMINAL DOMAIN
   LDALP
pdb | 1QMH | 1QMH-A CRYSTAL STRUCTURE OF RNA 3'-TERMINAL PHOSPHATE CYCLASE, AN UBIQUITOUS ENZYME
   LQTV, VLPAL, LVLQTVLPAL
pdb | 1LYP | 1LYP CAP18 (RESIDUES 106 - 137)
   IQG, IQGL, LPKL, LLPKL
pdb | 1B9O | 1B9O-A HUMAN ALPHA-LACTALBUMIN
   LPEL
pdb | 1GLU | 1GLU-A GLUCOCORTICOID RECEPTOR (DNA-BINDING DOMAIN)
   PARP
pdb | 2KIN | 2KIN-B KINESIN (MONOMERIC) FROM RATTUS NORVEGICUS
   MTRI
pdb | 1SMP | 1SMP-I MOL_ID: 1; MOLECULE: SERRATIA METALLO PROTEINASE; CHAIN: A
   LQKL, LQKLL, PEAP, LQKLLPEAP
pdb | 1ES7 | 1ES7-B COMPLEX BETWEEN BMP-2 AND TWO BMP RECEPTOR IA ECTODOMAINS
   LPQ, PTLP, LQPTL
pdb | 1BHX | 1BHX-F X-RAY STRUCTURE OF THE COMPLEX OF HUMAN ALPHA THROMBIN WITH THE INHIBITOR SDZ 229-357
   LQV, LQVV
pdb | 1VCB | 1VCB-A THE VHL-ELONGINC-ELONGINB STRUCTURE
   PELP
pdb | 1CQK | 1CQK-A CRYSTAL STRUCTURE OF THE CH3 DOMAIN FROM THE MAK33 ANTIBODY
   PAAP, PAAPQ, PAAPQV
ndb | 1FCB | 1FCB-A FLAVOCYTOCHROME
   LQG,
pdb | 1LDC | 1LDC-A L-LACTATE DEHYDROGENASE: CYTOCHROME C OXIDOREDUCTASE (FLAVOCYTOCHROME B=2=) (E.C.1.1.2.3) MUTANT WITH TYR 143 REPLACED BY PHE (Y143F) COMPLEXED WITH PYRUVATE
   LQG
pdb | 1BFB | 1BFB BASIC FIBROBLAST GROWTH FACTOR COMPLEXED WITH HEPARIN TETRAMER FRAGMENT
   LPAL, PALP, PALPE
pdb | 1MBF | 1MBF MOUSE C-MYB DNA-BINDING DOMAIN REPEAT 1
   LPN
pdb | 1R2A | 1R2A-A THE MOLECULAR BASIS FOR PROTEIN KINASE A
   LQG, LTELL
pdb | 1CKA | 1CKA-B C-CRK (N-TERMINAL SH3 DOMAIN) (C-CRKSH3-N) COMPLEXED WITH C3G PEPTIDE (PRO-PRO-PRO-ALA-LEU-PRO-PRO-LYS-LYS-ARG)
   PALP
pdb | 1RLQ | 1RLQ-R C-SRC (SH3 DOMAIN) COMPLEXED WITH THE PROLINE-RICH LIGAND RLP2 (RALPPLPRY) (NMR, MINIMIZED AVERAGE STRUCTURE)
   LPPL, PPLP
pdb | 1TNT | 1TNT MU TRANSPOSASE (DNA-BINDING DOMAIN) (NMR, 33 STRUCTURES)
   LPG, LPGL, LPK
pdb | 1GJS | 1GJS-A SOLUTION STRUCTURE OF THE ALBUMIN BINDING DOMAIN OF STREPTOCOCCAL PROTEIN G
   LAAL, LAALP
pdb | 1GBR | 1GBR-B GROWTH FACTOR RECEPTOR-BOUND PROTEIN 2 (GRB2, N-TERMINAL SH3 DOMAIN) COMPLEXED WITH SOS-A PEPTIDE (NMR, 29 STRUCTURES)
   LPKL, PKLP
pdb | 1A78 | 1A78-A COMPLEX OF TOAD OVARY GALECTIN WITH THIO-DIGALACTOSE
   VLPSIP
pdb | 1ISA | 1ISA-A IRON(II) SUPEROXIDE DISMUTASE (E.C.1.15.1.1)
   LPAL, PALP
pdb | 1FZV | 1FZV-A THE CRYSTAL STRUCTURE OF HUMAN PLACENTA GROWTH FACTOR-1 (PLGF-1), AN ANGIOGENIC PROTEIN AT 2.0A RESOLUTION
   PAVP, MLPAVP
pdb | 1JLI | 1JLI HUMAN INTERLEUKIN 3 (IL-3) MUTANT WITH TRUNCATION AT BOTH N- AND C-TERMINI AND 14 RESIDUE CHANGES, NMR, MINIMIZED AVERAGE
   LPC, LPCL, PCLP
pdb | 1HSS | 1HSS-A 0.19 ALPHA-AMYLASE INHIBITOR FROM WHEAT
   VPALP
pdb | 3CRX | 3CRX-A CRE RECOMBINASE/DNA COMPLEX INTERMEDIATE I
   LPA, LPAL, PALP
pdb | 1PRX | 1PRX-A HORF6 A NOVEL HUMAN PEROXIDASE ENZYME
   PTIP, VLPTIP
pdb | 1RCY | 1RCY RUSTICYANIN (RC) FROM THIOBACILLUS FERROOXIDANS
   VLPGFP
pdb | 1A3Z | 1A3Z REDUCED RUSTICYANIN AT 1.9 ANGSTROMS
   PGFP, VLPGFP
pdb | 1GER | 1GER-A GLUTATHIONE REDUCTASE (E.C.1.6.4.2) COMPLEXED WITH FAD
   LPALP, PALP
pdb | 1PBW | 1PBW-A STRUCTURE OF BCR-HOMOLOGY (BH) DOMAIN
   PALP
pdb | 1BBS | 1BBS RENIN (E.C.3.4.23.15)
   MPALP

AI188872 11.3 366 327 18 382 [Homo sapiens]qd27c01.x1 Soares_placenta_8to9weeks_2NbHP8to9W Homo sapiens cDNA clone IMAGE: 1724928 3' similar to gb:J00117 CHORIOGONADOTROPIN BETA CHAIN PRECURSOR (HUMAN);, mRNA sequence.; minus strand; translated
   MXRVLQGVLPALPQVVC, MXRV, MXR,
AI126906 19.8 418 343 1 418 [Homo sapiens]qb95f01.x1 Soares_fetal_heart_NbHH19W Homo sapiens cDNA clone IMAGE:1707865 3' similar to gb:J00117 CHORIOGONADOTROPIN BETA CHAIN PRECURSOR (HUMAN);, mRNA sequence.; minus strand; translated
   ITRVMQGVIPALPQVVC
AI221581 29.1 456 341 23 510 [Homo sapiens]qg20a03.x1 Soares_placenta_8to9weeks_2NbHP8to9W Homo sapiens cDNA clone IMAGE:1760044 3' similar to gb:J00117 CHORIOGONADOTROPIN BETA CHAIN PRECURSOR (HUMAN);, mRNA sequence.; minus strand; translated
   MTRVLQVVLLALPQLV
Mm.42246.3 Mm.42246 101.3 837 304 28 768 GENE=Pck1 PROTSIM=pir:T24168 phosphoenolpyruvate carboxykinase 1, cytosolic; translated
   KVIQGSLDSLPQAV, LDSL, LPQ
Mm.22430.1 Mm.22430 209.4 1275 157 75 1535 GENE=Ask-pending PROTSIM=pir:T02633 activator of S phase kinase; translated
   VLQAILPSAPQ, LQA, LQAIL, PSAP, LPS
Hs.63758.4 Hs.63758 93.8 3092 1210 51 2719 GENE=TFR2 PROTSIM=pir:T30154 transferrin receptor 2; translated
   KVLQGRLPAVAQAV, LQG, LPA, LPAV
Mm.129320.2 Mm.129320 173.0 3220 571 55 2769 GENE= PROTSIM=pir:T16409 Sequence 8 from Patent WO9950284; translated
   LVQKVVPMLPRLLC, LVQ, LPRL, PMLP
Mm.22430.1 Mm.22430 209.4 1275 157 75 1535 GENE=Ask-pending PROTSIM=pir:T02633 activator of S phase kinase; translated
   VLQAILPSAPQ, LQA, LQAIL, PSAP, PSAPQ
P20155 IAC2_HUMAN Acrosin-trypsin inhibitor II precursor (HUSI-II) [SPINK2] [Homo sapiens]
   LPGCPRHFNPV, LPG, LPGC
Rn.2337.1 Rn.2337 113.0 322 104 1 327 GENE= PROTSIM=PRF:1402234A Rat pancreatic secretory trypsin inhibitor type II (PSTI-II) mRNA, complete cds; minus strand; translated
   LVGCPRDYDPV, LVG, LVGC
Hs.297775.1 Hs.297775 43.8 1167 753 31 1291 GENE= PROTSIM=sp:O00268 ESTs, Weakly similar to T2D3_HUMAN TRANSCRIPTION INITIATION FACTOR TFIID 135 KDA SUBUNIT [H.sapiens]; minus strand; translated
   PGCPRG, PGCP
Mm.1359.1 Mm.1359 PROTSTM=pir.A39743 urokinase plasmiogen activator receptor
   LPGCP, PGCP, LPG, LPGC
sptrembl | O56177 | O56177 ENVELOPE GLYCOPROTEIN
   VLPAAP, PAAP
sptrembl | Q9W234 | Q9W234 CG13509 PROTEIN.//:trembl | AE003458 | AE003458_7 gene: "CG13509"; Drosophila melanogaster genomic scaffold
   LAGTIPATP, LAG, PATP
swiss | P81272 | NS2B HUMAN NITRIC-OXIDE SYNTHASE IIB (EC 1.14.13.39) (NOS, TYPE II B) (NOSIIB) (FRAGMENTS)
   GVLPAVP, LPA, VLPAVP, PAVP
sptrembl | O30137 | O30137 HYPOTHETICAL 17.2 KDA
   GVLPALP, PALP, LPAL
sptrembl | Q9IYZ3 | Q9IYZ3 DNA POLYMERASE
   GLLPCLP, LPC, LPCL, PCLP
sptrembl | Q9PVW5 | Q9PVW5 NUCLEAR PROTEIN NP220
   PGAP, LPQRPRGPNP, LPQ, PRGP, PNP
Hs.303116.2 PROTSIM=pir;T33097 stromal cell-derived factor 2-like1; translated
   GCPR
pdb | 1DU3 | 1DU3-A CRYSTAL STRUCTURE OF TRAIL-SDR5
   GCPRGM
pdb | 1D0G | 1D0G-R CRYSTAL STRUCTURE OF DEATH RECEPTOR 5 (DR5) BOUND TO APO2L/TRAIL
   GCPRGM
pdb | 1BIO | 1BIO HUMAN COMPLEMENT FACTOR D IN COMPLEX WITH ISATOIC ANHYDRIDE INHIBITOR
   LQHV
pdb | 4NOS | 4NOS-A HUMAN INDUCIBLE NITRIC OXIDE SYNTHASE WITH INHIBITOR
   FPGC, PGCP
pdb | 1FL7 | 1FL7-B HUMAN FOLLICLE STIMULATING HORMONE
   PARP, VPGC
pdb | 1HR6 | 1HR6-A YEAST MITOCHONDRIAL PROCESSING PEPTIDASE
   CPRG, LKGC
pdb | 1BFA | 1BFA RECOMBINANT BIFUNCTIONAL HAGEMAN FACTOR/AMYLASE INHIBITOR FROM
   PPGP, LPGCPREV, LPGC, PGCP, CPRE
swissnew | P01229 | LSHB HUMAN Lutropin beta chain precursor
   MMRVLQAVLPPLPQVVC, MMR, MMRV, LQA, LQAV, VLPPLP, PPLP, QVVC, VVC, VLPPLPQ, AVLPPLP, AVLPPLPQ
swissnew | P07434 | CGHB PAPAN Choriogonadotropin beta chain precursor
   MMRVLQAVLPPVPQVVC, MMR, MMRV, LQA, LQAG, VLPPVP, VLPPVPQ, QVVC, VVC, AVLPPVP, AVLPPVPQ
swissnew | Q28376 | TSHB HORSE Thyrotropin beta chain precursor
   MTRD, LPK, QDVC, DVC, IPGC, PGCP
swissnew | P95180 | NUOB MYCTU NADH dehydrogenase I chain B
   LPGC, PGCP
sptrembl | Q9Z284 | Q9Z284 NEUTROPHIL ELASTASE
   PALP, PALPS
sptrembl | Q9UCG8 | Q9UCG8 URINARY GONADOTROPHIN PEPTIDE (FRAGMENT).
   LPGGPR, LPG, LPGG, GGPR
XP_028754 growth hormone releasing hormone [Homo sapiens]
   LQRG, LQRGV, LGQL

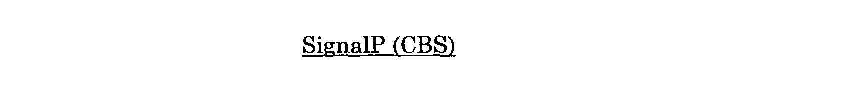

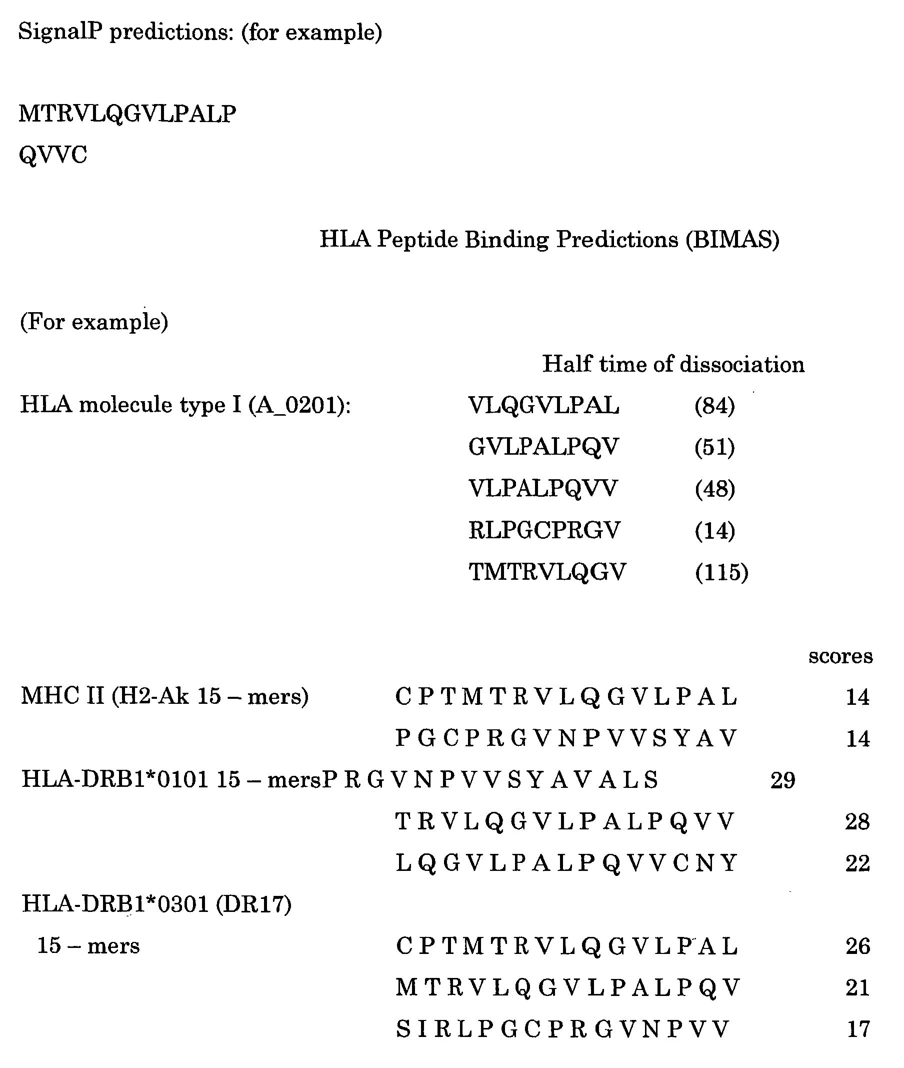

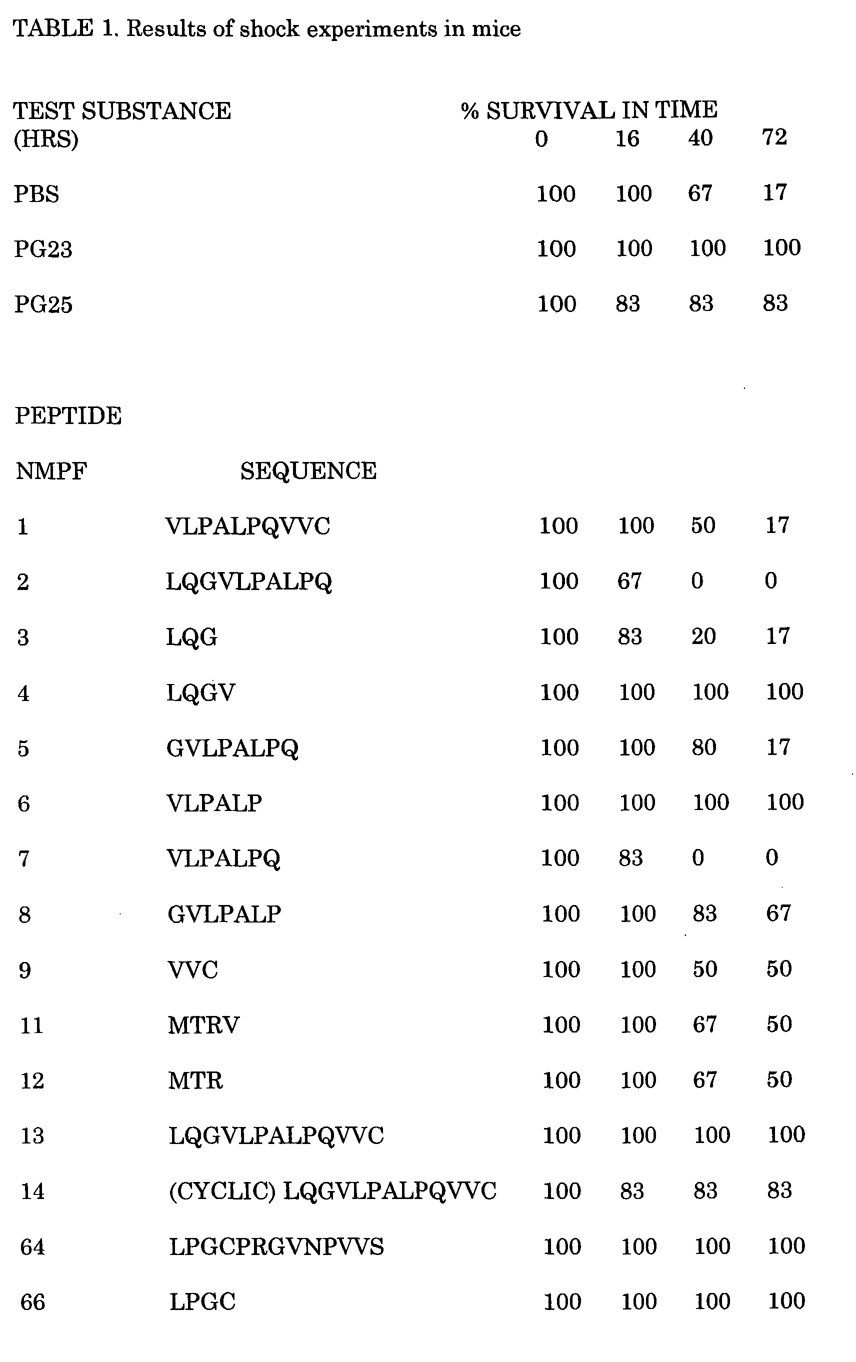

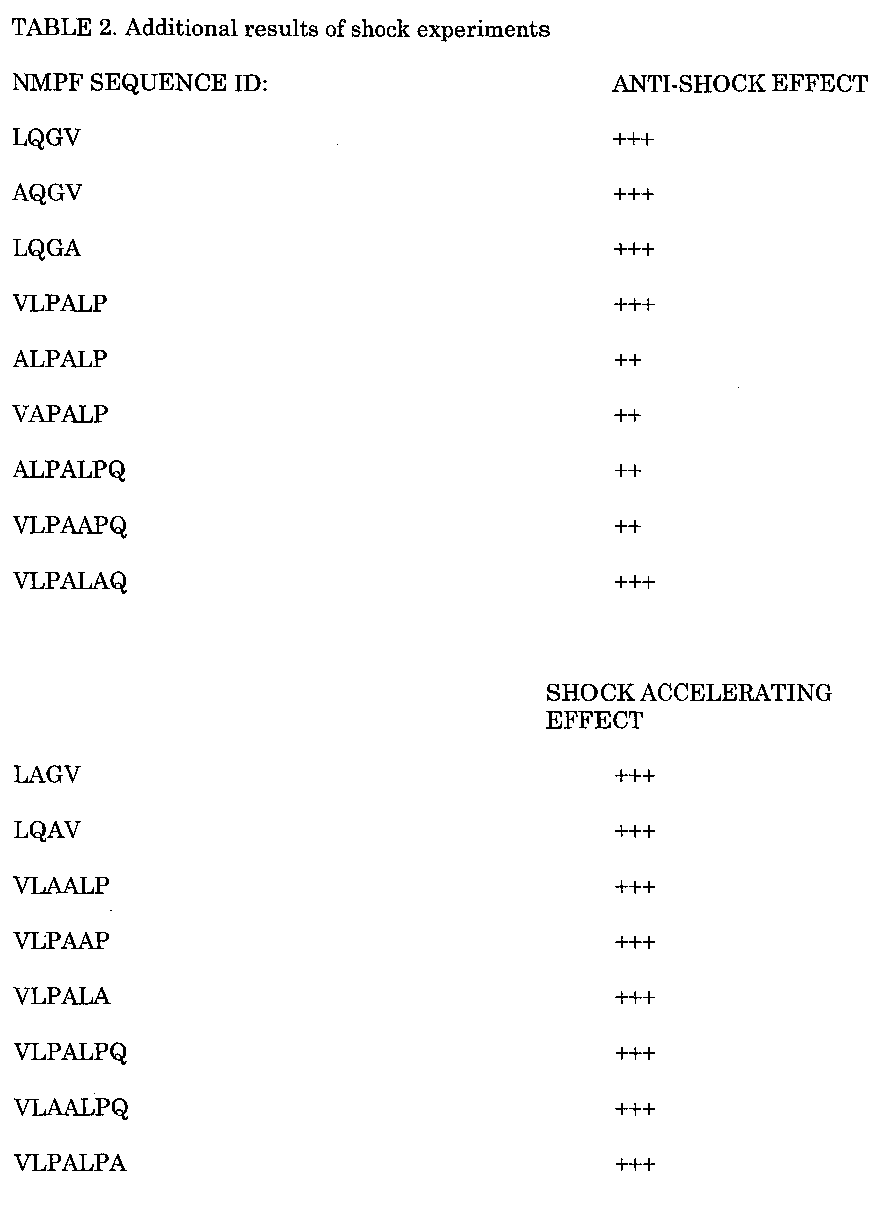

**TABLE 3.**

| Further additional results of shock experiments | | | | |
|---|---|---|---|---|
| NMPF PEPTIDES | % SURVIVAL IN TIME (HRS) | | | |
| | Tx | | Tx | |
| | 0 | 14 | 24 | 48 |
| PBS | 100 | 100 | 100 | 0 |
| NMPF-3 | 100 | 100 | 100 | 0 |
| NMPF-5 | 100 | 100 | 100 | 100 |
| NMPF-7 | 100 | 100 | 100 | 67 |
| NMPF-8 | 100 | 100 | 100 | 100 |
| NMPF-9 | 100 | 100 | 100 | 100 |
| NMPF-11 | 100 | 100 | 100 | 100 |
| NMPF-12 | 100 | 100 | 100 | 100 |
| NMPF-43 | 100 | 100 | 100 | 100 |
| NMPF-45 | 100 | 100 | 100 | 100 |
| NMPF-46 | 100 | 100 | 100 | 100 |
| NMPF-50 | 100 | 100 | 100 | 100 |
| NMPF-53 | 100 | 100 | 100 | 100 |
| NMPF-58 | 100 | 100 | 100 | 100 |
| NMPF-60 | 100 | 100 | 100 | 100 |

**TABLE 4.**

| Further additional results | | | | |
|---|---|---|---|---|
| NMPF PEPTIDES | SICKNESS SCORES | | | |
| | Tx | | Tx | |
| | 0 | 14 | 24 | 48 |
| PBS | 0,0,0,0,0,0 | 5,5,5,5,4,4 | 5,5,5,5,5,5 | †††††† |
| NMPF-3 | 0,0,0,0,0,0 | 3,3,3,3,3,4 | 4,4,4,4,4,4 | †††††† |
| NMPF-5 | 0,0,0,0,0,0 | 5,5,5,5,5,5 | 5,5,5,5,5,5 | 2,2,2,2,2,2 |
| NMPF-7 | 0,0,0,0,0,0 | 1,1,4,4,4,4 | 5,5,5,5,5,5 | 2,2,2,2,†† |
| NMPF-8 | 0,0,0,0,0,0 | 3,3,5,5,5,5 | 5,5,5,5,5,5 | 2,2,4,4,4,5 |
| NMPF-9 | 0,0,0,0,0,0 | 3,3,4,4,5,5 | 2,2,2,2,2,2 | 1,1,2,2,2,2 |
| NMPF-11 | 0,0,0,0,0,0 | 1,1,3,3,4,4, | 2,2,2,2,4,4 | 1,1,1,1,1,1 |
| NMPF-12 | 0,0,0,0,0,0 | 1,1,1,1,3,3 | 1,1,1,1,1,1 | 1,1,1,1,1,1 |
| NMPF-43 | 0,0,0,0,0,0 | 1,1,4,4,4,4 | 1,1,1,1,3,3 | 2,2,2,2,2,2 |
| NMPF-45 | 0,0,0,0,0,0 | 5,5,5,5,4,4 | 3,3,4,4,5,5 | 2,2,4,4,5,5 |
| NMPF-46 | 0,0,0,0,0,0 | 1,1,2,2,3,3 | 1,1,2,2,2,2 | 1,1,1,1,1,1 |
| NMPF-50 | 0,0,0,0,0,0 | 1,1,1,1,3,3 | 2,2,2,2,3,3 | 1,1,1,1,1,1 |
| NMPF-53 | 0,0,0,0,0,0 | 5,5,5,5,5,5 | 5,5,5,5,5,5 | 1,12,2,2,2 |
| NMPF-58 | 0,0,0,0,0,0 | 5,5,5,5,3,3 | 5,5,5,5,3,3 | 1,1,1,1,1,1 |
| NMPF-60 | 0,0,0,0,0,0 | 1,1,4,4,2,2 | 2,2,2,2,4,4 | 1,1,1,1,1,1 |

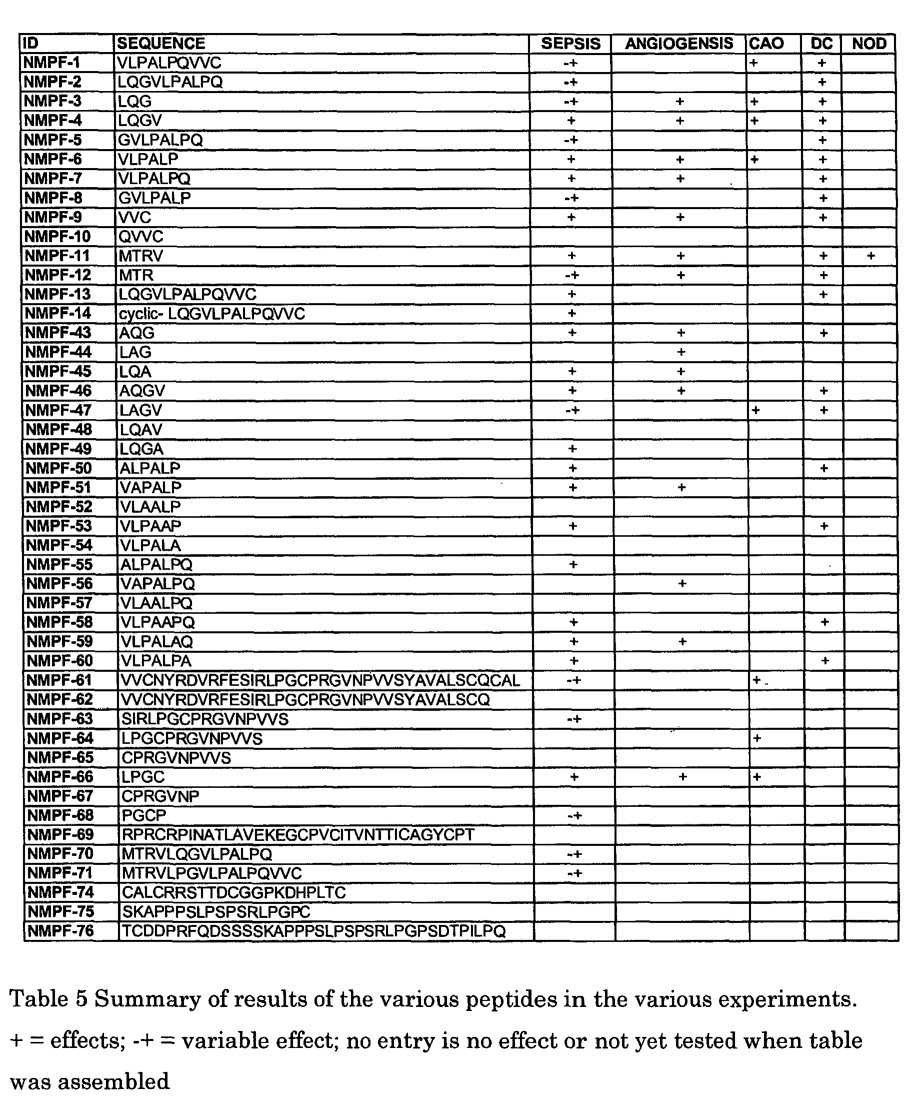

### Monkey experiment

Efficacy of NMPF, here a mixture 1:1:1 of LGQV, AQGV and VLPALP, administered in a gram-negative induced rhesus monkey sepsis model for prevention of septic shock.

Overwhelming inflammatory and immune responses are essential features of septic shock and play a central part in the pathogenesis of tissue damage, multiple organ failure, and death induced by sepsis. Cytokines, especially tumour necrosis factor (TNF)-□ interleukin (IL)-1□, and macrophage migration inhibitory factor (MIF) have been shown to be critical mediators of septic shock. Yet, traditional anti-TNF and anti-IL-1 therapies have not demonstrated much benefit for patients with severe sepsis. We have designed peptides that block completely LPS induced septic shock in mice, even when treatment with these peptides is started up to 24 hours after LPS injection. These peptides are also able to inhibit the production of MIF. This finding provides the possibility of therapeutic use of these peptides for the treatment of patients suffering from septic shock. Since primates are evolutionary more closer to humans, we tested these peptides for their safetv and effectiveness in a primate svstem.

| *EXPERIMENTAL DESIGN* | | | | |
|---|---|---|---|---|
| GROUP ) | EXPERIMENTAL TREATMENT (independent variable, e.g. placebo treated control group) | BIOTECHNIQUES | NUMBER | |
| animal I | i.v. infusion of a lethal dose of live Escherichia.coli (10E10 CFU/kg) + antibiotics + placebo treated | Live E.coli infusion Blood sampling No recovery (section) | N=1 | |
| animal II | i.v. infusion of a lethal dose of live Escherichia.coli (10E10 CFU/kg) + antibiotics + oligopeptide (5mg/kg) | Live E.coli infusion Blood sampling No recovery (section) | N=1 | |

Only naive monkeys were used in this preclinical study to exclude any interaction with previous treatments. The animals were sedated with ketamine hydrochloride. Animals were intubated orally and are allowed to breathe freely. The animals were kept anesthesized with O₂/N₂O/isoflurane. The animals received atropin as pre-medication for O₂/N₂O/isoflurane anesthesia. A level of surgical anesthesia was maintained during the 2 h infusion of *E.coli* and for 6 h following *E.coli* challenge after which the endothracheal tubes were removed and the animals were euthanized. Before bacteria were induced, a 1 hour pre-infusion monitoring of heart-rate and blood pressure was performed.

Two rhesus monkeys were infused with a 10 ^{10 CFU} per kg of the gram negative bacterium E.coli to induce a fatal septic shock. One monkey received placebo-treatment and was sacrificed within 6 hours after infusion of the bacteria without recovery from the anesthesia. The second monkey received treatment with test compound and was sacrificed at the same time point.

In a limited dose-titration experiment performed with the same bacterium strain in 1991 the used dose proved to induce fatal shock within 8 hours. In recent experiments a 3-fold lower dose was used inducing clear clinical and pathomorphological signs of septic shock without fatal outcome.

The monkeys were kept anaesthetized throughout the observation period and sacrificed 6 hours after the start of the bacterium infusion for pathological examination. The animals underwent a gross necropsy in which the abdominal and throrac cavities were opened and internal organs examined in situ.

### RESULTS

### Preliminary monkey results

An anti-shock effect of the test compound on sepsis in the monkey treated with the oligopeptide mixture , namely the inhibition of the effect of the sepsis in this early 6-hour trajectory of this primate model was observed. Immunomodulatory effects with these peptides have been observed *in vitro*/*ex vivo* such as, in T-cell assays the inhibition of pathological Th1 immune responses, suppression of inflammatory cytokines (MIF), increase in production of anti-inflammatory cytokines (IL-10, TGF-beta) and immunomodulatory effects on antigen presenting cells (APC) like dendritic cells, macrophages.

The following organs were weighed and a bacterial count were performed:
kidneys
liver
lungs
lymp nodes
gross lesions

Tissues of all organs were preserved in neutral aqueous phosphate buffered 4% solution of formaldehyde. Lymphoid organs were be cryopreserved. All tissues will be processed for histopathological examination.

### Genomic experiment

PM1 T-cell line was obtained from American Type Culture Collection (Manassas, VA) and was cultured at 37°C in 5% CO2. These cells were maintained and cultured in RPMI 1640, 10% fetal bovine serum, 2 mM L-glutamine, and antibiotics penicillin and streptomycin. For genomic experiements cells (2 x10⁶/ml) were incubated with phytohemagglutinin (PHA, 10□g/ml) and IL-2 (200 IU/ml) or PHA, IL-2 and peptide LQGV (10mg/ml) in a volume of 2 ml in 6-wells plates. After 4 h of cultures 10 x10⁶ cells were washed and prepared for genechip probe arrays experiment. The genechip expression analysis was performed according to the manufacturer's instructions (Expression Analysis, Technical Manual, Affymetrix Genechip). The following major steps outline Genechip Expression Analysis: 1) Target preparation 2) Target hybridization 3) Experiment and fluidics station setup 4) Probe Array washing and staining 5) Probe array scan and 6) Data analysis.

### RESULTS

### Genomic experiment

The gene chip expression analysis revealed that due to LQGV treatment of PM1 (T-cell line) cells for 4 hours in the presence of PHA/IL-2 down-regulated at least 120 genes more than 2 fold as compared to control PM1 cells (stimulated with PHA/IL-2) only. Moreover, at least 6 genes were up-regulated more than 2 fold in peptide treated cells as compared to control cells.

Down regulated genes due to treatment with LQGV in genomics experiment

### Fold Change/Descriptions

21.2 M11507 Human transferrin receptor mRNA, complete cds
   (_5, _M, _3 represent transcript regions 5 prime, Middle, and 3 prime respectively)
10.1 Human (c-myb) gene, complete primary cds, and five complete alternatively spliced cds
   (U22376 /FEATURE=cds#5 /DEFINITION=HSU22376)
9.7 Cluster Incl. X68836:H.sapiens mRNA for S-adenosylmethionine synthetase
   (cds=(65,1252) /gb=X68836 /gi=36326 /ug=Hs.77502 /len=1283)
9.3 M97935 Homo sapiens transcription factor ISGF-3 mRNA, complete cds
   (_5, _MA, MB, _3 represent transcript regions 5 prime, MiddleA, MiddleB, and 3 prime respectively)
8.7 Human mRNA for phosphatidylinositol transfer protein (PI-TPbeta), complete cds
   (D30037 /FEATURE= /DEFINITION=HUMPITPB)
7.5 Cluster Incl. U28964:Homo sapiens 14-3-3 protein mRNA, complete cds
   (cds=(126,863) /gb=U28964 /gi=899458 /ug=Hs.75103 /len=1030)
6.7 Human CDK tyrosine 15-kinase WEE1Hu (Wee1Hu) mRNA, complete cds
   (U10564 /FEATURE= /DEFINITION=HSU10564)
6.7 Homo sapiens E2F-related transcription factor (DP-1) mRNA, complete cds
   (L23959 /FEATURE= /DEFINITION=HUMDP1A)
6.5 Cluster Incl. W29030:55c4 Homo sapiens cDNA
   (gb=W29030 /gi=1308987 /ug=Hs.4963 /len=758)
6.1 Cluster Incl. U08997:Human glutamate dehydrogenase gene, complete cds
   (cds=(0,1676) /gb=U08997 /gi=478987 /ug=Hs.239377 /len=1677)
5.7 M97935 Homo sapiens transcription factor ISGF-3 mRNA, complete cds
   (_5, _MA, MB, _3 represent transcript regions 5 prime, MiddleA, MiddleB, and 3 prime respectively)
5.6 Cluster Incl. Y00638:Human mRNA for leukocyte common antigen (T200)
   (cds=(86,4000) /gb=Y00638 /gi=34280 /ug=Hs.170121 /len=4315)
5.3 Ras-Like Protein Tc21
5.3 H.sapiens mRNA for Fas/Apo-1 (clone pCRTM11-Fasdelta(4,7))
   ( X83492 /FEATURE=exons#1-2 /DEFINITION=HSFAS47)
4.8 Cluster Incl. AJ002428:Homo sapiens VDAC1 pseudogene
   (cds=(0,853) /gb=AJ002428 /gi=3183956 /ug=Hs.201553 /len=854)
4.7 Ras-Related Protein Rap 1b
4.6 Cluster Incl. AL080119:Homo sapiens mRNA; cDNA DKFZp564M2423 (from clone DKFZp564M2423)
   (cds=(85,1248) /gb=AL080119 /gi=5262550 /ug=Hs.165998 /len=2183)
4.5 Cluster Incl. AF047448:Homo sapiens TLS-associated protein TASR mRNA, complete cds
   (cds=(29,580) /gb=AF047448 /gi=2961148 /ug=Hs.239041 /len=620)
4.5 Cluster Incl. D14710:Human mRNA for ATP synthase alpha subunit, complete cds
   (cds=(63,1724) /gb=D14710 /gi=559324 /ug=Hs.155101 /len=1857)
4.5 Cluster Incl. X59618:H.sapiens RR2 mRNA for small subunit ribonucleotide reductase
   (cds=(194,1363) /gb=X59618 /gi=36154 /ug=Hs.75319 /len=2475)
4.5 Human mRNA for annexin II, 5 UTR (sequence from the 5 cap to the start codon)
   (D28364 /FEATURE= /DEFINITION=HUMAI23)
4.5 Cluster Incl. AA477898:zu34f08.r1 Homo sapiens cDNA, 5 end /clone=IMAGE-739911 /clone_end=5
   (gb=AA477898 /gi=2206532 /ug=Hs.239414 /len=449)
4.4 Cluster Incl. L19161:Human translation initiation factor eIF-2 gamma subunit mRNA, complete cds
   (cds=(0,1418) /gb=L19161 /gi=306899 /ug=Hs.211539 /len=1440gb=AA477898 / gi=2206532 /ug=Hs.239414 /len=449)
4.4 Human serine/threonine-protein kinase PRP4h (PRP4h) mRNA, complete cds
   (U48736 /FEATURE= /DEFINITION=HSU48736)
4.4 Cluster Incl. L43821:Homo sapiens enhancer of filamentation (HEF1) mRNA, complete cds
   (cds=(163,2667) /gb=L43821 /gi=1294780 /ug=Hs.80261 /len=3817)
4.4 Ras-Like Protein Tc21
4.4 Human (c-myb) gene, complete primary cds, and five complete alternatively spliced cds
   (U22376 /FEATURE=cds#3 /DEFINITION=HSU22376)
4.3 Cluster Incl. U18271:Human thymopoietin (TMPO) gene
   (cds=(313,2397) /gb=U18271 /gi=2182141 /ug=Hs.170225 /len=2796)
4.2 Fk506-Binding Protein, Alt. Splice 2
4.2 Human proliferating cell nuclear antigen (PCNA) gene, promoter region
   ( J05614 /FEATURE=mRNA /DEFINITION=HUMPCNAPRM)
4.1 Human insulin-stimulated protein kinase 1 (ISPK-1) mRNA, complete cds
   (U08316 /FEATURE= /DEFINITION=HSU08316)
4.1 Cluster Incl. W28732:50h7 Homo sapiens cDNA
   (gb=W28732 /gi=1308680 /ug=Hs.177496 /len=818)
4.1 Cluster Incl. Y00638:Human mRNA for leukocyte common antigen (T200)
   (cds=(86,4000) /gb=Y00638 /gi=34280 /ug=Hs.170121 /len=4315)
4 Homo sapiens putative purinergic receptor P2Y10 gene, complete cds
   (AF000545 /FEATURE=cds /DEFINITION=HSAF000545)
3.8 Cluster Incl. U08997:Human glutamate dehydrogenase gene, complete cds
   (cds=(0,1676) /gb=U08997 /gi=478987 /ug=Hs.239377 /len=1677)
3.6 Human mRNA for raf oncogene
   (X03484 /FEATURE=cds /DEFINITION=HSRAFR)
3.6 Cluster Incl. M32886:Human sorcin CP-22 mRNA, complete cds
   (cds=(12,608) /gb=M32886 /gi=338481 /ug=Hs.117816 /len=952)
3.6 Homo sapiens GTP-binding protein (RAB1) mRNA, complete cds
   (M28209 /FEATURE= /DEFINITION=HUMRAB1A)
3.5 Human FKBP-rapamycin associated protein (FRAP) mRNA, complete cds
   (L34075 /FEATURE= /DEFINITION=HUMFRAPX)
3.5 Human DNA topoisomerase II (top2) mRNA, complete cds
   (J04088 /FEATURE= /DEFINITION=HUMTOPII)
3.4 Human translation initiation factor eIF-2 gamma subunit mRNA, complete cds
   (L19161 /FEATURE= /DEFINITION=HUMIEF2G)
3.4 Human mRNA for pre-mRNA splicing factor SRp20, 5 UTR (sequence from the 5 cap to the start codon)
   (D28423 /FEATURE= /DEFINITION=HUMPSF82)
3.4 Cluster Incl. AA442560:zv75g07.r1 Homo sapiens cDNA, 5 end /clone=IMAGE-759516 /clone_end=5
   (gb=AA442560 /gi=2154438 /ug=Hs.135198 /len=566)
3.4 Cluster Incl. X98248:H.sapiens mRNA for sortilin /cds=(21,2522)
   (gb=X98248 /gi=1834494 /ug=Hs.104247 /len=3723)
3.3 Cluster Incl. AB020670:Homo sapiens mRNA for KIAA0863 protein, complete cds
   (cds=(185,3580) /gb=AB020670 /gi=4240214 /ug=Hs.131915 /len=4313)
3.3 Cluster Incl. W28869:53h2 Homo sapiens cDNA
   (gb=W28869 /gi=1308880 /ug=Hs.74637 /len=975)
3.3 Cluster Incl. Z12830:H.sapiens mRNA for SSR alpha subunit /cds=(29,889)
   (gb=Z12830 /gi=551637 /ug=Hs.76152 /len=974)
3.3 Cluster Incl. AL021546:Human DNA sequence from BAC 15E1 on chromosome 12.
   Contains Cytochrome C Oxidase Polypeptide VIa-liver precursor gene,
   60S ribosomal protein L31 pseudogene, pre-mRNA splicing factor SRp30c gene,
   two putative genes, ESTs, STSs and putative CpG islands
   (cds=(0,230) /gb=AL021546 /gi=2826890 /ug=Hs.234768 /len=547)
3.2 Cluster Incl. U78082:Human RNA polymerase transcriptional regulation mediator (h-MED6) mRNA, complete cds
   (cds=(50,523) /gb=U78082 /gi=2618737 /ug=Hs.167738 /len=885)
3.2 H.sapiens RbAp48 mRNA encoding retinoblastoma binding protein
   (X74262 /FEATURE=cds /DEFINITION=HSRBAP48)
3.1 Cluster Incl. M64174:Human protein-tyrosine kinase (JAK1) mRNA, complete cds
   (cds=(75,3503) /gb=M64174 /gi=190734 /ug=Hs.50651 /len=3541)
3.1 Cluster Incl. AI862521:wj15a06.x1 Homo sapiens cDNA, 3 end /clone=IMAGE-2402866 /clone_end=3
   (gb=AI862521 /gi=5526628 /ug=Hs.146861 /len=606)
3.1 Cluster Incl. W27517:31h6 Homo sapiens cDNA
   (gb=W27517 /gi=1307321 /ug=Hs.13662 /len=732)
3 Human rab GDI mRNA, complete cds
   (D13988 /FEATURE= /DEFINITION=HUMRABGDI)
3 Cluster Incl. AL080119:Homo sapiens mRNA; cDNA DKFZp564M2423 (from clone DKFZp564M2423)
   (cds=(85,1248) /gb=AL080119 /gi=5262550 /ug=Hs.165998 /len=2183)
3 Human cAMP-dependent protein kinase type I-alpha subunit (PRKAR1A) mRNA, complete cds
   (M33336 /FEATURE= /DEFINITION=HUMCAMPPK)
3 Cluster Incl. L75847:Human zinc finger protein 45 (ZNF45) mRNA, complete cds
   (cds=(103,2151) /gb=L75847 /gi=1480436 /ug=Hs.41728 /len=2409)
3 Cluster Incl. M21154:Human S-adenosylmethionine decarboxylase mRNA, complete cds
   (cds=(248,1252) /gb=M21154 /gi=178517 /ug=Hs.75744 /len=1805)
3 Cluster Incl. AA675900:g02504r Homo sapiens cDNA, 5 end /clone=g02504 /clone_end=5
   (gb=AA675900 /gi=2775247 /ug=Hs.119325 /len=647)
3 Cluster Incl. M97936:Human transcription factor ISGF-3 mRNA sequence
   (cds=UNKNOWN /gb=M97936 /gi=475254 /ug=Hs.21486 /len=2607)
2 M33336 /DEFINITION=HUMCAMPPK Human cAMP-dependent protein kinase type I-alpha subunit (PRKAR1A)
   mRNA, complete cds
2 U16720 /FEATURE=mRNA /DEFINITION=HSU16720 Human interleukin 10 (IL10) gene, complete cds
2 M33336 HUMCAMPPK Human cAMP-dependent protein kinase type I-alpha subunit (PRKAR1A) mRNA

2 U50079 /FEATURE= /DEFINITION=HSU50079 Human histone deacetylase HD1 mRNA, complete cds
2 U16720 /FEATURE=mRNA /DEFINITION=HSU16720 Human interleukin 10 (IL10) gene, complete cds
2 X87212 /FEATURE=cds /DEFINITION=HSCATHCGE H.sapiens mRNA for cathepsin C
2 Cluster Incl. AI740522:wg16b07.x1 Homo sapiens cDNA, 3 end /clone=IMAGE-2365237 /clone_end=3 /gb=AI740522
2 M21154 /FEATURE=mRNA /DEFINITION=HUMAMD Human S-adenosylmethionine decarboxylase mRNA, complete cds
2 X00737 /FEATURE=cds /DEFINITION=HSPNP Human mRNA for purine nucleoside phosphorylase (PNP; EC 2.4.2.1)
2.1 Cluster Incl. AF034956:Homo sapiens RAD51D mRNA, complete cds /cds=(124,993) /gb=AF034956 /gi=2920581
2.1 Ras Inhibitor Inf
2.1 Cluster Incl. M27749:Human immunoglobulin-related 14.1 protein mRNA, complete cds /cds=(118,759) /gb=M27749
2.1 Ras-Like Protein Tc4
2.1 X92106 /FEATURE=cds /DEFINITION=HSBLEO H.sapiens mRNA for bleomycin hydrolase
2.1 D88674 /FEATURE= /DEFINITION=D88674 Homo sapiens mRNA for antizyme inhibitor, complete cds
2.1 Cluster Incl. H15872:ym22b12.r1 Homo sapiens cDNA, 5 end /clone=IMAGE-48838 /clone_end=5 /gb=H15872
2.1 Cluster Incl. L07541:Human replication factor C, 38-kDa subunit mRNA, complete cds /cds=(9,1079) /gb=L07541
2.1 V01512 /FEATURE=mRNA#1 /DEFINITION=HSCFOS Human cellular oncogene c-fos (complete sequence)
2.1 Cluster Incl. L23959:Homo sapiens E2F-related transcription factor (DP-1) mRNA, complete cds /cds=(37,1269)
2.1 Stimulatory Gdp/Gtp Exchange Protein For C-Ki-Ras P21 And Smg P21
2.1 Cluster Incl. L13943:Human glycerol kinase (GK) mRNA exons 1-4, complete cds /cds=(66,1640) /gb=L13943 /gi=348166
2.1 Cluster Incl. X78925:H.sapiens HZF2 mRNA for zinc finger protein /cds=(0,2198) /gb=X78925 /gi=498722 /ug=Hs.2480
2.1 X74794 /FEATURE=cds /DEFINITION=HSP1CDC21 H.sapiens P1-Cdc21 mRNA
2.1 U78733 /FEATURE=mRNA#1 /DEFINITION=HSSMAD2S8 Homo sapiens mad protein homolog Smad2 gene, exon 11
2.2 Cluster Incl. L07540:Human replication factor C, 36-kDa subunit mRNA, complete cds /cds=(9,1031) /gb=L07540
2.2 Cluster Incl. AF040958:Homo sapiens lysosomal neuraminidase precursor, mRNA, complete cds /cds=(129,1376)
2.2 D00596 /FEATURE=cds /DEFINITION=HUMTS1 Homo sapiens gene for thymidylate synthase, exons 1, 2, 3, 4, 5, 6, 7,
2.2 Cluster Incl. AI659108:tu08c09.x1 Homo sapiens cDNA, 3 end /clone=IMAGE-2250448 /clone_end=3 /gb=AI659108
2.2 Cluster Incl. AF042083:Homo sapiens BH3 interacting domain death agonist (BID) mRNA, complete cds /cds=(140,727)
2.2 Cluster Incl. W28907:53e12 Homo sapiens cDNA /gb=W28907 /gi=1308855 /ug=Hs.111429 /len=989
2.3 Cluster Incl. AF073362:Homo sapiens endo/exonuclease Mre11 (MRE11A) mRNA, complete cds /cds=(0,2126)
2.3 Escherichia coli /REF=J04423 /DEF=E coli bioD gene dethiobiotin synthetase /LEN=676 (-5 and -3 represent transcript
2.3 Cluster Incl. D59253:Human mRNA for NCBP interacting protein 1, complete cds /cds=(36,506) /gb=D59253
2.3 M21154 /FEATURE=mRNA /DEFINITION=HUMAMD Human S-adenosylmethionine decarboxylase mRNA, complete cds
2.3 Proto-Oncogene C-Myc, Alt. Splice 3, Orf 114
2.3 Cluster Incl. W26787:15d8 Homo sapiens cDNA /gb=W26787 /gi=1306078 /ug=Hs.195188 /len=768
2.4 L12002 /FEATURE= /DEFINITION=HUMITGA4A Human integrin alpha 4 subunit mRNA, complete cds
2.4 Cluster Incl. M55536:Human glucose transporter pseudogene /cds=UNKNOWN /gb=M55536 /gi=183299 /ug=Hs.121583 2.4 X98743 /FEATURE=cds /DEFINITION=HSRNAHELC H.sapiens mRNA for RNA helicase (Myc-regulated dead box protein)
2.4 S75881 /FEATURE= /DEFINITION=S75881 A-myb=DNA-binding transactivator {3 region} [human, CCRF-CEM T
   leukemia line, mRNA Partial, 831 nt]
2.4 Cluster Incl. AF050110:Homo sapiens TGFb inducible early protein and early growth response protein alpha genes,
   complete cds /cds=(123,1565) /gb=AF050110 /gi=3523144 /ug=Hs.82173 /len=2899
2.5 Cluster Incl. M86667:H.sapiens NAP (nucleosome assembly protein) mRNA,
   complete cds /cds=(75,1250) /gb=M86667
   /gi=189066 /ug=Hs.179662 /len=1560
2.5 U17743 /FEATURE= /DEFINITION=HSU17743 Human JNK activating kinase (JNKK1) mRNA, complete cds
2.5 Cluster Incl. U90549:Human non-histone chromosomal protein (NHC) mRNA,
   complete cds /cds=(691,963) /gb=U90549
   /gi=2062699 /ug=Hs.63272 /len=1981
2.5 Cluster Incl. U31382:Human G protein gamma-4 subunit mRNA, complete cds
   /cds=(98,325) /gb=U31382 /gi=995916
   /ug=Hs.32976 /len=670
2.5 Cluster Incl. S81916:phosphoglycerate kinase {alternatively spliced} [human, phosphoglycerate kinase deficient patient
   with episodes of muscl, mRNA Partial Mutant, 307 nt] /cds=(0,143)
   /gb=S81916 /gi=1470308 /ug=Hs.169313 /len=307
2.5 Cluster Incl. M64595:Human small G protein (Gx) mRNA, 3 end /cds=(0,542)
   /gb=M64595 /gi=183708 /ug=Hs.173466
   /len=757
2.5 Serine Hydroxymethyltransferase, Cytosolic, Alt. Splice 3
2.5 U88629 /FEATURE=cds /DEFINITION=HSU88629 Human RNA polymerase II elongation factor ELL2, complete cds
2.5 Cluster Incl. U72518:Human destrin-2 pseudogene mRNA, complete cds
   /cds=(268,798) /gb=U72518 /gi=1673523
   /ug=Hs.199299 /len=1057
2.5 Cluster Incl. L14595:Human alanine/serine/cysteine/threonine transporter (ASCT1) mRNA, complete cds

2.5 Cluster Incl. AB014584:Homo sapiens mRNA for KIAA0684 protein, partial
   cds /cds=(0,2711) /gb=AB014584
   /gi=3327181 /ug=Hs.24594 /len=4124
2.5 Cluster Incl. AI924594:wn57a1l. Homo sapiens cDNA, 3 end /clone=IMAGE-2449532 /clone_end=3 /gb=AI924594
   /gi=5660558 /ug=Hs.122540 /len=685
2.5 U68111 /FEATURE=mRNA /DEFINITION=HSPPP1R2E6 Human protein phosphatase inhibitor 2 (PPP1R2) gene, exon 6
2.5 Cluster Incl. AL009179:dJ97D16.4 (Histone H2B) /cds=(25,405) /gb=AL009179 /gi=3217024 /ug=Hs.137594 /len=488
2.6 Cluster Incl. AF091077:Homo sapiens clone 558 unknown mRNA, complete sequence /cds=(1,300) /gb=AF091077
   /gi=3859991 /ug=Hs.40368 /len=947
2.7 Cluster Incl. M28211:Homo sapiens GTP-binding protein (RAB4) mRNA, complete cds /cds=(70,711) /gb=M28211
   /gi=550067 /ug=Hs.234038 /len=735
2.6 X69549 /FEATURE=cds /DEFINITION=HSRHO2 H.sapiens mRNA for rho GDP-dissociation Inhibitor 2
2.6 Cluster Incl. Z85986:Human DNA sequence from clone 108K11 on chromosome 6p21 Contains SRP20 (SR protein family member), Ndr protein kinase gene similar to yeast suppressor protein SRP40, EST and GSS /cds=(0,932) /gb=Z85986
   gi=4034056 /ug=Hs.152400 /len=933
2.6 Zinc Finger Protein, Kruppel-Like
2.7 D10656 /FEATURE= /DEFINITION=HUMCRK Human mRNA for CRK-II, complete cds
2.7 M28211 /FEATURE= /DEFINITION=HUMRAB4A Homo sapiens GTP-binding protein (RAB4) mRNA, complete cds
2.7 Cluster Incl. AB019435:Homo sapiens mRNA for putative phospholipase, complete cds /cds=(72,3074) /gb=AB019435
   /gi=4760646 /ug=Hs.125670 /len=3088
2.8 U39318 /FEATURE= /DEFINITION=HSU39318 Human E2 ubiquitin conjugating enzyme UbcH5C (UBCH5C) mRNA,
   complete cds
2.9 Cluster Incl. X78711:H.sapiens mRNA for glycerol kinase testis specific 1
   /cds=(26,1687) /gb=X78711 /gi=515028
   /ug=Hs.1466 /len=1838
2.8 Cluster Incl. W27594:34h4 Homo sapiens cDNA /gb=W27594 /gi=1307542 /ug=Hs.8258 /len=702
2.8 X05360 /FEATURE=cds /DEFINITION=HSCDC2 Human CDC2 gene involved in cell cycle control
2.8 V00568 /FEATURE=cds /DEFINITION=HSMYC1 Human mRNA encoding the c-myc oncogene
2.8 Cluster Incl. L24804:Human (p23) mRNA, complete cds /cds=(232,714) /gb=L24804 /gi=438651 /ug=Hs.75839 /len=782
2.10 Cluster Incl. Y09443:H.sapiens mRNA for alkyl-dihydroxyacetonephosphate synthase precursor /cds=(15,1991)
   /gb=Y09443 /gi=1922284 /ug=Hs.22580 /len=2074
2.8 Cluster Incl. Z82200:Human DNA sequence from clone 333E23 on chromosome Xq21.1 Contains putative purinergic
   receptor P2Y10 /cds=(0,1019) /gb=Z82200 /gi=2370075 /ug=Hs.166137 /len=1020
2.9 L05624 /FEATURE= /DEFINITION=HUMMKK Homo sapiens MAP kinase kinase mRNA, complete cds
2.9 Cluster Incl. D88357:Homo sapiens mRNA for CDC2 delta T, complete cds
   /cds=(27,749) /gb=D88357 /gi=3126638
   /ug=Hs.184572/len=780

Up regulated genes due to LQGV treatment

### Fold Change/Descriptions

4.9 Cluster Incl. AF043324:Homo sapiens N-myristoyltransferase 1 mRNA, complete cds (cds=(10,1500)/gb=AF043324 /gi=3005062 /ug=Hs.111039 /len=4378)
3.3 Cluster Incl. L08096:Human CD27 ligand mRNA, complete cds /cds=(150,731)
   (gb=L08096 /gi=307127 /ug=Hs.99899 /len=926)

2 Cluster Incl. AF043325:Homo sapiens N-myristoyltransferase 2 mRNA, complete cds /cds=(46,1542) /gb=AF043325
   /gi=3005064 /ug=Hs.122647 /len=2838
2.1 Cluster Incl. AL031681:dJ862K6.2.2 (splicing factor, arginine/serine-rich 6 (SRP55-2)(isoform 2)) /cds=(106,513)
2.1 Cluster Incl. X87838:H.sapiens mRNA for beta-catenin /cds=(214,2559) /gb=X87838 /gi=1154853 /ug=Hs.171271
2.2 Cluster Incl. AW024285:wt69d06.x1 Homo sapiens cDNA, 3 end /clone=IMAGE-2512715 /clone_end=3 /gb=AW024285
2.2 Cluster Incl. D38524:Human mRNA for 5-nucleotidase /cds=(83,1768) /gb=D38524 /gi=633070 /ug=Hs.138593
2.2 Cluster Incl. L38935:Homo sapiens GT212 mRNA /cds=UNKNOWN /gb=L38935 /gi=1008845 /ug=Hs.83086 /len=1165
2.5 Cluster Incl. L12711:Homo sapiens transketolase (tk) mRNA, complete cds /cds=(98,1969) /gb=L12711 /gi=388890
2.6 Cluster Incl. AF026029:Homo sapiens poly(A) binding protein II (PABP2) gene, complete cds /cds=(1282,2202)
2.8 Cluster Incl. X70683:H.sapiens mRNA for SOX-4 protein /cds=(350,1774) /gb=X70683 /gi=36552 /ug=Hs.83484

### Further examples of use

Examples of different receptor-intracellular signalling pathways involved in different disease pathogenesis where signalling molecules according to the invention find their use are:

LPS stimulation of antigen presenting cells (like monocytes) through different Toll-like receptors activates different signalling pathways including, MAPK pathways, ERK, JNK and p38 pathways. These pathways directly or indirectly phosphorylate and activate various transcription factors, including E1k-1 c-Jun, c-Fos, ATF-1, ATF-2, SRF, and CREB. In addition, LPS activates the IKK pathway of MyD88, IRAK, and TRAF6. TAK1-TAB2 and MEKK1-ECSIT complexes phosphorylate IKKb, which in turn phosphorylates IkBs. Subsequent degradation of IkBs permits nuclear translocation of NFkB/Rel complexes, such as p50/p65. Moreover, the P13K-Akt pathway phosphorylates and activates p65 via an unknown kinase. Some of these pathways could also be regulated by other receptor signalling such as hormones/growth factor receptor tyrosine kinases (PKC/Ras/IRS pathway) and cytokine receptors (JAK/STAT pathway). In the genomic experiment with the T-cell line several of these genes appeared to be downregulated or upregulated by the peptide used (LQGV). It is now clear that other peptides in T cells and the same and other peptides in other cell types similarly down-regulate or up-regulate several of these transcription factors and signalling molecules. In DC and fertilized eggs experiments NMPF had the ability to modulate growth factor (GM-CSF, VEGF) and LPS signalling. Some diseases associated with dysregulation of NF-kB and related transcription factors are: Atherosclerosis, asthma, arthritis, cachexia, cancer, diabetes, euthyroid sick syndrome, AIDS, inflammatory bowel disease, stroke, (sepsis) septic shock, inflammation, neuropathological diseases, autoimmunity, thrombosis, cardiovascular disease, psychological disease, post surgical depression, wound healing, burn-wounds healing and neurodegenerative disorders.

PKC plays an essential role in T cell activation via stimulation of for example AP-1 and NF-kB that selectively translocate to the T cell synapse via Vav/Rac pathway. PKC is involved in a variety of immunological and non-immunological diseases as is clear from standard text books of internal medicine (examples are metabolic diseases, cancer, angiogenesis, immune mediated disorders, diabetes etc.)

LPS and ceramide induce differential multimeric receptor complexes, including CD14, CD11b, Fc-gRIII, CD36, TAPA, DAF and TLR4. This signal transduction pathway explains the altered function of monocytes in hypercholesterolemia and lipid disorders.

Oxidized low-density lipoproteins contribute to stages of the atherogenic process and certain concentrations of oxidized low-density lipoproteins induce apoptosis in macrophages through signal transduction pathways. These pathways are involved in various vascular diseases such as atheroclerosis, thrombosis etc.

Bacterial DNA is recognized by cells of the innate immune system. This recognition requires endosomal maturation and leads to activation of NFkB and the MAPK pathway. Recently it has been shown that signaling requires the toll like receptor 9 (TLR9) and the signalling adaptor protein MyD88. Recognition of dsRNA during viral infection seems to be dependent on intracellular recognition by the dsRNA dependent protein kinase PKR. TLRs play an essential role in the immune system and they are important in bridging and blancing innate immunity and adoptive immunity. Modulation of these receptors or their down-stream signalling pathways are important for the treatment of various immunological conditions such as infections, cancer, immune-mediated diseases, autoimmunity, certain metabolic diseases with immunological component, vascular diseases, inflammatory diseases etc.

Effect of growth factor PDGF-AA on NF-kB and proinflammatory cytokine expression in rheumatoid synoviocytes; PDGF-AA augmented NF-kB activity and mRNA expression of IL-1b, IL-8 and MIP-1a. Therefore, PDGF-AA may play an important role in progression of inflammation as well as proliferation of synoviocytes in RA.

Dendritic cell (DC) activation is a critical event for the induction of immune responses. DC activation induced by LPS can be separated into two distinct processes: first, maturation, leading to upregulation of MHC and costimulatory molecules, and second, rescue from immediate apoptosis after withdrawal of growth factors (survival). LPS induces NF-kB transcription factor. Inhibition of NF-kB activation blocked maturation of DCs interms of upregulation of MHC and costimulatory molecules. In addition, LPS activates the extracellular signal-regulated kinases (ERK), and specific inhibition of MEK1, the kinase which activates ERK, abrogate the ability of LPS to prevent apoptosis but do not inhibit DC maturation or NF-kB nuclear translocation. This shows that ERK and NF-kB regulate different aspects of LPS induced DC activation. Our DC data and NF-kB data also show the various effects of NMPF peptide on DC maturation and proliferation in the present or absent of LPS. NMPF peptides modulate these pathways and are novel tools for the regulation of DC function and immunoregulation. This open new ways for the treatment of immune diseases, particularly those in which immune system is dysblance (DC1-DC2, Th1-Th2 regulatory cell etc.)
DC mediate NK cell activation which can result in tumour growth inhibition. DC cells and other antigen presenting cells (like macrophages, B-cells) play an essential role in the immune system and they are also important in bridging and balancing innate immunity and adoptive immunity. Modulation of these cells or their down-stream signaling pathways are important for the treatment of various immunological conditions such as infections, cancer, immune-mediated diseases, autoimmunity, certain metabolic diseases with immunological component, vascular diseases, inflammatory diseases etc. There is also evidence in the literature that mast cells play important roles in exerting the innate immunity by releasing inflammatory cytokines and recruitment of neutrophils after recognition of infectious agents through TLRs on mast cells.

In murine macrophages infected with Mycobacterium tuberculosis through JAK pathway activate STAT1 and activation of STAT1 may be the main transcription factor involved in IFN-g-induced MHC class II inhibition.

Recognition of mannose-binding lectin (MBL) through TLRs influences multiple immune mechanisms in response to infection and involved in innate immunity. Balance between innate and adoptive immunity is crucial for balanced immune system and dysregulation in immune system lead to different spectrum of diseases such as, inflammatory diseases, autoimmunity, infection diseases, pregnancy associated diseases (like miscarriage and pre-eclampsia), diabetes, atherosclerosis and other metabolic diseases.

Nuclear factor-kappaB (NFkappaB) is critical for the transcription of multiple genes involved in myocardial ischemia-reperfusion injury. Clinical and experimental studies have shown that myocardial ischemia-reperfusion injury results in activation of the TLRs and the complement system through both the classical and the alternative pathway in myocardial infaraction, atherosclerosis, intestinal ischaemia, hemorrhagic shock and pulmonary injury.

Peroxisome proliferator-activated receptors (PPARs) are ligand-activated transcription factors which function as regulators of lipid and lipoprotein metabolism, glucose homeostasis, influence cellular proliferation, differentiation and apoptosis and modulation of inflammatory responses. PPAR alpha is highly expressed in liver, muscle, kidney and heart, where it stimulates the beta-oxidative degradation of fatty acids. PPAR gamma is predominantly expressed in intestine and adipose tissue, where it triggers adipocyte differentiation and promotes lipid storage. Recently, the expression of PPAR alpha and PPAR gamma was also reported in cells of the vascular wall, such as monocyte/macrophages, endothelial and smooth muscle cells. The hypolipidemic fibrates and the antidiabetic glitazones are synthetic ligands for PPAR alpha and PPAR gamma, respectively. Furthermore, fatty acid-derivatives and eicosanoids are natural PPAR ligands: PPAR alpha is activated by leukotriene B4, whereas prostaglandin J2 is a PPAR gamma ligand, as well as of some components of oxidized LDL, such as 9- and 13-HODE. These observations suggested a potential role for PPARs not only in metabolic but also in inflammation control and, by consequence, in related diseases such as atherosclerosis. More recently, PPAR activators were shown to inhibit the activation of inflammatory response genes (such as IL-2, IL-6, IL-8, TNF alpha and metalloproteases) by negatively interfering with the NF-kappa B, STAT and AP-1 signalling pathways in cells of the vascular wall. Furthermore, PPARs may also control lipid metabolism in the cells of the atherosclerotic plaque. PPARs are also involved in a variety of immunological and non-immunological diseases as is clear from standard text books of internal medicine (examples are metabolic diseases, cancer, angiogenesis, immune mediated disorders, diabetes etc.)

As mentioned above the nuclear receptor PPARg is important in adipogenesis, lipid storage and involved in atherosclerosis. While expressed in adipose tissue this receptor is also expressed in macrophages and in the colon. In addition, PPARg is implicated in a number of processes such as cancer and inflammation. Moreover, microbes, via its cognate receptors, typified by the TLRs, possess the capacity to regulate PPARg dependent metabolic functions and as such illustrates the intricate interplay between the microbial flora and metabolic control in the alimentary tract.

Cyclo-oxygenase 2 (COX2), an inducible isoform of prostaglandin H synthase, which mediates prostaglandin synthesis during inflammation, and which is selectively overexpressed in colon tumours, is thought to play an important role in colon carcinogenesis. Induction of COX2 by inflammatory cytokines or hypoxia-induced oxidative stress can be mediated by nuclear factor kappa B (NF-kappaB). So, inhibition of NF-kB modulate COX pathway and this inhibition of NF-kB can be therapeutically useful in diseases in which COXs are involved, such as inflammation, pain, cancer (especially colorectal cancer), inflammatory bowel disease and others. Neuronal subsets in normal brains constitutively express functionally competent C5a receptors. The functional role of C5a receptors revealed that C5a triggered rapid activation of protein kinase C and activation and nuclear translocation of the NF-kappa B transcription factor. In addition, C5a was found to be mitogenic for undifferentiated human neuroblastoma cells, a novel action for the C5aR. In contrast, C5a protects terminally differentiated human neuroblastoma cells from toxicity mediated by the amyloid A beta peptide. This shows that normal hippocampal neurons as well as undifferentiated and differentiated human neuroblastoma cells express functional C5a receptors. These results show the role of neuronal C5aR receptors in normal neuronal development, neuronal homeostasis, and neuroinflammatory conditions such as Alzheimer's disease.

Activation of the complement system plays also an important role in the pathogenesis of atherosclerosis. The proinflammatory cytokine interleukin (IL)-6 is potentially involved in the progression of the disease. Here the complement system induces IL-6 release from human vascular smooth-muscle cells (VSMC) by a Gi-dependent pathway involving the generation of oxidative stress and the activation of the redox sensitive transcription factors NF-kB and AP-1. Modulation of complement system is important for broad ranges of disorders such as blood disorders, infections, some metabolic diseases (diabetes), vascular diseases, transplantation rejection and related disorders, autoimmune diseases, and other immunological diseases.

Different transcription factors like NF-kB and intracellular signaling molcules such as different kinases are also involved in multiple drug resistance. So, it is reasonable to believe that NMPF peptides will be effective against multiple drug resistance. Moreover, our genomic data shows that number of genes and signalling molecules involved in tumorogenesis and metastasis are modulated. In addition since oligopeptides have also effect on angiogenesis, thus these peptides will also be used for the treatment of cancer and related diseases whereby angiogenesis requires modulation.
Proliferative diabetic retinopathy (PDR) is one of the major causes of acquired blindness. The hallmark of PDR is neovascularisation (NV), abnormal angiogenesis that may ultimately cause severe vitreous cavity bleeding and/or retinal detachment. Since NMPF peptides have angiogenesis stimulatory as well as inhibitory effects and have the ability to modulate intracellular signaling involved in growth factors (like insulin), pharmacologic therapy with certain NMPF peptides can improve metabolic control (like glucose) or blunt the biochemical consequences of hyperglycaemia (through mechanisms such as in which aldose reductase, protein kinase C (PKC), PPARs are involved). For this metabolic control or diabetes (type 2) NMPF (LQGV, VLPALP, VLPALPQ, AQG, LAG, LQA, AQGV, VAPALP, VAPALPQ, VLPALPA, LPGC, MTR, LQG are recommneded. The angiogenesis in PDR could be also treated with above mentioned oligopeptides.

## Claims

1. A method for modulating expression of a gene in a cell comprising providing said cell with a signalling molecule comprising a peptide or functional analogue thereof.

2. A method according to claim 1 wherein said peptide is selected from the group of peptides LQG, AQG, LQGV, AQGV, LQGA, VLPALP, ALPALP, VAPALP, ALPALPQ, VLPAAPQ, VLPALAQ, LAGV, VLAALP, VLAALP, VLPALA, VLPALPQ, VLAALPQ, VLPALPA, GVLPALP, LQGVLPALPQVVC, LPGCPRGVNPVVS, LPGC, MTRV, MTR, VVC, and functional analogues or derivatives thereof.

3. A method according to claim 1 or 2 wherein said signalling molecule modulates translocation and/or activity of a gene transcription factor.

4. A method according to claim 3 wherein said gene transcription factor comprises a NF-kappaB/Rel protein.

5. A method for identifying or obtaining a signalling molecule comprising a peptide or functional derivative or analogue thereof capable of modulating expression of a gene in a cell comprising providing said cell with a peptide or derivative or analogue thereof and determining the activity and/or nuclear translocation of a gene transcription factor.

6. A method according to claim 5 further comprising determining whether said signalling molecule is membrane-permeable.

7. A method according to claim 5 or 6 wherein said gene transcription factor comprises a NF-kappaB/Rel protein.

8. A method for identifying or obtaining a signalling molecule comprising a peptide or functional derivative or analogue thereof capable of modulating expression of a gene in a cell comprising providing said cell with a peptide or derivative or analogue thereof and determining relative up-regulation and/or down-regulation of at least one gene expressed in said cell.

9. A method according to claim 5, 6 or 7 further comprising determining relative up-regulation and/or down-regulation of at least one gene expressed in said cell.

10. A method according to claim 8 or 9 further comprising determining relative up-regulation and/or down-regulation of a multitude of genes expressed in said cell.

11. A method for identifying or obtaining a signalling molecule comprising a peptide or functional derivative or analogue thereof capable of modulating expression of a gene in a cell comprising providing a peptide or derivative or analogue thereof and determining binding of said peptide or derivative or analogue thereof to a factor related to gene control.

12. A method according to claim 11 further comprising providing a multitude of peptides or derivatives or analogues thereof and determining binding of at least one of said peptides or derivatives or analogues thereof to a factor related to gene control.

13. A method according to claim 11 or 12 wherein said factor related to gene control comprises a transcription factor.

14. A method according to claim 13 wherein said transcription factor comprises a NF-kappaB-Rel protein.

15. A method according anyone of claims 11 to 14 further comprising providing a cell with said peptide or derivative or analogue thereof and determining the activity and/or nuclear translocation of a gene transcription factor in said cell.

16. A method according to anyone of claims 11 to 15 further comprising providing a cell with said peptide or derivative or analogue thereof and determining relative up-regulation and/or down-regulation of at least one gene expressed in said cell.

17. A signalling molecule useful in modulating expression of a gene in a cell and identifiable or obtainable by employing a method according to any one of claims 5 to 16.

18. A signalling molecule according to claim 17 selected from the group of peptides LQG, AQG, LQGV, AQGV, LQGA, VLPALP, ALPALP, VAPALP, ALPALPQ, VLPAAPQ, VLPALAQ, LAGV, VLAALP, VLAALP, VLPALA, VLPALPQ, VLAALPQ, VLPALPA, GVLPALP, LQGVLPALPQVVC, LPGCPRGVNPVVS, LPGC, MTRV, MTR, VVC, and functional analogues or derivatives thereof.

19. A signalling molecule capable of modulating expression of a gene in a cell comprising a peptide of at most 30 amino acids or a functional analogue or derivative thereof.

20. A signalling molecule according to claim 19 wherein said peptide is an oligopeptide of from about 3 to at about 15 amino acids long.

21. An inhibitor of NF-kappaB/Rel protein activation comprising a signalling molecule according to anyone of claims 17 to 20.

22. Use of a signalling molecule according to anyone of claim 17 to 20 for the production of a pharmaceutical composition for the modulation of gene expression.

23. Use according to claim 22 for the modulation of gene expression by inhibiting NF-kappaB/Rel protein activation.
